(19) Europäisches Patentamt
European Patent Office
Office européen des brevets

(11) EP 4 353 724 A1

(12) **EUROPEAN PATENT APPLICATION**
published in accordance with Art. 153(4) EPC

(43) Date of publication:
**17.04.2024 Bulletin 2024/16**

(21) Application number: **22819620.0**

(22) Date of filing: **09.06.2022**

(51) International Patent Classification (IPC):
***C07D 471/04*** *(2006.01)* ***A61K 31/519*** *(2006.01)*
***A61P 35/00*** *(2006.01)*

(52) Cooperative Patent Classification (CPC):
**A61K 31/519; A61P 35/00; C07D 471/04**

(86) International application number:
**PCT/CN2022/097927**

(87) International publication number:
**WO 2022/258023 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.06.2021 CN 202110644327**
**30.09.2021 CN 202111164489**

(71) Applicants:
- **TYK Medicines (Zhengzhou), Inc.**
  **Zhengzhou, Henan 451162 (CN)**
- **TYK Medicines Inc.**
  **Huzhou, Zhejiang 313100 (CN)**

(72) Inventors:
- **LI, Jun**
  **Huzhou, Zhejiang 313100 (CN)**
- **NIU, Chengshan**
  **Zhengzhou, Henan 451162 (CN)**
- **CHEN, Mingtao**
  **Zhengzhou, Henan 451162 (CN)**
- **LIANG, Apeng**
  **Huzhou, Zhejiang 313100 (CN)**
- **LI, Meihua**
  **Huzhou, Zhejiang 313100 (CN)**
- **DONG, Shengli**
  **Huzhou, Zhejiang 313100 (CN)**
- **WU, Yusheng**
  **Huzhou, Zhejiang 313100 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) COMPOUND AS CDK KINASE INHIBITOR AND USE THEREOF

(57) The present invention relates to a compound as a CDK kinase inhibitor and the use thereof. Specifically, the compound of the present invention has a structure as shown in formula (I), wherein the definitions of each group and each substituent are as described in the description. Further disclosed in the present invention are a method for preparing the compound and the use of the compound in regulating a CDK kinase activity or treating diseases related to CDK.

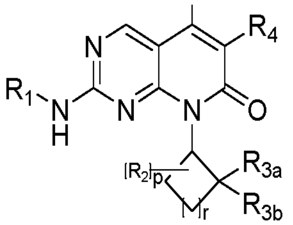
(I)

EP 4 353 724 A1

## Description

### Technical field

The invention relates to the field of medical technology, in particular to a compound as a CDK kinase inhibitor and its use in regulating CDK kinase activity or treating CDK-related diseases.

### Background

The cell cycle refers to the whole process that cells go through from the completion of one division to the end of the next division, which is divided into two stages: interphase and division phase. The interphase is further divided into three phases, the DNA synthesis prophase (G1 phase), the DNA synthesis phase (S phase) and the DNA synthesis anaphase (G2 phase); the division phase is the M phase. Cyclins and cyclin-dependent kinases (CDKs) are the core molecules in the entire cell cycle regulation mechanism. At least 16 mammalian cyclins have been identified, among which cyclin B/CDK1 (CyclinB/CDK1), cyclin A/CDK2 (CyclinA/CDK2), cyclin E/CDK2 (CyclinE/CDK2), cyclin D/CDK4 (CyclinD/CDK4), Cyclin D/CDK6 (CyclinD/CDK6) and possibly other heterodynes are important regulators of cell cycle progression. Other functions of cyclin/CDK heterodynes include transcriptional regulation, DNA repair, differentiation and apoptosis.

It has been confirmed that cell cycle dysregulation is a common feature of human cancer, and inhibitors of cyclin-dependent kinases (CDKs) play an important role in the regulation of cell cycle. It has broad application prospects in the field of cancer treatment. The CDK4/6 inhibitors palbociclib, ribociclib, and abemaciclib target breast cancer and other cancers; however, like other kinase inhibitors, their effects may be limited by the development of primary or acquired resistance over time. Therefore, PF-06873600 developed by Pfizer, as a new multi-target CDK kinase inhibitor, is expected to solve the phenomenon of drug resistance under existing conditions. PF-06873600 has also entered phase I clinical trials for the treatment of metastatic breast cancer.

Cyclin-dependent kinases (CDKs) inhibitors are the most promising field of cancer treatment, and the development of new compounds with CDK kinase inhibitory activity and better pharmacodynamics and pharmacokinetic property has become an important research project in the development of new anti-tumor drugs, and will eventually be used in the treatment of diseases such as human tumors.

### Summary of the Invention

The purpose of the present invention is to provide a compound represented by formula (I), preparation method therefor and application as CDK2/4/6 kinases inhibitor drugs.

The first aspect of the present invention provides a compound of formula (I), or a pharmaceutically acceptable salt, a stereoisomer, a solvate or a prodrug thereof,

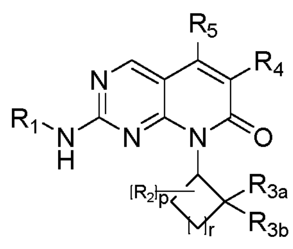 (I)

wherein,

$R_1$ is selected from the group consisting of: unsubstituted or 1-4 $R_6$ substituted C3-C10 cycloalkyl, unsubstituted or 1-4 $R_6$ substituted 5-15 membered fused ring without or containing 1-3 heteroatoms selected from N, O and S, unsubstituted or 1-4 $R_6$ substituted 5-15 membered spiro ring without or containing 1-3 heteroatoms selected from N, O and S, unsubstituted or 1-4 $R_6$ substituted 5-15 membered bridged ring without or containing 1-3 heteroatoms selected from N, O and S, unsubstituted or 1-4 $R_6$ substituted 3-10 membered heterocycloalkyl containing 1-4 heteroatoms selected from N, O and S, unsubstituted or 1-4 $R_6$ substituted C6-C10 aryl, unsubstituted or 1-4 $R_6$ substituted 3-10 membered heteroaryl containing 1-5 heteroatoms selected from N, O and S, and unsubstituted or 1-4 $R_6$ substituted C1-C6 alkyl;

$R_2$ is selected from the group consisting of: H, F, OH, C1-C4 alkyl, C1-C4 haloalkyl, C1-C4 alkoxy, C1-C4 haloalkoxy, and $R_2$ can be connected with atoms on the ring to form a spiro ring, a bridged ring, or a fused ring structure;

$R_{3a}$ and $R_{3b}$ are independently selected from the group consisting of: H, F, OH, C1-C4 alkyl, C1-C4 haloalkyl, C1-C4 alkoxy, and C1-C4 haloalkoxy;

among them, each of C1-C4 alkyl and C1-C4 haloalkyl in $R_2$, $R_{3a}$, and $R_{3b}$ is optionally substituted by halogen, OH, C1-C4 alkoxy or C1-C4 haloalkoxy;

$R_4$ is selected from the group consisting of: Br, substituted or unsubstituted 3-10 membered heterocycloalkyl containing 1-4 heteroatoms selected from N, O and S, $-OR_4'$, $-SR_4'$, $-NR_4'R_4''$, wherein $R_4'$ and $R_4''$ are independently selected from the group consisting of: H, $COR_7$, substituted or unsubstituted C1-C6 alkyl, substituted or unsubstituted C3-C6 cycloalkyl, substituted or unsubstituted 3-6 membered heterocycloalkyl containing 1-4 heteroatoms selected from N, O and S; and the "substituted" refers to being substituted by 1-3 substituents selected from the group consisting of: deuterium, halogen, OH, CN, =O, C1-C4 alkyl, C1-C4 alkoxy, and C1-C4 haloalkoxy;

$R_5$ is selected from the group consisting of: H, halogen, C1-C2 alkyl, C1-C2 haloalkyl, C1-C4 alkoxy, C1-C4 haloalkoxy;

each $R_6$ is independently selected from the group consisting of: H, deuterium, hydroxyl, halogen, cyano, =O, $COR_7$, $CO_2R_7$, $CONR_8R_9$, $CO_2NR_8R_9$, $SO_2R_7$, $SO_2NR_8R_9$, $NR_8SO_2R_7$ , $NHSO_2NR_8R_9$,

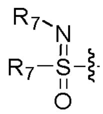
,

$NR_8R_9$, R‴ substituted or unsubstituted 3-10 membered heterocycloalkyl containing 1-3 heteroatoms selected from N, O and S, C1-C4 alkyl, C1-C4 haloalkyl, C1-C4 alkoxy, C1-C4 haloalkoxy, C3-C8 cycloalkyl, C3-C8 halocycloalkyl, C4-C10 spiro ring, C3-C10 fused ring, C4-C10 bridged ring, C1-C6 thioalkyl, C6-C10 aryl, and 3-10 membered heteroaryl containing 1-5 heteroatoms selected from N, O and S;

R‴ is selected from the group consisting of: =O, NR'R", C1-C4 alkyl, and C1-C4 haloalkyl;

each $R_7$ is independently selected from the group consisting of: C1-C4 alkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S), -L-(3-10 membered aryl), wherein the C1-C4 alkyl, C3-C8 cycloalkyl, 3-10 membered heterocycloalkyl, 3-10 membered heteroaryl, and 3-10 membered aryl are optionally substituted by 0 to 4 D, OH, halogen, CN, C1-C4 alkyl, C1-C4 haloalkyl, $N(C1\text{-}C4\ alkyl)_2$, NHCO(C1-C4 alkyl), $SO_2$(C1-C4 alkyl), $CO_2$(C1-C4 alkyl), 3-6 membered heterocycloalkyl containing 1-4 heteroatoms selected from N, O and S, CO(C1-C4 alkyl), C1-C4 alkoxy or C1-C4 haloalkoxy;

$R_8$ and $R_9$ are independently selected from the group consisting of: H, C1-C4 alkyl, C1-C4 haloalkyl, C3-C8 cycloalkyl, -L-(C3-C8 cycloalkyl), -L-(3-10 membered heterocycloalkyl containing 1-4 heteroatoms selected from N, O and S), and -L-(3-10 membered heteroaryl containing 1-5 heteroatoms selected from N, O and S) ;or

$R_8$ and $R_9$ together with the N to which they are attached can form a substituted or unsubstituted 4-6 membered heterocyclyl, and the "substituted" refers to being substituted by 1-3 substituents selected from the group consisting of: =O, NR'R", and C1-C6 alkyl;

each R' and R" is independently selected from the group consisting of: H, and C1-C4 alkyl;

each L is independently a bond or C1-C4 alkylene, and the C1-C4 alkylene is optionally substituted by OH, C1-C4 alkoxy or C1-C4 haloalkoxy;

p is selected from the group consisting of: 0, 1, 2, 3 and 4;

r is selected from the group consisting of: 0, 1, 2, 3 and 4.

In another preferred embodiment, $R_1$ is selected from the group consisting of: unsubstituted or 1-4 $R_6$ substituted C3-C10 cycloalkyl, unsubstituted or 1-4 $R_6$ substituted 5-15 membered fused ring without or containing 1-3 heteroatoms selected from N, O and S, unsubstituted or 1-4 $R_6$ substituted 5-15 membered spiro ring without or containing 1-3 heteroatoms selected from N, O and S, unsubstituted or 1-4 $R_6$ substituted 5-15 membered bridged ring without or containing 1-3 heteroatoms selected from N, O and S, unsubstituted or 1-4 $R_6$ substituted 3-10 membered heterocycloalkyl containing 1-4 heteroatoms selected from N, O and S, unsubstituted or 1-4 $R_6$ substituted C6-C10 aryl, and unsubstituted or 1-4 $R_6$ substituted 3-10 membered heteroaryl containing 1-5 heteroatoms selected from N, O and S;

$R_2$ is selected from the group consisting of: H, and F;

$R_{3a}$ and $R_{3b}$ are independently selected from the group consisting of: H, F, OH, and C1-C4 alkyl;

$R_4$ is selected from the group consisting of: substituted or unsubstituted 3-10 membered heterocycloalkyl containing 1-4 heteroatoms selected from N, O and S, $-OR_4'$, $-NR_4'R_4''$, wherein $R_4'$ and $R_4''$ are independently selected from the group consisting of: H, and substituted or unsubstituted C1-C6 alkyl, and the "substituted" refers to being substituted by 1-3 substituents selected from the group consisting of: deuterium, halogen, and C1-C4 alkyl;

$R_5$ is selected from the group consisting of: H, and C1-C2 alkyl;

each $R_6$ is independently selected from the group consisting of: H, deuterium, hydroxyl, halogen, cyano, =O, $COR_7$, $CO_2R_7$, $CONR_8R_9$, $SO_2R_7$, $SO_2NR_8R_9$, $NR_8SO_2R_7$, $NHSO_2NR_8R_9$, $NR_8R_9$, R‴ substituted or unsubstituted 3-10 membered heterocycloalkyl containing 1-3 heteroatoms selected from N, O and S, C1-C4 alkyl, C1-C4 haloalkyl, C1-C4 alkoxy, C1-C4 haloalkoxy, C3-C8 cycloalkyl, C3-C8 halocycloalkyl, C4-C10 spiro ring, C3-C10 fused ring, C4-C10 bridged ring, C1-C6 thioalkyl, C6-C10 aryl, 3-10 membered heteroaryl containing 1-5 heteroatoms selected from N, O and S;

R‴ is selected from the group consisting of: =O, NR'R", C1-C4 alkyl, and C1-C4 haloalkyl;

each $R_7$ is independently selected from the group consisting of: C1-C4 alkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S), -L-(3-10 membered aryl), wherein the C1-C4 alkyl, C3-C8 cycloalkyl, 3-10 membered heterocycloalkyl, 3-10 membered heteroaryl and 3-10 membered aryl are optionally substituted by 0 to 4 D, OH, halogen, CN, C1-C4 alkyl, C1-C4 haloalkyl, $N(C1\text{-}C4\ alkyl)_2$, NHCO(C1-C4 alkyl), $SO_2$(C1-C4 alkyl), $CO_2$(C1-C4 alkyl), 3-6 membered heterocycloalkyl containing 1-4 heteroatoms selected from N, O and S, CO(C1-C4 alkyl), C1-C4 alkoxy or C1-C4 haloalkoxy;

$R_8$ and $R_9$ are independently selected from the group consisting of: H, C1-C4 alkyl, C1-C4 haloalkyl, C3-C8 cycloalkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S); or

$R_8$ and $R_9$ together with the N to which they are attached can form a substituted or unsubstituted 4-6 membered heterocyclyl, and the "substituted" refers to being substituted by 1-3 substituents selected from the group consisting of: =O, NR'R", and C1-C6 alkyl;

each R' and R" is independently selected from the group consisting of: H, and C1-C4 alkyl;

each L is independently a bond or C1-C4 alkylene, and the C1-C4 alkylene is optionally substituted by OH, C1-C4 alkoxy or C1-C4 haloalkoxy;

p is selected from the group consisting of: 0, 1, 2, 3 and 4;

r is selected from the group consisting of: 0, 1, 2, 3 and 4.

In another preferred embodiment, $R_1$ is selected from the group consisting of: unsubstituted or 1-4 $R_6$ substituted C3-C10 cycloalkyl, unsubstituted or 1-4 $R_6$ substituted 3-10 membered heterocycloalkyl containing 1-4 heteroatoms selected from N, O and S, unsubstituted or 1-4 $R_6$ substituted C6-C10 aryl, unsubstituted or 1-4 $R_6$ substituted 3-10 membered heteroaryl containing 1-5 heteroatoms selected from N, O and S;

$R_2$ is selected from the group consisting of: H, and F;

$R_{3a}$ and $R_{3b}$ are independently selected from the group consisting of: H, F, OH, and C1-C4 alkyl;

$R_4$ is selected from the group consisting of: substituted or unsubstituted 3-10 membered heterocycloalkyl containing 1-4 heteroatoms selected from N, O and S, and $-OR_4'$, wherein $R_4'$ is independently selected from the group consisting of: H, and substituted or unsubstituted C1-C6 alkyl, and the "substituted" refers to being substituted by 1-3 substituents selected from the group consisting of: deuterium, halogen, and C1-C4 alkyl;

$R_5$ is selected from the group consisting of: H, and C1-C2 alkyl;

each $R_6$ is independently selected from the group consisting of: H, deuterium, hydroxyl, halogen, cyano, =O, $COR_7$, $CO_2R_7$, $CONR_8R_9$, $SO_2R_7$, $SO_2NR_8R_9$, $NR_8SO_2R_7$, $NHSO_2NR_8R_9$, $NR_8R_9$, R‴ substituted or unsubstituted 3-10 membered heterocycloalkyl containing 1-3 heteroatoms selected from N, O and S, C1-C4 alkyl, C1-C4 haloalkyl, C1-C4 alkoxy, C1-C4 haloalkoxy, C3-C8 cycloalkyl, C3-C8 halocycloalkyl, C4-C10 spiro ring, C3-C10 fused ring, C4-C10 bridged ring, C1-C6 thioalkyl, C6-C10 aryl, and 3-10 membered heteroaryl containing 1-5 heteroatoms selected from N, O and S;

R‴ is selected from the group consisting of: =O, NR'R", C1-C4 alkyl, and C1-C4 haloalkyl;

each $R_7$ is independently selected from the group consisting of: C1-C4 alkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S), -L-(3-10 membered aryl), wherein the C1-C4 alkyl, C3-C8 cycloalkyl, 3-10 membered heterocycloalkyl, 3-10 membered heteroaryl and 3-10 membered aryl are optionally substituted by 0 to 4 D, OH, halogen, CN, C1-C4 alkyl, C1-C4 haloalkyl, $N(C1\text{-}C4\ alkyl)_2$, NHCO(C1-C4 alkyl), $SO_2$(C1-C4 alkyl), $CO_2$(C1-C4 alkyl), 3-6 membered heterocycloalkyl containing 1-4 heteroatoms selected from N, O and S, CO(C1-C4 alkyl), C1-C4 alkoxy or C1-C4 haloalkoxy;

$R_8$ and $R_9$ are independently selected from the group consisting of: H, C1-C4 alkyl, C1-C4 haloalkyl, C3-C8 cycloalkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S); or

$R_8$ and $R_9$ together with the N to which they are attached can form a substituted or unsubstituted 4-6 membered

heterocyclyl, and the "substituted" refers to being substituted by 1-3 substituents selected from the group consisting of: =O, NR'R", and C1-C6 alkyl;

each R' and R" is independently selected from the group consisting of: H, and C1-C4 alkyl;

each L is independently a bond or C1-C4 alkylene, and the C1-C4 alkylene is optionally substituted by OH, C1-C4 alkoxy or C1-C4 haloalkoxy;

p is selected from the group consisting of: 0, 1, 2, 3 and 4;

r is selected from the group consisting of: 0, 1, 2, 3 and 4.

In another preferred embodiment, $R_1$ is selected from the group consisting of: unsubstituted or 1-4 $R_6$ substituted C3-C10 cycloalkyl, unsubstituted or 1-4 $R_6$ substituted 5-15 membered fused ring without or containing 1-3 heteroatoms selected from N, O and S, unsubstituted or 1-4 $R_6$ substituted 5-15 membered spiro ring without or containing 1-3 heteroatoms selected from N, O and S, unsubstituted or 1-4 $R_6$ substituted 5-15 membered bridged ring without or containing 1-3 heteroatoms selected from N, O and S, unsubstituted or 1-4 $R_6$ substituted 3-10 membered heterocycloalkyl containing 1-4 heteroatoms selected from N, O and S, unsubstituted or 1-4 $R_6$ substituted C6-C10 aryl, unsubstituted or 1-4 $R_6$ substituted 3-10 membered heteroaryl containing 1-5 heteroatoms selected from N, O and S, and unsubstituted or 1-4 $R_6$ substituted C1-C6 alkyl;

$R_2$ is selected from the group consisting of: H, F, OH, C1-C4 alkyl, C1-C4 haloalkyl, C1-C4 alkoxy, C1-C4 haloalkoxy, and $R_2$ can be connected with atoms on the ring to form a spiro ring, a bridged ring, or a fused ring structure;

$R_{3a}$ and $R_{3b}$ are independently selected from the group consisting of: H, F, OH, C1-C4 alkyl, C1-C4 haloalkyl, C1-C4 alkoxy, and C1-C4 haloalkoxy;

among them, each of the C1-C4 alkyl and C1-C4 haloalkyl in $R_2$, $R_{3a}$ and $R_{3b}$ is optionally substituted by halogen, OH, C1-C4 alkoxy or C1-C4 haloalkoxy;

$R_4$ is selected from the group consisting of: Br, substituted or unsubstituted 3-10 membered heterocycloalkyl containing 1-4 heteroatoms selected from N, O and S, $-OR_4'$, $-SR_4'$, $-NR_4'R_4''$, wherein $R_4'$ and $R_4''$ are independently selected from the group consisting of: H, $COR_7$, substituted or unsubstituted C1-C6 alkyl, substituted or unsubstituted C3-C6 cycloalkyl, substituted or unsubstituted 3-6 membered heterocycloalkyl containing 1-4 heteroatoms selected from N, O and S; and the "substituted" refers to being substituted by 1-3 substituents selected from the group consisting of: deuterium, halogen, OH, CN, =O, C1-C4 alkyl, C1-C4 alkoxy, and C1- C4 haloalkoxy;

$R_5$ is selected from the group consisting of: H, halogen, C1-C2 alkyl, C1-C2 haloalkyl, C1-C4 alkoxy, and C1-C4 haloalkoxy;

each $R_6$ is independently selected from the group consisting of: H, deuterium, hydroxyl, halogen, cyano, =O, $COR_7$, $CO_2R_7$, $CONR_8R_9$, $CO_2NR_8R_9$, $SO_2R_7$, $SO_2NR_8R_9$, $NR_8SO_2R_7$ , $NHSO_2NR_8R_9$,

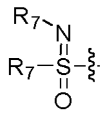
,

$NR_8R_9$, R''' substituted or unsubstituted 3-10 membered heterocycloalkyl containing 1-3 heteroatoms selected from N, O and S, C1-C4 alkyl, C1-C4 haloalkyl, C1-C4 alkoxy, C1-C4 haloalkoxy, C3-C8 cycloalkyl, C3-C8 halocycloalkyl, C4-C10 spiro ring, C3-C10 fused ring, C4-C10 bridged ring, C1-C6 thioalkyl, C6-C10 aryl, and 3-10 membered heteroaryl containing 1-5 heteroatoms selected from N, O and S;

R''' is selected from the group consisting of: =O and NR'R";

each $R_7$ is independently selected from the group consisting of: C1-C4 alkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S), -L-(3-10 membered aryl), wherein the C1-C4 alkyl, C3-C8 cycloalkyl, 3-10 membered heterocycloalkyl, 3-10 membered heteroaryl, and 3-10 membered aryl are optionally substituted by 0 to 4 D, OH, halogen, CN, C1-C4 alkyl, C1-C4 haloalkyl, $N(C1\text{-}C4\ alkyl)_2$, NHCO(C1-C4 alkyl), $SO_2$(C1-C4 alkyl), 3-6 membered heterocycloalkyl containing 1-4 heteroatoms selected from N, O and S, CO(C1-C4 alkyl), C1-C4 alkoxy or C1-C4 haloalkoxy;

$R_8$ and $R_9$ are independently selected from the group consisting of: H, C1-C4 alkyl, C1-C4 haloalkyl, C3-C8 cycloalkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S); or

$R_8$ and $R_9$ together with the N to which they are attached can form a substituted or unsubstituted 4-6 membered heterocyclyl, and the "substituted" refers to being substituted by 1-3 substituents selected from the group consisting of: =O, NR'R", and C1-C6 alkyl;

each R' and R" is independently selected from the group consisting of: H, and C1-C4 alkyl;

each L is independently a bond or C1-C4 alkylene, and the C1-C4 alkylene is optionally substituted by OH, C1-C4 alkoxy or C1-C4 haloalkoxy;
p is selected from the group consisting of: 0, 1, 2, 3 and 4;
r is selected from the group consisting of: 0, 1, 2, 3 and 4.

In another preferred embodiment, the compound has the structure shown in formula (II) or formula (III):

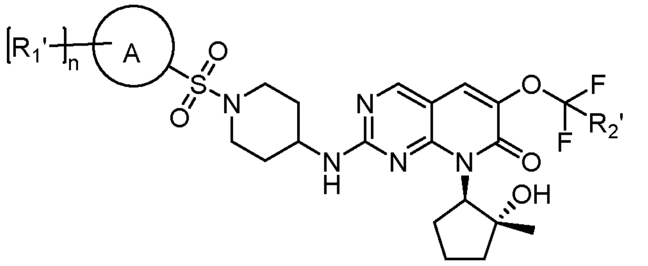

(II)

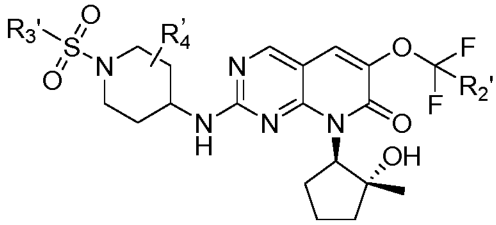

(III)

wherein,

ring A is selected from the group consisting of: 0-4 $R_1$' substituted 6-10 membered aryl, 0-4 $R_1$' substituted C3-C8 cycloalkyl, 0-4 $R_1$' substituted 5-10 membered heteroaryl containing 1-5 heteroatoms selected from N, O and S;
each $R_1$' is independently selected from the group consisting of: deuterium, halogen, OH, CN, $SO_2R_{31}$, $COR_{31}$, $CO_2R_{31}$, $NR_{41}R_{51}$, $NHCOR_{41}$, $CONR_{41}R_{51}$, $OCONR_{41}R_{51}$, $NHCONR_{41}R_{51}$, $NHCOOR_{41}$, C1-C4 alkyl, C1-C4 alkoxy, C1-C4 haloalkoxy, substituted or unsubstituted C3-C6 cycloalkyl, substituted or unsubstituted 3-6 membered heterocycloalkyl containing 1-4 heteroatoms selected from N, O and S, and the "substituted" refers to being substituted by 1-3 substituents selected from the group consisting of: deuterium, halogen, OH, CN, =O, C1-C4 alkyl, C1-C4 alkoxy, and C1-C4 haloalkoxy;
$R_2$' is selected from the group consisting of: H, and deuterium;
$R_3$' is selected from the group consisting of: substituted or unsubstituted C1-C4 alkyl, and the "substituted" refers to being substituted by 1-3 substituents selected from the group consisting of: deuterium, halogen, OH, CN, =O, C1-C4 alkyl, C1-C4 alkoxy, C1-C4 haloalkoxy;
$R_4$' is selected from the group consisting of: H, deuterium, OH; $R_{31}$ is selected from the group consisting of: H, C1-C4 alkyl, C1-C4 haloalkyl, C1-C4 alkoxy, C1-C4 haloalkoxy, substituted or unsubstituted C3-C6 cycloalkyl, substituted or unsubstituted 3-6 membered heterocycloalkyl containing 1-4 heteroatoms selected from N, O and S, and the "substituted" refers to being substituted by 1-3 substituents selected from the group consisting of: deuterium, halogen, OH, CN, =O, C1-C4 alkyl, C1-C4 alkoxy, and C1-C4 haloalkoxy;
$R_{41}$ and $R_{51}$ are independently selected from the group consisting of: H, C1-C4 alkyl, C1-C4 haloalkyl, C3-C8 cycloalkyl, -L'-(C3-C8 cycloalkyl), -L'-(3-10 membered heterocycloalkyl containing 1-4 heteroatoms selected from N, O and S), and -L'-(3-10 membered heteroaryl containing 1-5 heteroatoms selected from N, O and S aryl), and L' is a bond or C1-C6 alkylene; or
$R_{41}$ and $R_{51}$ together with the N to which they are attached can form a substituted or unsubstituted 4-6 membered heterocyclyl, and the "substituted" refers to being substituted by 1-3 substituents selected from the group consisting of: halogen, OH, =O, and C1-C6 alkyl;
n is selected from the group consisting of: 0, 1, 2, 3 and 4.

In another preferred embodiment, the compound has the structure shown in formula (II):

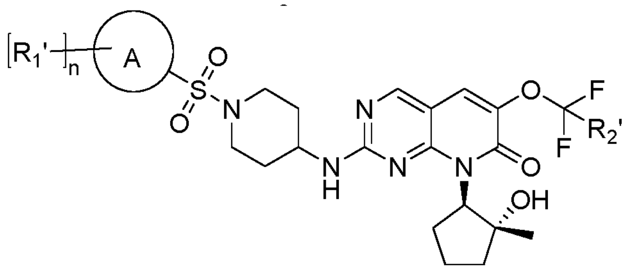

(II)

wherein,

ring A is selected from the group consisting of: 0-4 $R_1$' substituted 6-10 membered aryl, 0-4 $R_1$' substituted C3-C8 cycloalkyl, 0-4 $R_1$' substituted 5-10 membered heteroaryl containing 1-5 heteroatoms selected from N, O and S;

each $R_1$' is independently selected from the group consisting of deuterium, halogen, OH, CN, $SO_2R_{31}$, $COR_{31}$, $CO_2R_{31}$, $NR_{41}R_{51}$, $NHCOR_{41}$, $CONR_{41}R_{51}$, $OCONR_{41}R_{51}$, $NHCONR_{41}R_{51}$, $NHCOOR_{41}$, C1-C4 alkyl, C1-C4 alkoxy, C1-C4 haloalkoxy, substituted or unsubstituted C3-C6 cycloalkyl, substituted or unsubstituted 3-6 membered heterocycloalkyl containing 1-4 heteroatoms selected from N, O and S, and the "substituted" refers to being substituted by 1-3 substituents selected from the group consisting of: deuterium, halogen, OH, CN, =O, C1-C4 alkyl, C1-C4 alkoxy, and C1-C4 haloalkoxy;

$R_2$' is selected from the group consisting of: H, and deuterium;

$R_{31}$ is selected from the group consisting of: H, C1-C4 alkyl, C1-C4 haloalkyl, C1-C4 alkoxy, C1-C4 haloalkoxy, substituted or unsubstituted C3-C6 cycloalkyl, substituted or unsubstituted 3-6 membered heterocycloalkyl containing 1-4 heteroatoms selected from N, O and S, and the "substituted" refers to being substituted by 1-3 substituents selected from the group consisting of: deuterium, halogen , OH, CN, =O, C1-C4 alkyl, C1-C4 alkoxy, and C1-C4 haloalkoxy;

$R_{41}$ and $R_{51}$ are independently selected from the group consisting of: H, C1-C4 alkyl, C1-C4 haloalkyl, C3-C8 cycloalkyl, -L'-(C3-C8 cycloalkyl), -L'-(3-10 membered heterocycloalkyl containing 1-4 heteroatoms selected from N, O and S), and -L'-(3-10 membered heteroaryl containing 1-5 heteroatoms selected from N, O and S aryl), and L' is a bond or C1-C6 alkylene; or

$R_{41}$ and $R_{51}$ together with the N to which they are attached can form a substituted or unsubstituted 4-6 membered heterocyclyl, and the "substituted" refers to being substituted by 1-3 substituents selected from the group consisting of: halogen, OH, =O, and C1-C6 alkyl;

n is selected from the group consisting of: 0, 1, 2, 3 and 4.

In another preferred embodiment, ring A is 0-4 $R_1$' substituted 6-10 membered aryl.

In another preferred embodiment, $R_3$' is C1-C4 alkyl.

In another preferred embodiment, ring A is phenyl.

In another preferred embodiment,

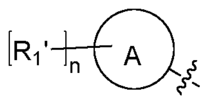

has the following structure:

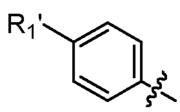

.

In another preferred embodiment, in

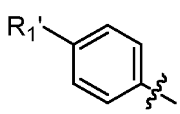

,

$R_1$' is selected from the group consisting of: H, halogen, CN, C1-C4alkoxy.

In another preferred embodiment, in

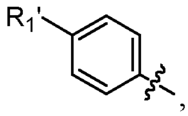

$R_1$' is F, OMe.

In another preferred embodiment, in

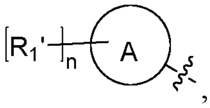

ring A is phenyl, n is 1, $R_1$' is selected from the group consisting of: H, halogen, CN, and C1-C4 alkoxy.

In another preferred embodiment, $R_1$' is F.

In another preferred embodiment,

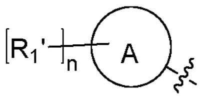

has the following structure:

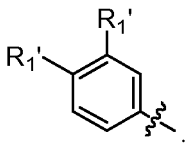

In another preferred embodiment, in

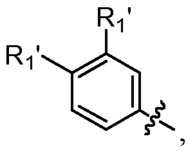

$R_1$' is F.

In another preferred embodiment, ring A is 0-4 $R_1$' substituted C3-C8 cycloalkyl.

In another preferred embodiment, the compound has the structure shown in formula (IV) or formula (V):

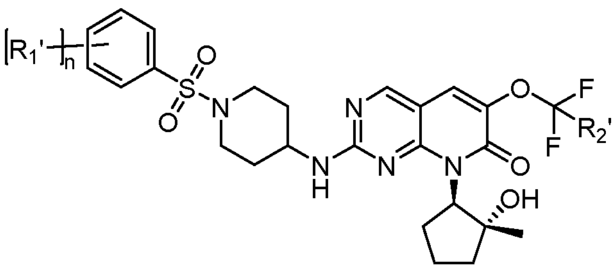

**(IV)**

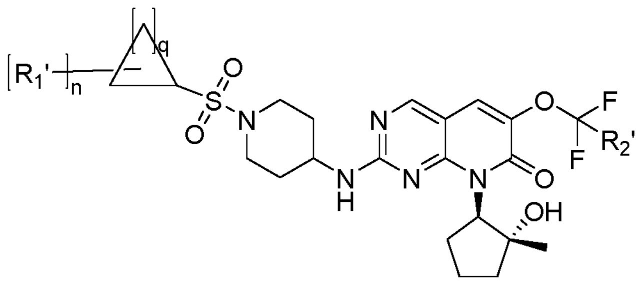

**(V)**

wherein, q is selected from the group consisting of: 1, 2, 3, 4, 5, and 6.

In another preferred embodiment, ring A is 0-4 $R_1$' substituted 5-10 membered heteroaryl containing 1-5 heteroatoms selected from N, O and S.

In another preferred embodiment, ring A is selected from the group consisting of: 0-4 $R_1$' substituted 6-10 membered aryl, 0-4 $R_1$' substituted C3-C8 cycloalkyl, 0-4 $R_1$' substituted 5-10 membered heteroaryl containing 1-5 heteroatoms selected from N, O and S;

each $R_1$' is independently selected from the group consisting of: deuterium, halogen, OH, CN, $SO_2R_{31}$, $COR_{31}$, $CO_2R_{31}$, $NR_{41}R_{51}$, $NHCOR_{41}$, $CONR_{41}R_{51}$, C1-C4 haloalkyl, C1-C4 alkoxy, C1-C4 haloalkoxy, substituted or unsubstituted C3-C6 cycloalkyl, substituted or unsubstituted 3-6 membered heterocycloalkyl containing 1-4 heteroatoms selected from N, O and S;
$R_2$' is selected from the group consisting of: H, and deuterium;
$R_4$' is selected from the group consisting of: H, and deuterium;
$R_{31}$ is C1-C4 alkyl;
$R_{41}$ and $R_{51}$ are independently selected from the group consisting of: H, and C1-C4 alkyl;
n is selected from the group consisting of: 0, 1, 2, 3 and 4.

In another preferred embodiment, ring A is selected from the group consisting of: 0-4 $R_1$' substituted 6-10 membered aryl, 0-4 $R_1$' substituted C3-C8 cycloalkyl, 0-4 $R_1$' substituted 5-10 membered heteroaryl containing 1-5 heteroatoms selected from N, O and S;

each $R_1$' is independently selected from the group consisting of: deuterium, halogen, OH, CN, $SO_2R_{31}$, $COR_{31}$, $CO_2R_{31}$, $NR_{41}R_{51}$, $NHCOR_{41}$, $CONR_{41}R_{51}$, C1-C4 haloalkyl, C1-C4 alkoxy, C1-C4 haloalkoxy, substituted or unsubstituted C3-C6 cycloalkyl, substituted or unsubstituted 3-6 membered heterocycloalkyl containing 1-4 heteroatoms selected from N, O and S;
$R_2$' is selected from the group consisting of: H, and deuterium;
$R_{31}$ is C1-C4 alkyl;
$R_{41}$ and $R_{51}$ are independently selected from the group consisting of: H, and C1-C4 alkyl;
n is selected from the group consisting of: 0, 1, 2, 3 and 4.

In another preferred embodiment, ring A is selected from the group consisting of: 0-4 $R_1$' substituted 6-10 membered aryl, and 0-4 $R_1$'substituted C3-C8 cycloalkyl;

$R_1$' is selected from the group consisting of halogen, OH, C1-C4 alkoxy, and C1-C4 haloalkoxy;
$R_2$' is selected from the group consisting of H, and deuterium;
n is selected from the group consisting of: 0, 1, 2, 3 and 4.

In another preferred embodiment, the compound is selected from the group consisting of:

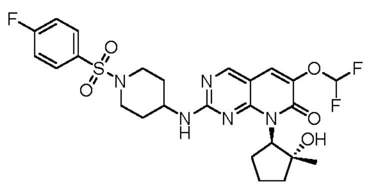

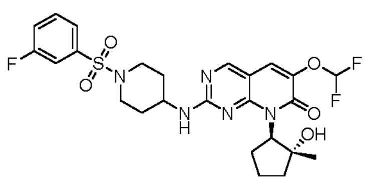

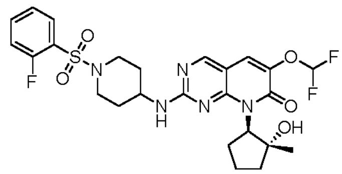

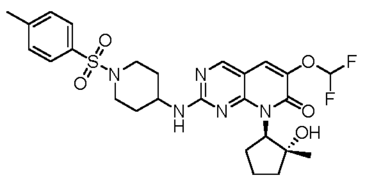
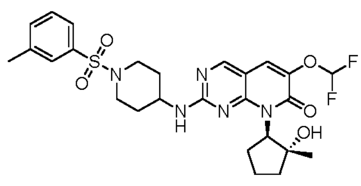
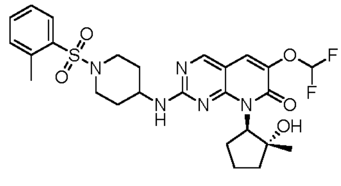
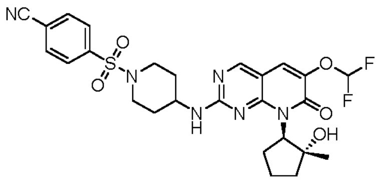
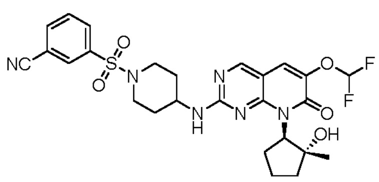
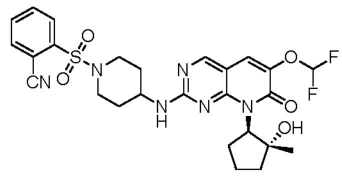
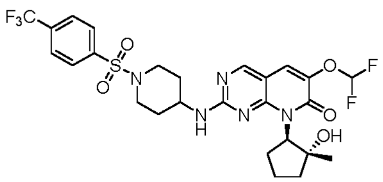
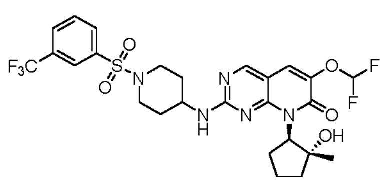
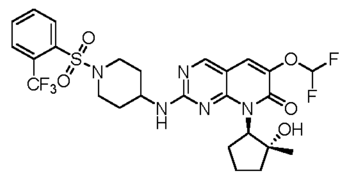
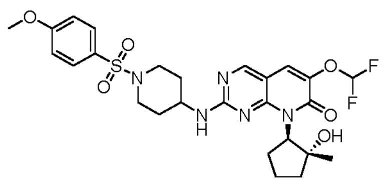
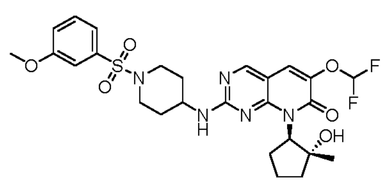
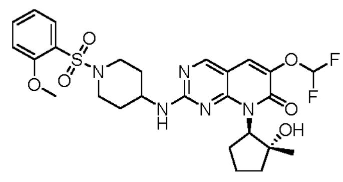
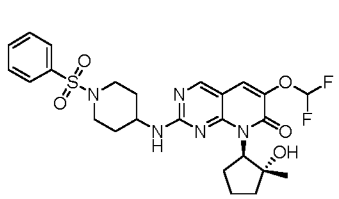
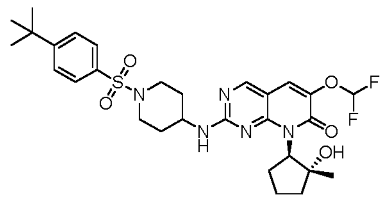
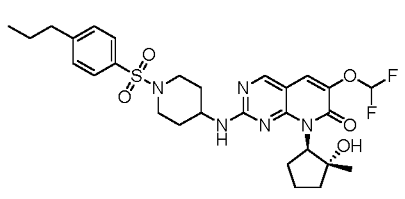
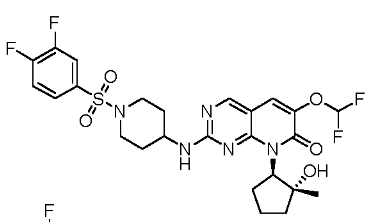
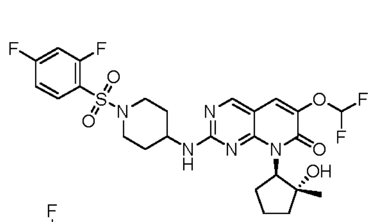
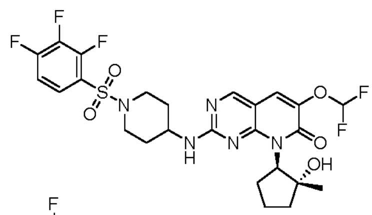
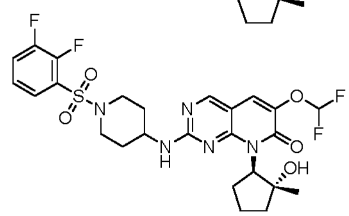
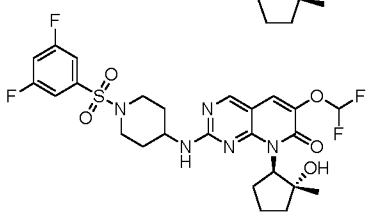
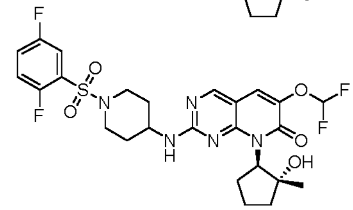
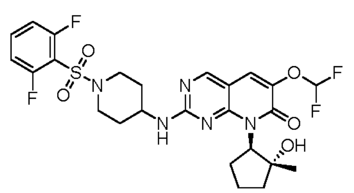
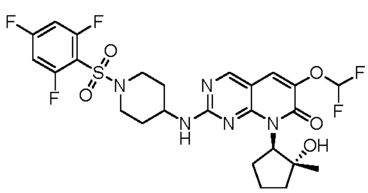
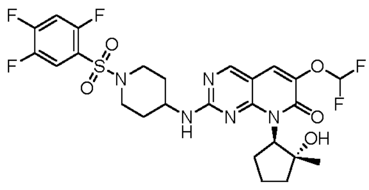

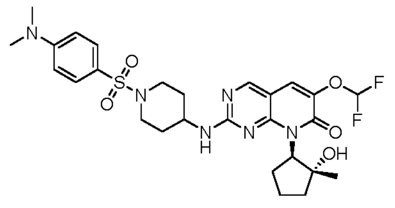
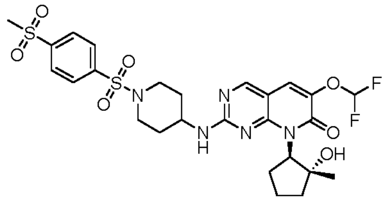
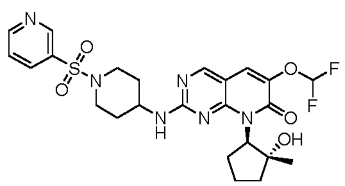
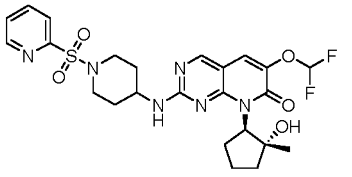
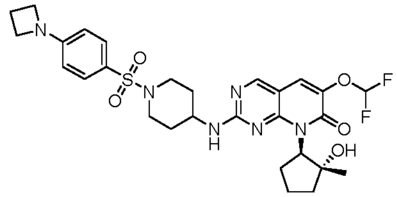
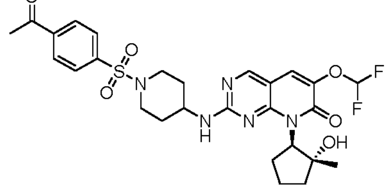
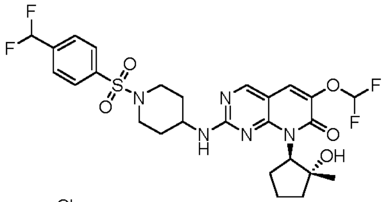
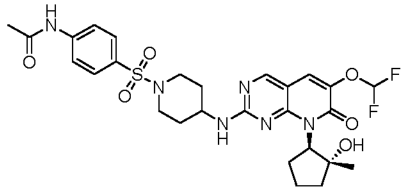
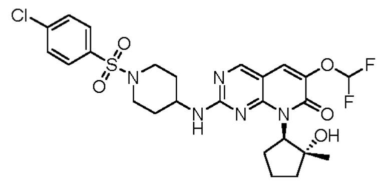
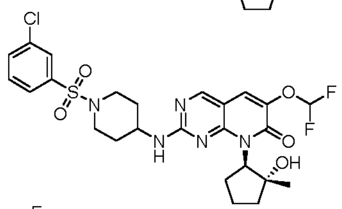
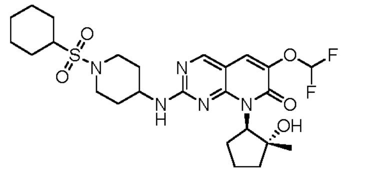
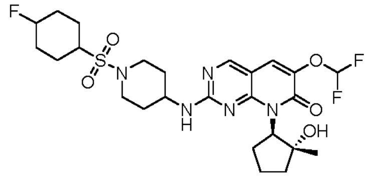
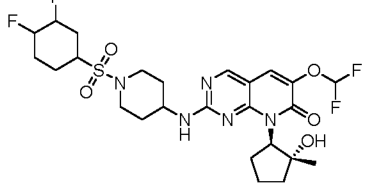
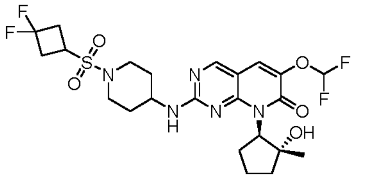
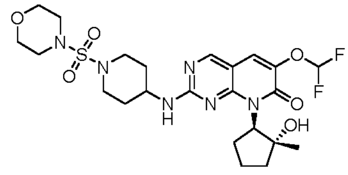
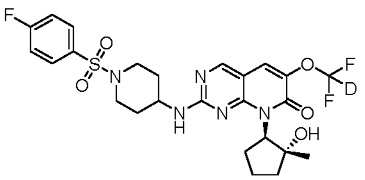
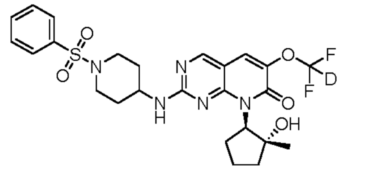
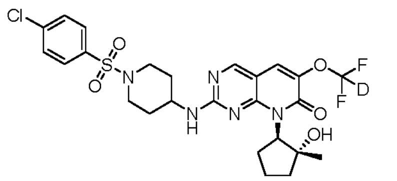
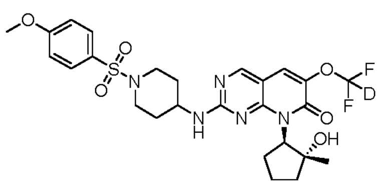
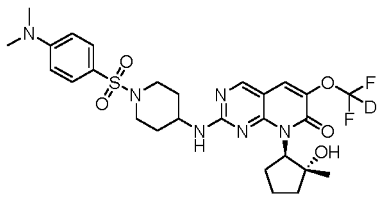
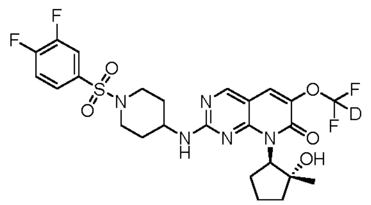
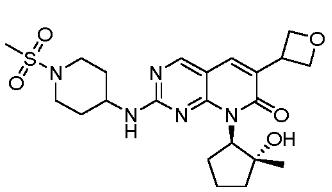
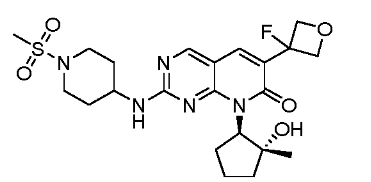
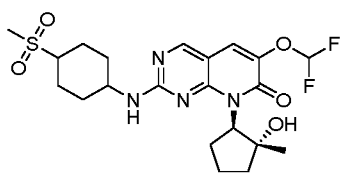

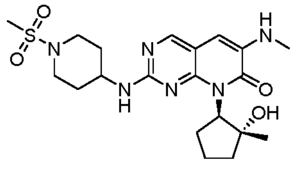
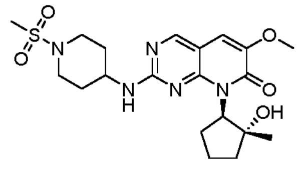
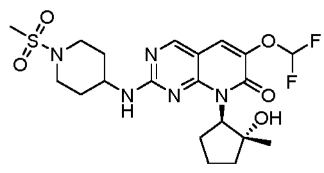

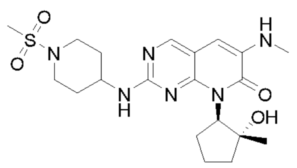
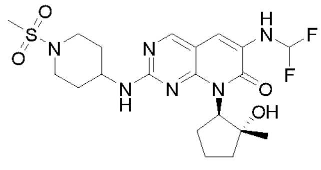
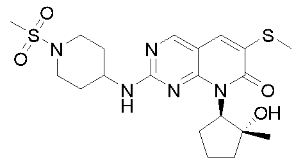

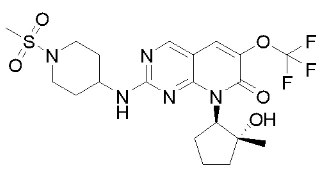
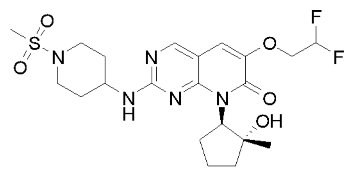
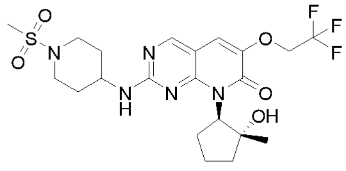

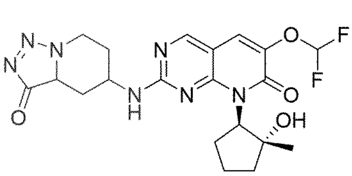
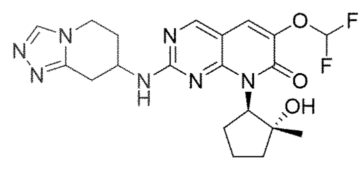
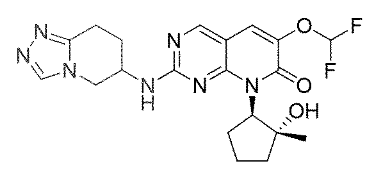

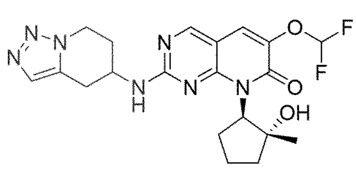
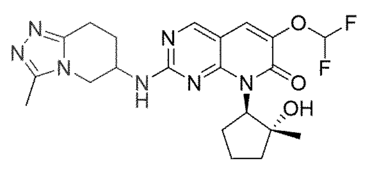
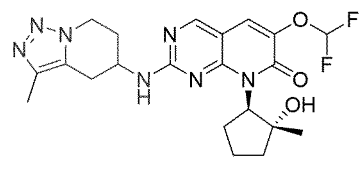

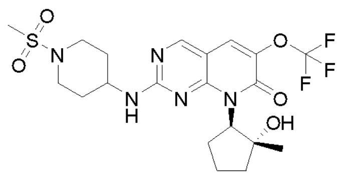
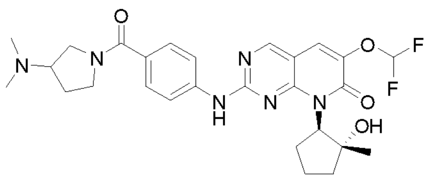
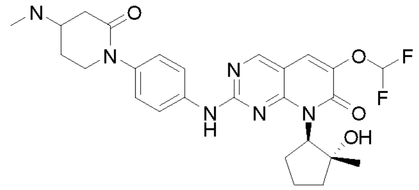

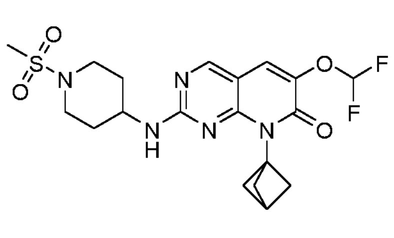
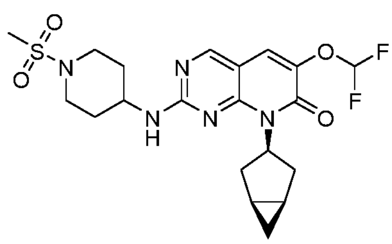
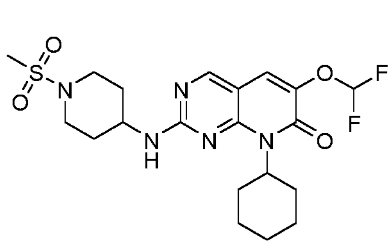

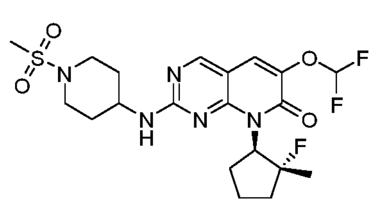
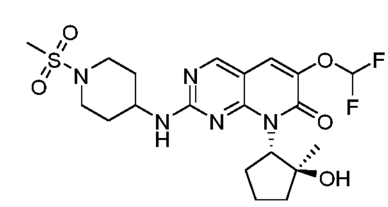
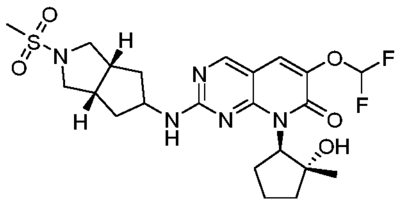
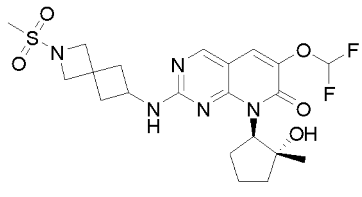
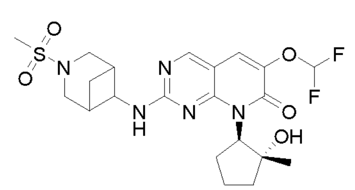
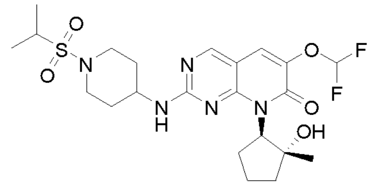
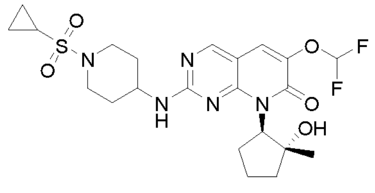
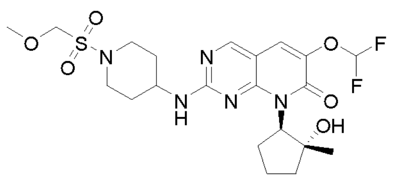
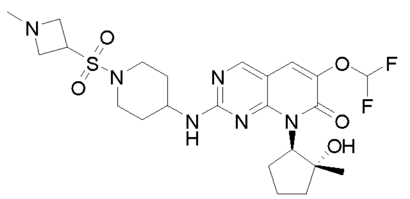
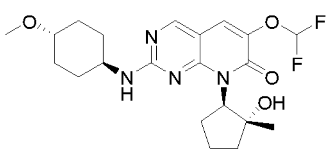
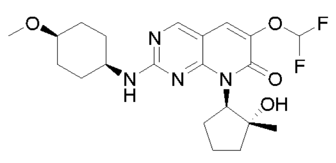
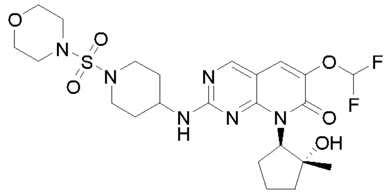
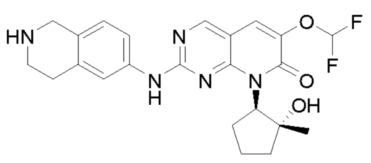
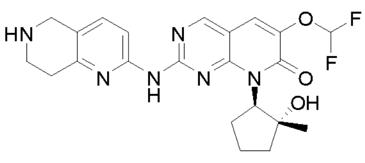
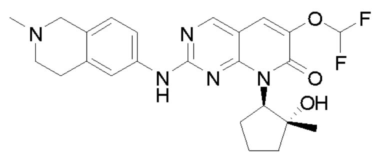
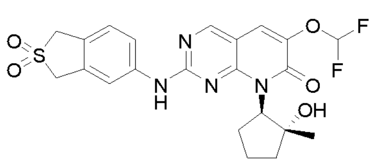
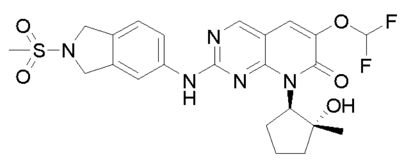
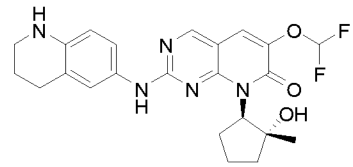
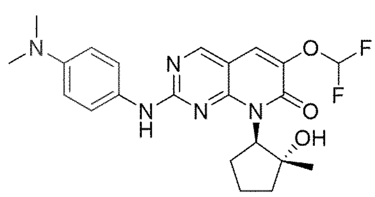
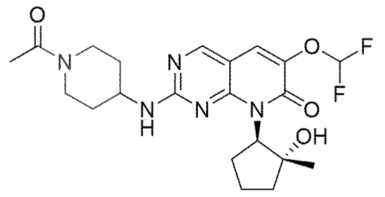
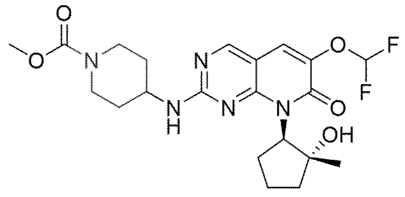

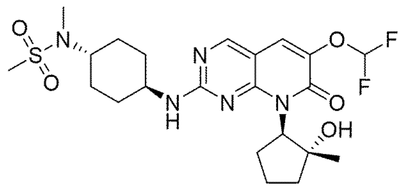
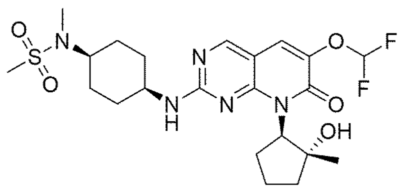
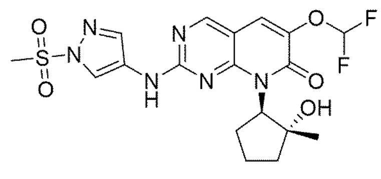

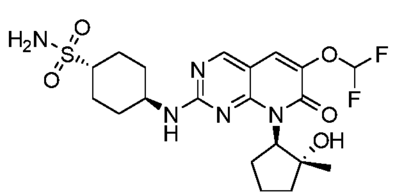
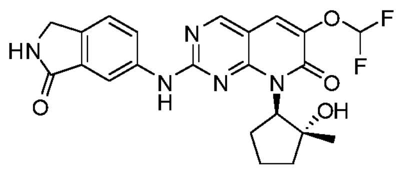
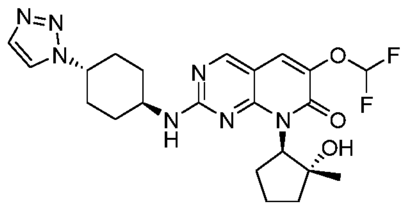

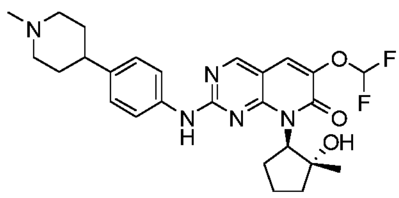
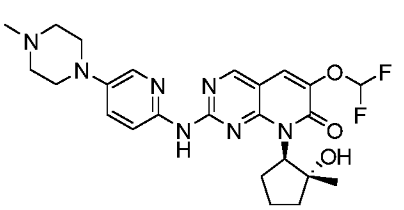
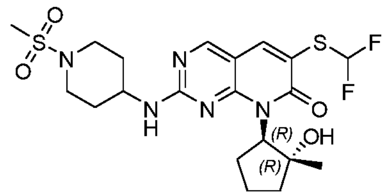

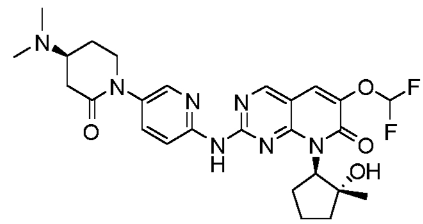
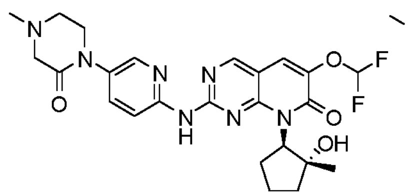
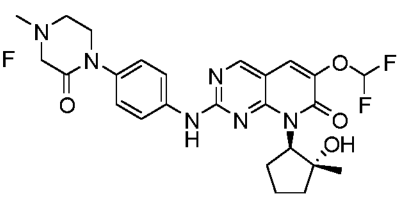

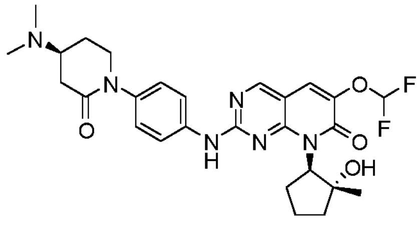
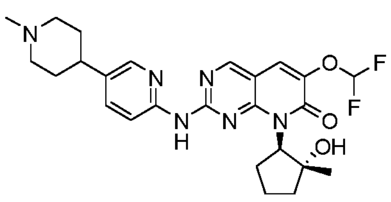
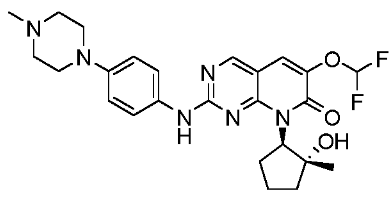

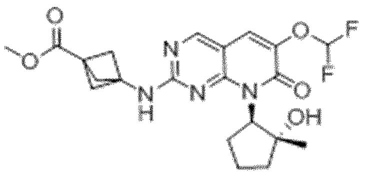
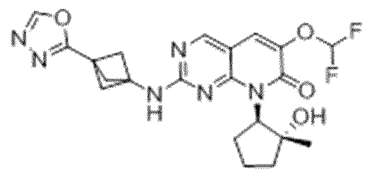
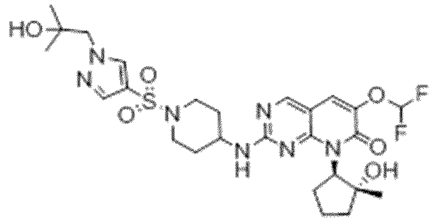

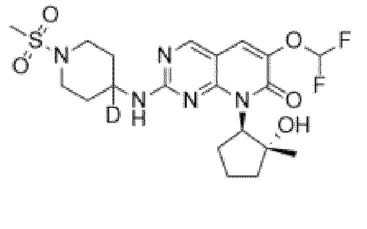
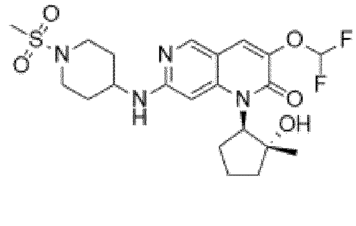
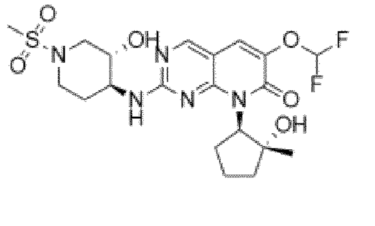

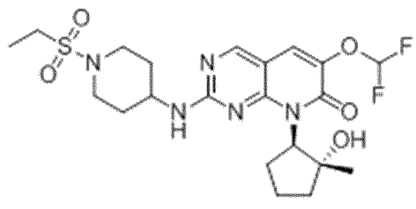
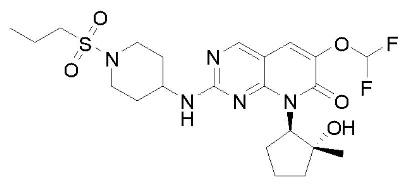
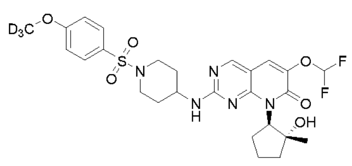
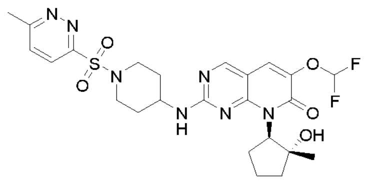
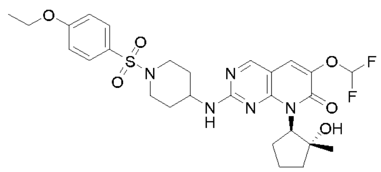
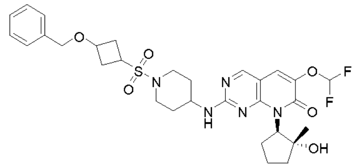
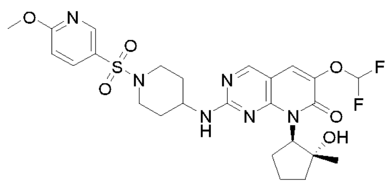
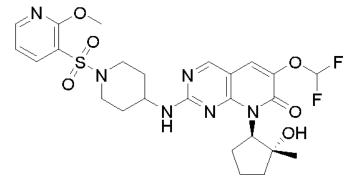
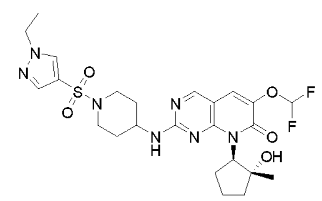
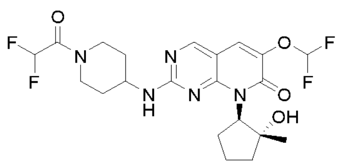
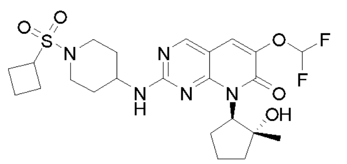
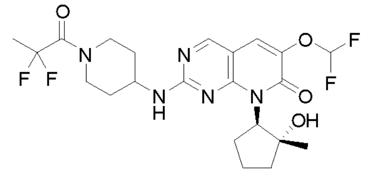
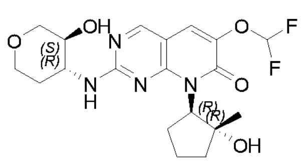
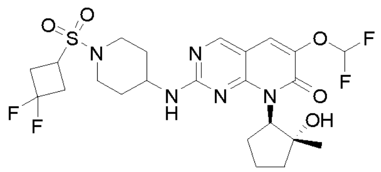
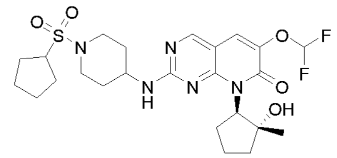
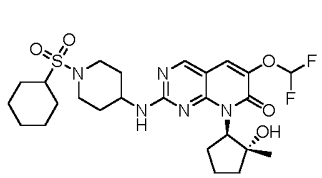
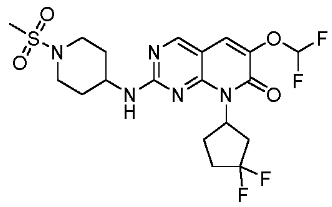
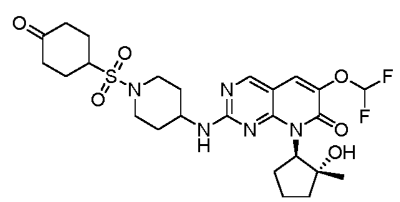
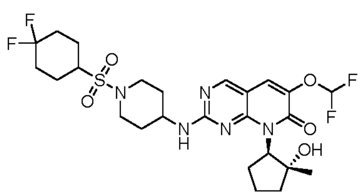
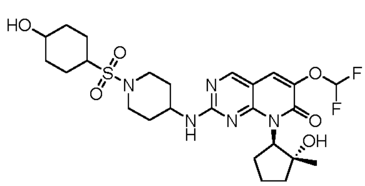
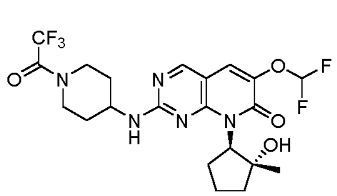

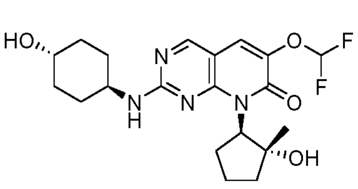
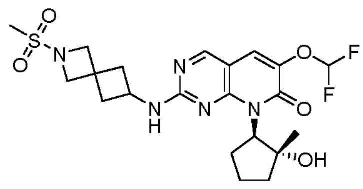
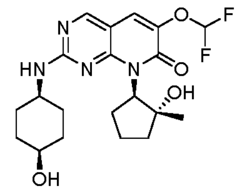

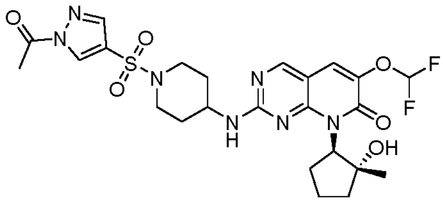
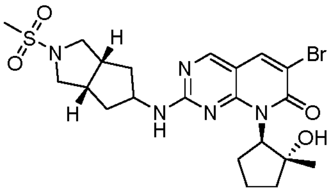
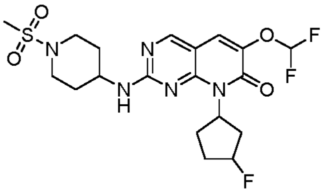

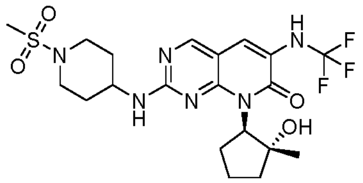
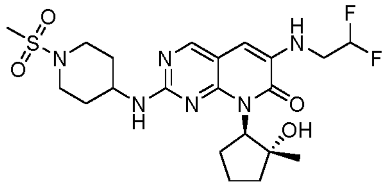
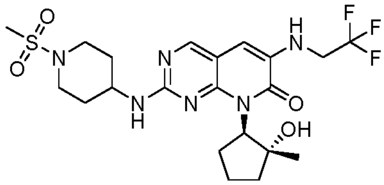

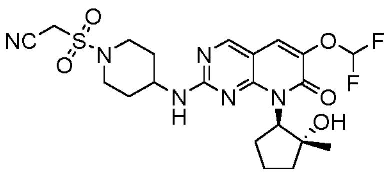
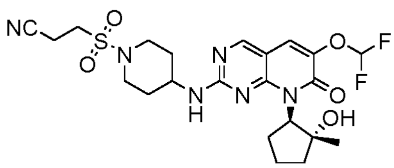
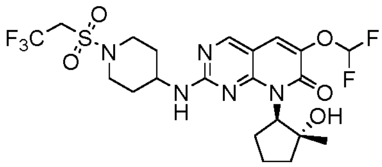
.

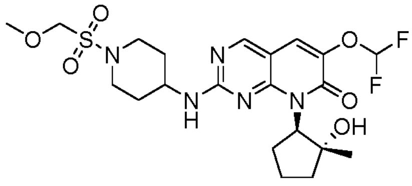
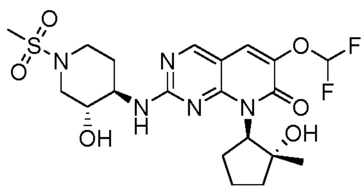
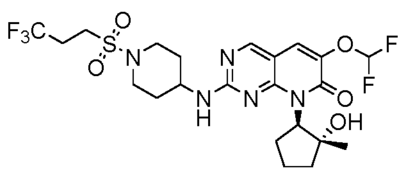
.

In another preferred embodiment, the compound has the structure shown in formula (III):

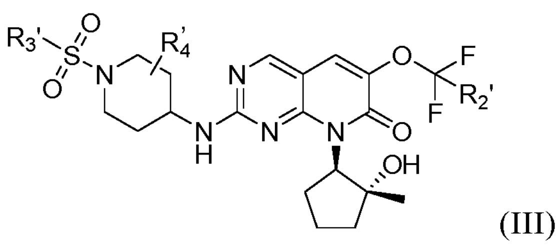

(III)

wherein,

$R_2$' is selected from the group consisting of: H, and deuterium;

$R_3$' is selected from the group consisting of: substituted or unsubstituted C1-C4 alkyl, and the "substituted" refers to being substituted by 1-3 substituents selected from the group consisting of: deuterium, halogen, OH, CN, =O, C1-C4 alkyl, C1-C4 alkoxy, C1-C4 haloalkoxy;

$R_4$' is selected from the group consisting of: H, deuterium, and OH;

In another preferred embodiment,

$R_2$' is H;

$R_3$' is selected from the group consisting of: substituted or unsubstituted C1-C4 alkyl, and the "substituted" refers to being substituted by 1-3 substituents selected from the group consisting of: halogen, CN, and C1-C4 alkoxy.

In another preferred embodiment, the compound is selected from the group consisting of:

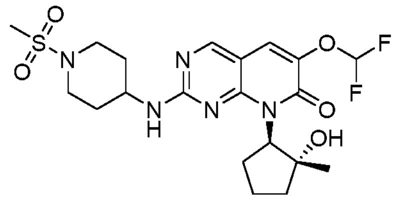
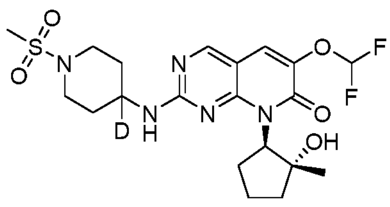
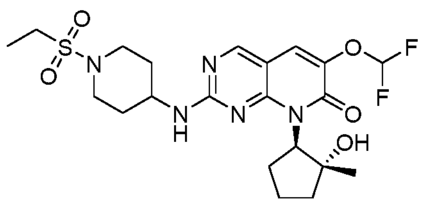

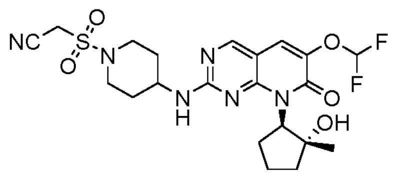
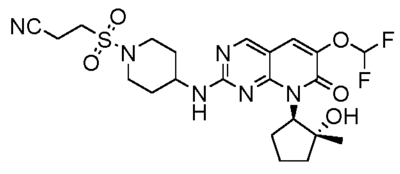
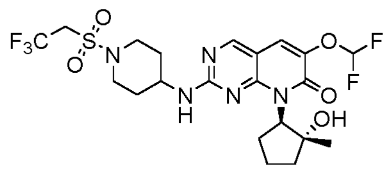

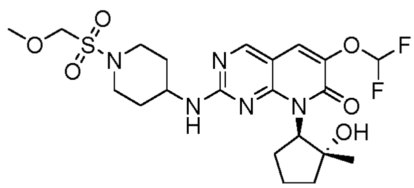
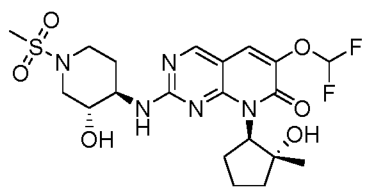
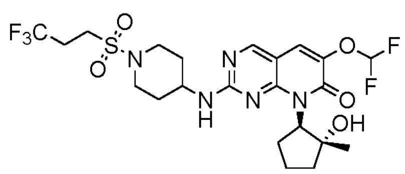.

In another preferred embodiment,

$R_1$ is selected from the group consisting of: unsubstituted or 1-4 $R_6$ substituted C3-C10 cycloalkyl, unsubstituted or 1-4 $R_6$ substituted 5-15 membered fused ring without or containing 1-3 heteroatoms selected from N, O and S, unsubstituted or 1-4 $R_6$ substituted 5-15 membered spiro ring without or containing 1-3 heteroatoms selected from N, O and S, unsubstituted or 1-4 $R_6$ substituted 5-15 membered bridged ring without or containing 1-3 heteroatoms selected from N, O and S, unsubstituted or 1-4 $R_6$ substituted 3-10 membered heterocycloalkyl containing 1-4 heteroatoms selected from N, O and S, unsubstituted or 1-4 $R_6$ substituted C6-C10 aryl, unsubstituted or 1-4 $R_6$ substituted 3-10 membered heteroaryl containing 1-5 heteroatoms selected from N, O and S, and unsubstituted or 1-4 $R_6$ substituted C1-C6 alkyl;

$R_2$ is selected from the group consisting of: H, F, OH, C1-C4 alkyl, C1-C4 haloalkyl, C1-C4 alkoxy, C1-C4 haloalkoxy, and $R_2$ can be connected with atoms on the ring to form a spiro ring, a bridged ring, or a fused ring structure;

$R_{3a}$ and $R_{3b}$ are independently selected from the group consisting of: H, F, OH, C1-C4 alkyl, C1-C4 haloalkyl, C1-C4 alkoxy, and C1-C4 haloalkoxy;

among them, each of the C1-C4 alkyl and C1-C4 haloalkyl in $R_2$, $R_{3a}$ and $R_{3b}$ is optionally substituted by halogen, OH, C1-C4 alkoxy or C1-C4 haloalkoxy;

$R_4$ is selected from the group consisting of: Br, substituted or unsubstituted 3-10 membered heterocycloalkyl containing 1-4 heteroatoms selected from N, O and S, $-OR_4$', $-SR_4$', $-NR_4$'$R_4$", wherein $R_4$' and $R_4$" are independently selected from the group consisting of: H, $COR_7$, substituted or unsubstituted C1-C6 alkyl, substituted or unsubstituted C3-C6 cycloalkyl, substituted or unsubstituted 3-6 membered heterocycloalkyl containing 1-4 heteroatoms selected from N, O and S; and the "substituted" refers to being substituted by 1-3 substituents selected from the group consisting of: deuterium, halogen, OH, CN, =O, C1-C4 alkyl, C1-C4 alkoxy, and C1-C4 haloalkoxy;

$R_5$ is selected from the group consisting of: H, halogen, C1-C2 alkyl, C1-C2 haloalkyl, C1-C4 alkoxy, and C1-C4 haloalkoxy;

each $R_6$ is independently selected from the group consisting of: H, deuterium, hydroxyl, halogen, cyano, =O, $COR_7$, $CO_2R_7$, $CONR_8R_9$, $CO_2NR_8R_9$, $SO_2R_7$, $SO_2NR_8R_9$, $NR_8SO_2R_7$, $NHSO_2NR_8R_9$,

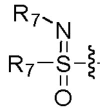

,

$NR_8R_9$, R‴ substituted or unsubstituted 3-10 membered heterocycloalkyl containing 1-3 heteroatoms selected from N, O and S, C1-C4 alkyl, C1-C4 haloalkyl, C1-C4 alkoxy, C1-C4 haloalkoxy, C3-C8 cycloalkyl, C3-C8 halocycloalkyl, C4-C10 spiro ring, C3-C10 fused ring, C4-C10 bridged ring, C1-C6 thioalkyl, C6-C10 aryl, and 3-10 membered heteroaryl containing 1-5 heteroatoms selected from N, O and S;

R‴ is selected from the group consisting of: =O, and NR'R";

each $R_7$ is independently selected from the group consisting of: C1-C4 alkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S), -L-(3-10 membered aryl), wherein the C1-C4 alkyl, C3-C8 cycloalkyl, 3-10 membered heterocycloalkyl, 3-10 membered heteroaryl, and 3-10 membered aryl are optionally substituted by 0 to 4 OH, halogen, C1-C4 alkyl, C1-C4 alkoxy or C1-C4 haloalkoxy;

$R_8$ and $R_9$ are independently selected from the group consisting of: H, C1-C4 alkyl, C1-C4 haloalkyl, C3-C8 cycloalkyl, -L-(C3-C8 cycloalkyl), -L-(3-10 membered heterocycloalkyl containing 1-4 heteroatoms selected from N, O and S), and -L-(3-10 membered heteroaryl containing 1-5 heteroatoms selected from N, O and S); or

$R_8$ and $R_9$ together with the N to which they are attached can form a substituted or unsubstituted 4-6 membered heterocyclyl, and the "substituted" refers to being substituted by 1-3 substituents selected from the group consisting of: =O, NR'R", and C1-C6 alkyl;

each R' and R" is independently selected from the group consisting of: H, and C1-C4 alkyl;

each L is independently a bond or C1-C4 alkylene, and the C1-C4 alkylene is optionally substituted by OH, C1-C4 alkoxy or C1-C4 haloalkoxy;

p is selected from the group consisting of: 0, 1, 2, 3 and 4;

r is selected from the group consisting of: 0, 1, 2, 3 and 4.

In another preferred embodiment, $R_1$ is selected from the group consisting of: unsubstituted or 1-4 $R_6$ substituted C3-C10 cycloalkyl, unsubstituted or 1-4 $R_6$ substituted 5-15 membered fused ring without or containing 1-3 heteroatoms selected from N, O and S, unsubstituted or 1-4 $R_6$ substituted 5-15 membered spiro ring without or containing 1-3 heteroatoms selected from N, O and S, unsubstituted or 1-4 $R_6$ substituted 5-15 membered bridged ring without or containing 1-3 heteroatoms selected from N, O and S, unsubstituted or 1-4 $R_6$ substituted C6-C10 aryl, and unsubstituted or 1-4 $R_6$ substituted 3-10 membered heteroaryl containing 1-5 heteroatoms selected from N, O and S;

$R_2$ is selected from the group consisting of: H, and F;

$R_{3a}$ and $R_{3b}$ are independently selected from the group consisting of: H, F, OH, and C1-C4 alkyl;

among them, each of the C1-C4 alkyl and C1-C4 haloalkyl in $R_2$, $R_{3a}$ and $R_{3b}$ is optionally substituted by halogen, OH, C1-C4 alkoxy or C1-C4 haloalkoxy;

$R_4$ is selected from the group consisting of: substituted or unsubstituted 3-10 membered heterocycloalkyl containing 1-4 heteroatoms selected from N, O and S, and $-NR_4'R_4''$, wherein $R_4'$ and $R_4''$ are independently selected from the group consisting of: H, substituted or unsubstituted C1-C6 alkyl, and the "substituted" refers to being substituted by 1-3 substituents selected from the group consisting of: deuterium, halogen, and C1-C4 alkyl;

$R_5$ is selected from the group consisting of: H, and C1-C2 alkyl;

each $R_6$ is independently selected from the group consisting of H, deuterium, hydroxyl, halogen, cyano, =O, $COR_7$, $CO_2R_7$, $CONR_8R_9$, $SO_2R_7$, $SO_2NR_8R_9$, $NR_8SO_2R_7$, $NHSO_2NR_8R_9$, $NR_8R_9$, R‴ substituted or unsubstituted 3-10 membered heterocycloalkyl containing 1-3 heteroatoms selected from N, O and S, C1-C4 alkyl, C1-C4 haloalkyl, C1-C4 alkoxy, C1-C4 haloalkoxy, C3-C8 cycloalkyl, C3-C8 halocycloalkyl, C4-C10 spiro ring, C3-C10 fused ring, C4-C10 bridged ring, C1-C6 thioalkyl, C6-C10 aryl, and 3-10 membered heteroaryl containing 1-5 heteroatoms selected from N, O and S;

R‴ is selected from the group consisting of: =O and NR'R";

each $R_7$ is independently selected from the group consisting of: C1-C4 alkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S), -L-(3-10 membered aryl), wherein the C1-C4 alkyl, C3-C8 cycloalkyl, 3-10 membered heterocycloalkyl, 3-10 membered heteroaryl, 3-10 membered aryl are optionally substituted by 0 to 4 OH, halogen, C1-C4 alkyl, C1-C4 alkoxy or C1-C4 haloalkoxy;

$R_8$ and $R_9$ are independently selected from the group consisting of: H, C1-C4 alkyl, C1-C4 haloalkyl, C3-C8 cycloalkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S); or

$R_8$ and $R_9$ together with the N to which they are attached can form a substituted or unsubstituted 4-6 membered heterocyclyl, and the "substituted" refers to being substituted by 1-3 substituents selected from the group consisting of: =O, NR'R", and C1-C6 alkyl;

each R' and R" is independently selected from the group consisting of: H, and C1-C4 alkyl;

each L is independently a bond or C1-C4 alkylene, and the C1-C4 alkylene is optionally substituted by OH, C1-C4 alkoxy or C1-C4 haloalkoxy;

p is selected from the group consisting of: 0, 1, 2, 3 and 4;

r is selected from the group consisting of: 0, 1, 2, 3 and 4.

In another preferred embodiment,

$R_1$ is selected from the group consisting of:

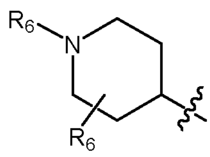, 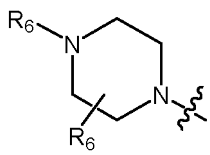,

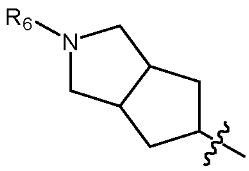, 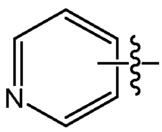, 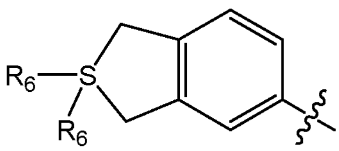, 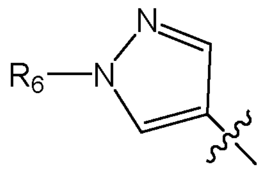,

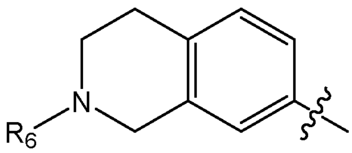, 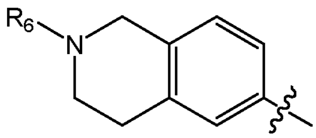, 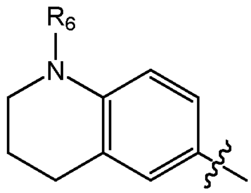, 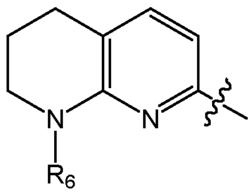,

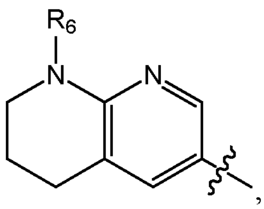, 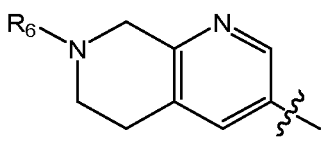, 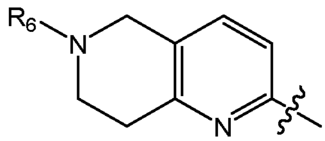, 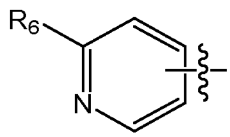,

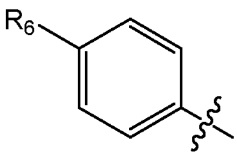, 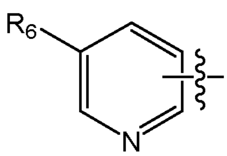, 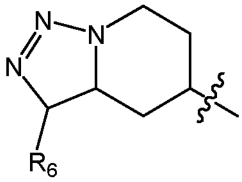, 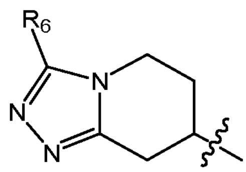,

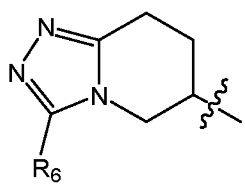, 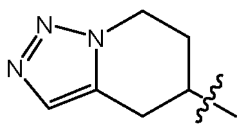, 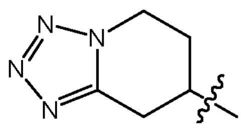, 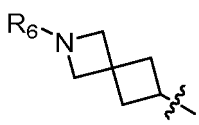, 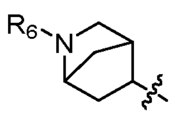,

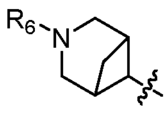, 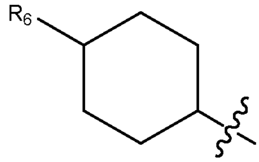, and 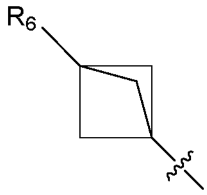;

$R_4$ is selected from the group consisting of: Br, substituted or unsubstituted 3-10 membered heterocycloalkyl containing 1-4 heteroatoms selected from N, O and S, substituted or unsubstituted C1-C4 alkoxy, substituted or unsubstituted C1-C4 alkylthio, substituted or unsubstituted C1-C4 alkylamino;
$R_6$ is as defined in claim 1.

In another preferred embodiment, $R_1$ is selected from the group consisting of:

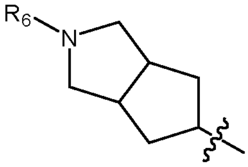, 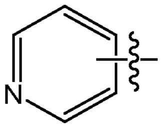, 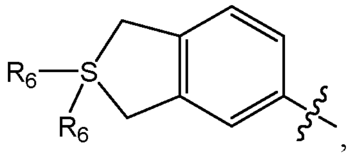, 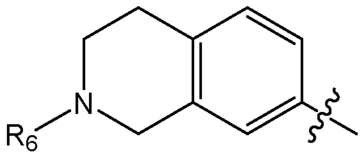,

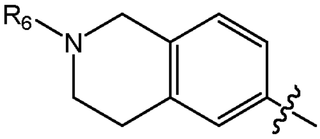, 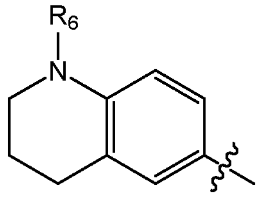, 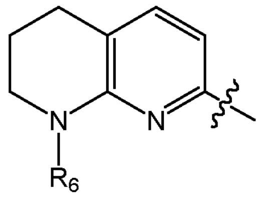, 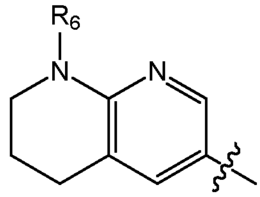,

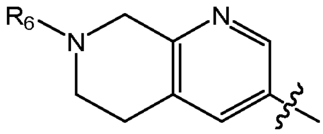, 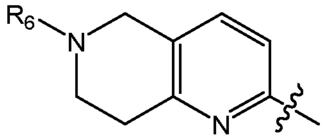, 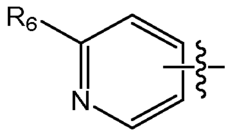, 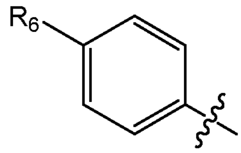,

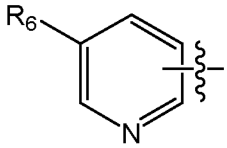, 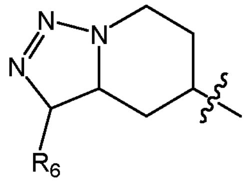, 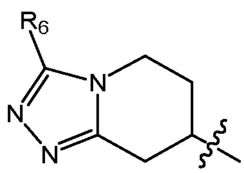, 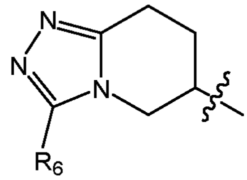,

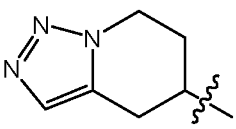, 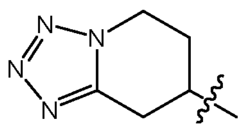, 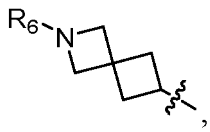, , 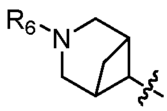, 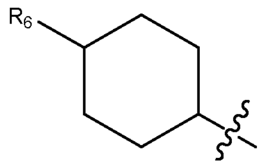,

and

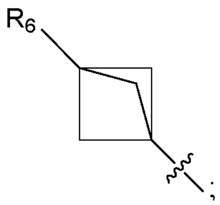;

$R_4$ is selected from the group consisting of: substituted or unsubstituted 3-10 membered heterocycloalkyl containing 1-4 heteroatoms selected from N, O and S, substituted or unsubstituted C1-C4 alkoxy, and substituted or unsubstituted C1-C4 alkylamino;

$R_6$ is as defined in claim 1.

In another preferred embodiment,

each $R_6$ is independently selected from the group consisting of: H, deuterium, hydroxyl, halogen, cyano, =O, $COR_7$, $CO_2R_7$, $CONR_8R_9$, $CO_2NR_8R_9$, $SO_2R_7$, $SO_2NR_8R_9$, $NR_8SO_2R_7$, $NHSO_2NR_8R_9$, $NR_8R_9$, R‴ substituted or unsubstituted 3-10 membered heterocycloalkyl containing 1-3 heteroatoms selected from N, O and S, C1-C4 alkyl, C1-C4 haloalkyl, C1-C4 alkoxy, C1-C4 haloalkoxy, C3-C8 cycloalkyl, and C3-C8 halocycloalkyl;

R‴ is selected from the group consisting of: =O, NR'R", C1-C4 alkyl, and C1-C4 haloalkyl;

$R_7$ is selected from the group consisting of: C1-C4 alkyl, and C1-C4 haloalkyl;

$R_8$ and $R_9$ are independently selected from the group consisting of: H, C1-C4 alkyl, C1-C4 haloalkyl, and C3-C8 cycloalkyl; or

$R_8$ and $R_9$ together with the N to which they are attached can form a substituted or unsubstituted 4-6 membered heterocyclyl, and the "substituted" refers to being substituted by 1-3 substituents selected from the group consisting of: =O, NR'R", and C1-C6 alkyl;

R' and R" are independently selected from the group consisting of: H, and C1-C4 alkyl.

In another preferred embodiment, p is 0, and r is 2.

In another preferred embodiment, $R_4$ is selected from the group consisting of: Br,

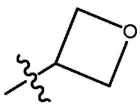, 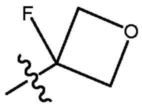, 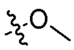, 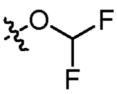, 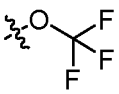, 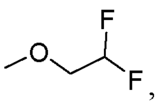, 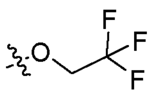, 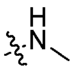,

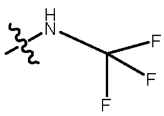, 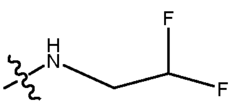, 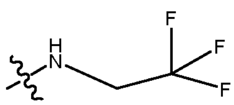, 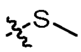, 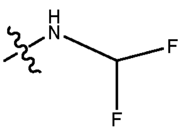, and 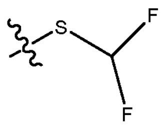.

In another preferred embodiment, the compound is selected from the group consisting of:

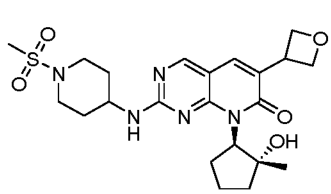 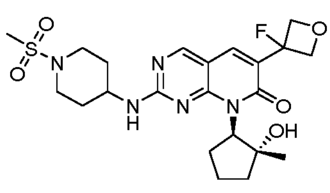 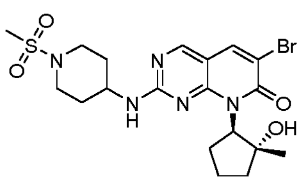

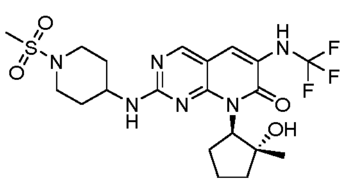 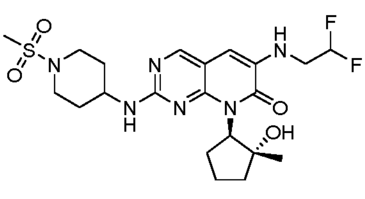 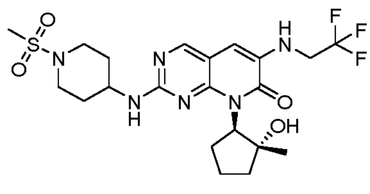

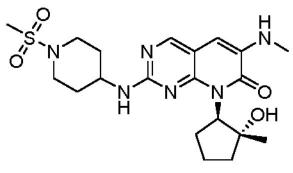
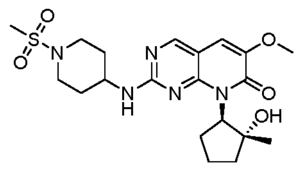
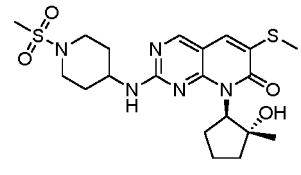

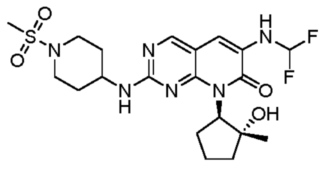
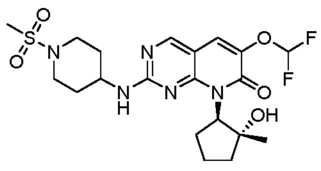
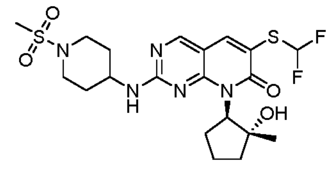

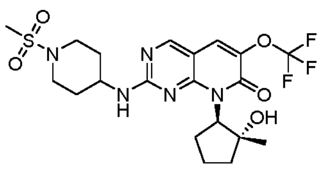
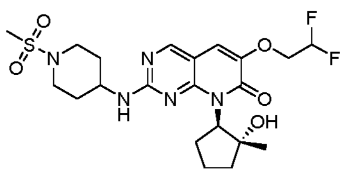
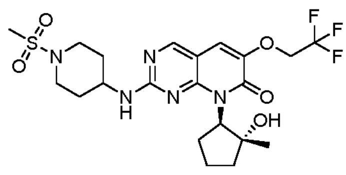

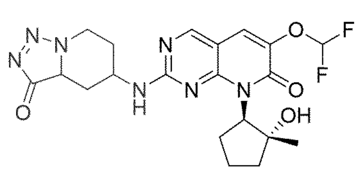
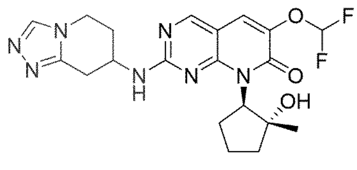
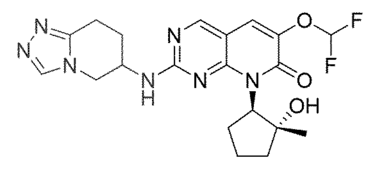

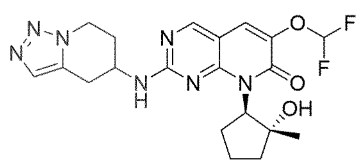
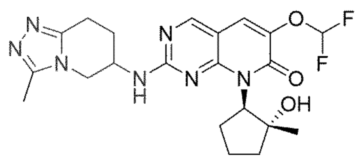
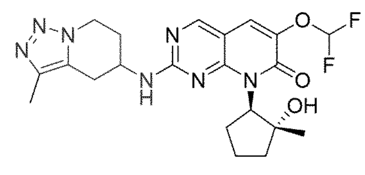

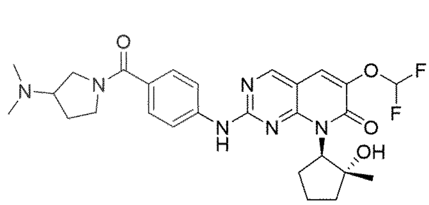
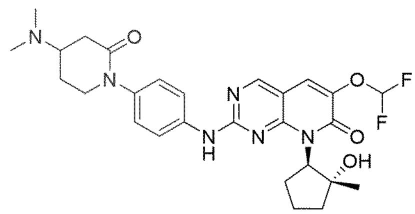
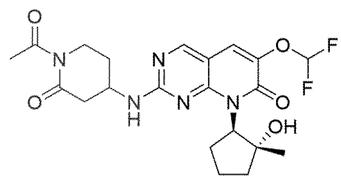

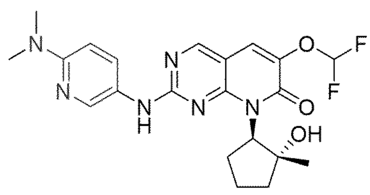
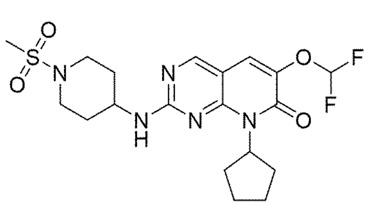
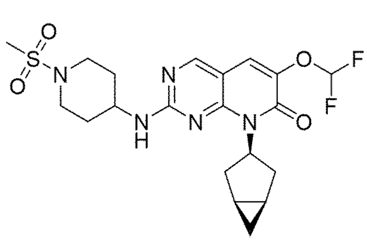

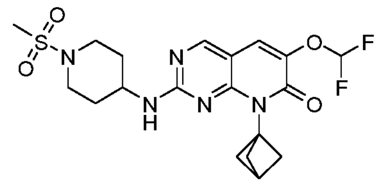
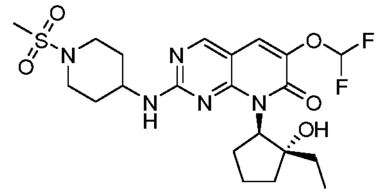
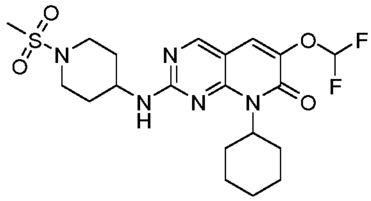

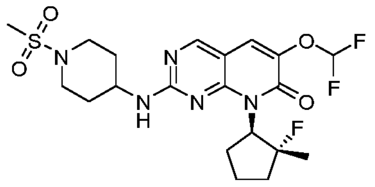
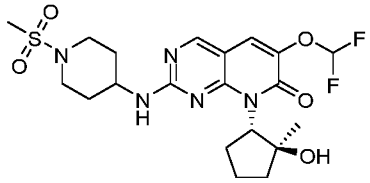
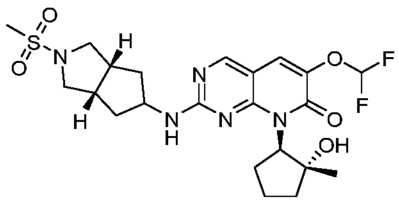

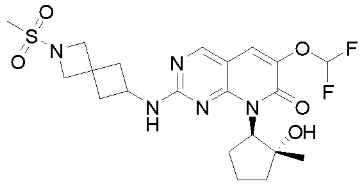
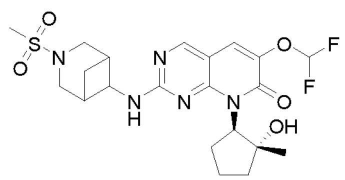
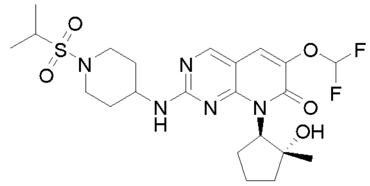

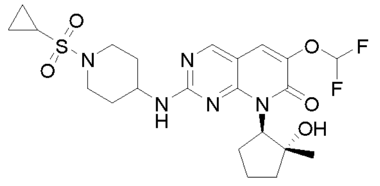
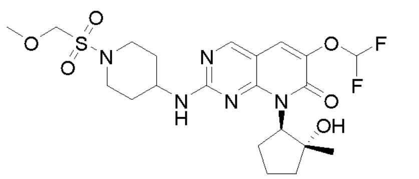
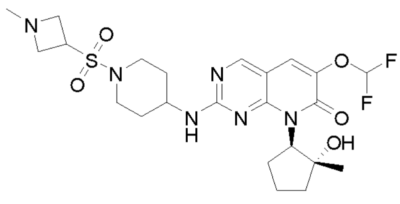

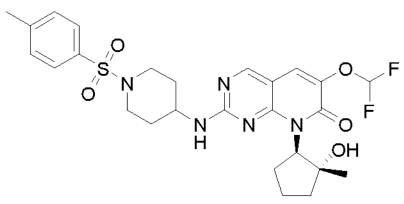
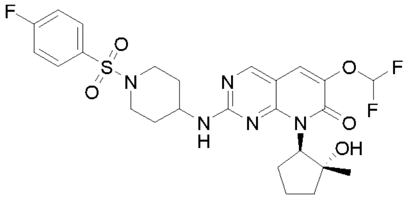
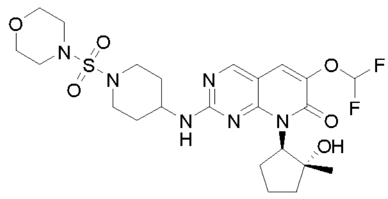

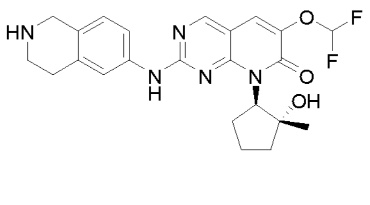
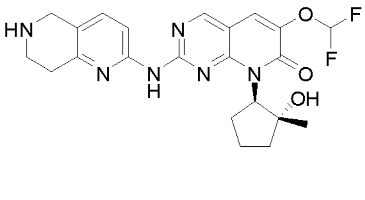
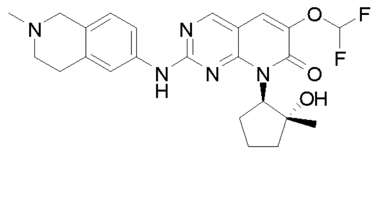

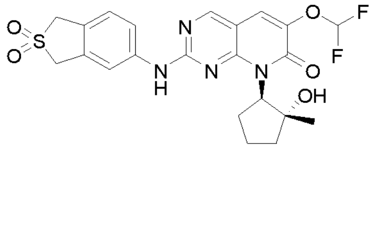
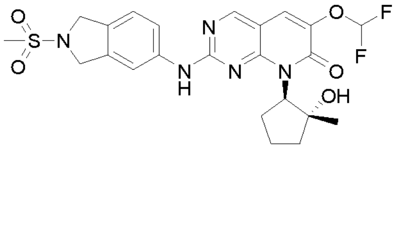
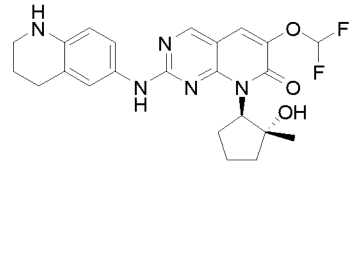

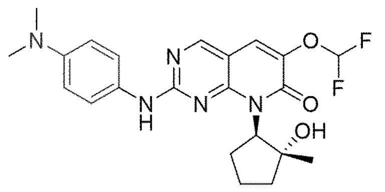
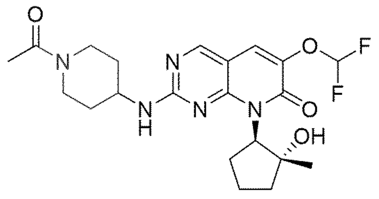
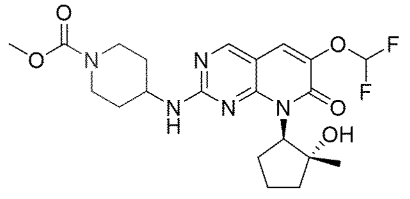
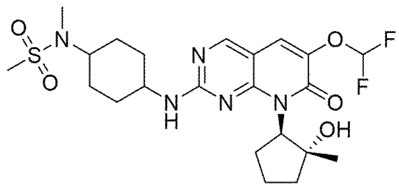
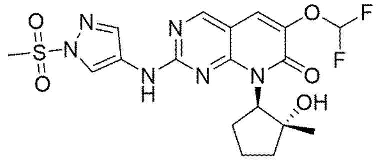
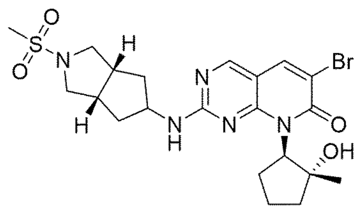
.

In another preferred embodiment, the pharmaceutically acceptable salt is an inorganic acid salt or an organic acid salt;

the inorganic acid salt is selected from the group consisting of hydrochloride, hydrobromide, hydroiodide, sulfate, bisulfate, nitrate, phosphate, and acid phosphate;
the organic acid salt is selected from the group consisting of formate, acetate, trifluoroacetate, propionate, pyruvate, glycolate, oxalate, malonate, fumarate, maleate, lactate, malate, citrate, tartrate, methanesulfonate, ethanesulfonate, benzenesulfonate, salicylate, picrate, glutamate, ascorbate, camphorate, camphor sulfonate, and camphorsulfonate.

The second aspect of the present invention provides a pharmaceutical composition comprising a therapeutically effective amount of the compound according to the first aspect of the present invention, or a pharmaceutically acceptable salt, a stereoisomer, a solvate or a prodrug thereof, and a pharmaceutically acceptable carrier.
The third aspect of the present invention provides a use of the compound according to the first aspect of the present invention, or a pharmaceutically acceptable salt, a stereoisomer, a solvate or a prodrug thereof in the preparation of CDK2/4/6 kinases inhibitor drugs.
The fourth aspect of the present invention provides a use of the compound according to the first aspect of the present invention, or a pharmaceutically acceptable salt, a stereoisomer, a solvate or a prodrug thereof in the preparation of drugs for regulating CDK kinase activity or treating CDK-related diseases.
In another preferred embodiment, the CDK-related disease is cancer or tumor.
In another preferred embodiment, the cancer or tumor is selected from the group consisting of breast cancer, ovarian cancer, bladder cancer, uterine cancer, lung cancer, colorectal cancer, prostate cancer, pancreatic cancer, gastric cancer, thyroid cancer, esophagus cancer, kidney cancer, liver cancer, head and neck cancer, glioblastoma, mantle cell lymphoma (MCL), chronic myeloid leukemia (CML) and acute myeloid leukemia (AML).
In another preferred embodiment, the lung cancer is non-small cell lung cancer.
It should be understood that within the scope of the present invention, the above-mentioned technical features of the present invention and the technical features specifically described in the following (such as Examples) can be combined with each other to form a new or preferred technical solution. Limited to space, they will not repeated herein.

## Description of the Drawings

Figure 1 is the experimental results of the xMCF-7_Palbo-R xenograft tumor model.
Figure 2 is the experimental results of the OVCAR-3 xenograft tumor model.
Figure 3 is the experimental results of the MV4-11 xenograft tumor model.

## Detailed Description of the Invention

After long-term and in-depth research, the present inventors unexpectedly prepared a compound with excellent CDK kinase inhibitory activity and preparation method therefor. On this basis, the inventors have completed the present invention.

## Terms

In the present invention, unless otherwise specified, the terms used herein have the ordinary meanings known to those skilled in the art.

In the present invention, the term "halogen" refers to F, Cl, Br or I.

In the present invention, "C1-C6 alkyl" refers to a linear or branched alkyl group including 1-6 carbon atoms, such as methyl, ethyl, propyl, isopropyl, butyl, isobutyl, tert-butyl, neopentyl, ter-pentyl, etc. The terms "C1-C4 alkyl", "C1-C2 alkyl" have similar meanings.

In the present invention, the term "C2-C6 alkenyl" refers to a linear or branched alkenyl group with 2-6 carbon atoms containing a double bond, including without limitation ethenyl, propenyl, butenyl, isobutenyl, pentenyl and hexenyl, etc.

In the present invention, the term "C2-C6 alkynyl" refers to a linearor branched alkynyl group with 2-6 carbon atoms containing a triple bond, including without limitation ethynyl, propynyl, butynyl, isobutynyl, pentynyl and hexynyl, etc.

In the present invention, the term "C3-C8 cycloalkyl" refers to a cyclic alkyl having 3-8 carbon atoms on the ring, including without limitation cyclopropyl, cyclobutyl, cyclopentyl, cyclohexyl, cycloheptyl, cyclooctyl, etc. The terms "C3-C10 cycloalkyl", "C3-C6 cycloalkyl" have similar meanings.

In the present invention, the term "C1-C6 alkoxy" refers to a linear or branched alkoxy group with 1-6 carbon atoms, including without limitation methoxy, ethoxy, propoxy, isopropoxy and butoxy, etc. preferably C1-C4 alkoxy.

In the present invention, the term "heterocycloalkyl" is a 4-8 membered heterocycloalkyl containing 1, 2 or 3 heteroatoms selected from N, O and S, including (but not limited to) the following groups:

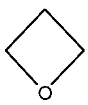, 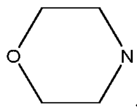, 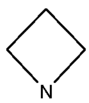, 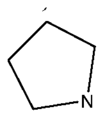, 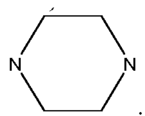.

The term "3-10 membered heterocycloalkyl containing 1-4 heteroatoms selected from N, O and S" has a similar meaning.

In the present invention, the term "aromatic ring" or "aryl" has the same meaning, preferably "C6-C10 aryl". The term "C6-C10 aryl" refers to an aromatic cyclic group having 6-10 carbon atoms without heteroatoms on the ring, such as phenyl, naphthyl and the like. Similarly, the term "3-10 membered aryl" refers to an aromatic cyclic group having 3-10 carbon atoms without heteroatoms on the ring, such as phenyl, naphthyl and the like. The term "6-10 membered aryl" has a similar meaning.

In the present invention, the term "heteroaromatic ring" or "heteroaryl" has the same meaning and refers to a heteroaromatic group containing one to more heteroatoms. For example, "3-10 membered heteroaryl" refers to an aromatic heterocyclic ring containing 1 to 4 heteroatoms selected from oxygen, sulfur and nitrogen and 3 to 10 carbon atoms. Non-limiting examples include: furyl, thienyl, pyridyl, pyrazolyl, pyrrolyl, N-alkylpyrrolyl, pyrimidinyl, pyrazinyl, imidazolyl, tetrazolyl, and the like. The heteroaryl ring may be fused to a ring of an aryl, heterocyclyl or cycloalkyl, wherein the ring bonded to the parent structure is a heteroaryl ring. Heteroaryl can be optionally substituted or unsubstituted.

In the present invention, the term "halo" refers to substituted by halogen.

In the present invention, the term "substituted" means that one or more hydrogen atoms on a specific group are substituted by a specific substituent. The specific substituents are the corresponding substituents described above, or the substituents present in each example. Unless otherwise specified, a substituted group may have a substituent selected from a specific group at any substitutable position, and the substituents may be the same or different at each position. Those skilled in the art will understand that the combinations of substituents contemplated by this invention are those that are stable or chemically feasible. The substituents are for example (but not limited to): halogen, hydroxyl, carboxyl (-COOH), C1-C6 alkyl, C2-C6 alkenyl, C2-C6 alkynyl, C3-C8 cycloalkyl, 3- to 12-membered heterocyclyl, aryl, heteroaryl, C1-C8 aldehyde, C2-C10 acyl, C2-C10 ester group, amino, C1-C6 alkoxy, C1-C10 sulfonyl, etc.

In the present invention, the term 1-6 means 1, 2, 3, 4, 5 or 6. Other similar terms have similar meanings.

## Compound

The present invention provides a compound of formula (I), or a pharmaceutically acceptable salt, a stereoisomer, a solvate or a prodrug thereof,

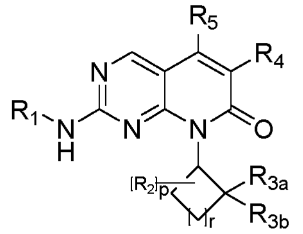

(I)

wherein, the definition of each group is as described above.

In another preferred embodiment, in the compound, any one of $R_1$, $R_2$, $R_{3a}$, $R_{3b}$, $R_4$, $R_5$, $R_6$, $R_7$, $R_8$, $R_9$, L, R', R", R‴, p and r is independently the corresponding group in the specific compound of the present invention.

More specifically, the present invention provides compounds of formula (II) or (III),

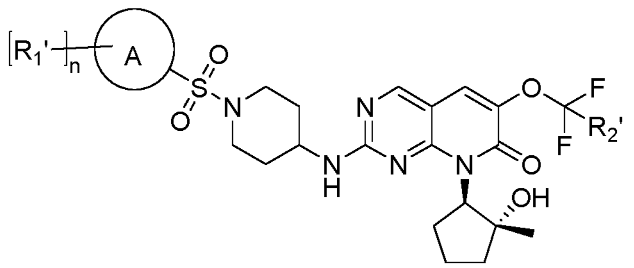

(II)

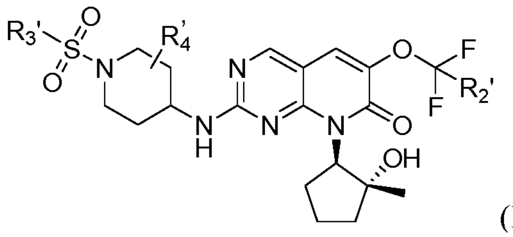

(III)

wherein, the definition of each group is as described above.

In another preferred embodiment, in the compound, any one of $R_1$', $R_2$', Ring A, $R_{31}$, $R_{41}$, $R_{51}$, L', and n is independently the corresponding group in the specific compound of the present invention.

In another preferred embodiment, the compound is preferably the compound prepared in the Examples of the present invention.

As used herein, the term "pharmaceutically acceptable salt" refers to a salt of a compound of the present invention formed with an acid or a base which is suitable for use as a medicine. Pharmaceutically acceptable salts include inorganic salts and organic salts. A preferred class of salts are the salts of the compounds of the invention formed with acids. Acids suitable for forming salts include, but are not limited to: inorganic acids such as hydrochloric acid, hydrobromic acid, hydrofluoric acid, sulfuric acid, nitric acid, phosphoric acid; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethanesulfonic acid, p-toluenesulfonic acid, benzenesulfonic acid, naphthalenesulfonic acid, etc; amino acids such as proline, phenylalanine, aspartic acid, and glutamic acid.

Another preferred class of salts are the salts of the compounds of the present invention with bases, such as alkali metal salts (e.g. sodium salt or potassium salt), alkaline earth metal salts (e.g. magnesium salt or calcium salt), ammonium salts (e.g. lower alkanolammonium salts and other pharmaceutically acceptable amine salts), such as methylamine salts, ethylamine salts, propylamine salts, dimethylamine salts, trimethylamine salts, diethylamine salts, triethylamine salts, tert-butyl amine salts, ethylenediamine salts, hydroxyethylamine salts, dihydroxyethylamine salts, trihydroxyethylamine salts, and amine salts formed from morpholine, piperazine, and lysine, respectively.

The term "solvate" refers to a complex in a specific ratio formed by a compound of the present invention coordinates with solvent molecules.

The term "prodrug" includes a class of compound that itself may be biologically active or inactive, and when taken in an appropriate manner, it undergoes metabolism or chemical reactions in the human body to convert into compounds of formula (I), or a salt or solution of a compound of formula (I). The prodrugs include (but are not limited

to) carboxylates (ester), carbonates (ester), phosphates (ester), nitrates (ester), sulfates (ester), sulfone esters, sulfoxide esters, amino compounds, carbamates, azo compounds, phosphoramide, glucoside, ether, acetal of the compounds and other forms.

## Preparation method

The preparation method of the compound of formula (I) of the present invention is described in more detail below, but these specific methods do not constitute any limitation to the present invention. The compounds of the present invention can also be conveniently prepared by optionally combining various synthetic methods described in the specification or known in the art, and such combinations can be easily performed by those skilled in the art to which the present invention belongs.

Typically, the preparation process of the compounds of the present invention is shown in the examples of the present invention, and the raw materials and reagents used therein can be purchased through commercial channels unless otherwise specified.

## Pharmaceutical composition and mode of administration

Since the compound of the present invention has excellent antitumor activity, the compound of the present invention and its various crystal forms, pharmaceutically acceptable inorganic or organic salts, hydrates or solvates, and parmaceutical compositions containing the compound of the present invention as the main active ingredient can be used for the treatment, prevention and alleviation of tumor-related diseases.

The pharmaceutical composition of the present invention comprises the compound of the present invention or a pharmacologically acceptable salt thereof within a safe and effective amount range and a pharmaceutically acceptable excipient or carrier. Wherein, "safe and effective amount" refers to: the amount of the compound is sufficient to obviously improve the condition without causing severe side effects. Usually, the pharmaceutical composition contains 1-2000 mg of the compound of the present invention per dose, more preferably 10-1000 mg of the compound of the present invention per dose. Preferably, the "one dose" is a capsule or a tablet.

"Pharmaceutically acceptable carrier" refers to one or more compatible solid or liquid fillers or gel substances, which are suitable for human use and must have sufficient purity and low toxicity. "Compatibility" herein refers to the ability of components of a composition to blend with the compounds of the invention and with each other without significantly reducing the efficacy of the compounds. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, Magnesium stearate), calcium sulfate, vegetable oil (such as soybean oil, sesame oil, peanut oil, olive oil, etc.), polyols (such as propylene glycol, glycerin, mannitol, sorbitol, etc.), emulsifiers (such as Tween®), wetting agents (such as sodium dodecyl sulfate), colorants, flavoring agents, stabilizers, antioxidants, preservatives, non-thermal raw water, etc.

The pharmaceutical composition is injection, capsule, tablet, pill, powder or granule.

The mode of administration of the compounds or pharmaceutical compositions of the present invention are not particularly limited, and representative mode of administration include, but are not limited to, oral, intratumoral, rectal, parenteral (intravenous, intramuscular or subcutaneous), and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with the following ingredients: (a) filler or compatibilizer, such as starch, lactose, sucrose, glucose, Mannitol and silicic acid; (b) adhesives, such as hydroxymethyl cellulose, alginate, gelatin, polyvinylpyrrolidone, sucrose and gum arabic; (c) moisturizing agents, such as glycerol; (d) disintegrating agents, such as agar, calcium carbonate, potato starch or cassava starch, algic acid, certain complex silicates, and sodium carbonate; (e) slow solvent, such as paraffin; (f) absorption accelerators, such as quaternary amine compounds; (g) wetting agents, such as cetyl alcohol and glycerol monostearate; (h) adsorbents, such as kaolin; and (i) lubricants, such as talc, calcium stearate, magnesium stearate, solid polyethylene glycol, sodium dodecyl sulfate, or mixtures thereof. In capsules, tablets and pills, dosage forms may also contain buffer agent.

Solid dosage forms such as tablets, sugar pills, capsules and granules may be prepared using coating and shell materials such as casing and other materials well known in the art. They may comprise an opacifying agent, and the release of the active compound or compound in such a composition may be released in a delayed manner in a part of the digestive tract. Examples of embedding components that can be employed are polymeric substances and wax substances. If necessary, the active compound may also form microcapsule form with one or more of the excipients described above.

Liquid dosage forms for oral administration include pharmaceutically acceptable emulsions, solutions, suspensions, syrups or tinctures. In addition to the active compounds, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil, or mixtures thereof.

In addition to these inert diluents, the composition may also contain auxiliaries such as wetting agents, emulsifiers, suspending agents, sweeteners, flavoring agents and flavors.

In addition to the active compound, the suspension may comprise suspending agents, such as ethoxylated isooctadecanol, polyoxyethylene sorbitol and dehydrated sorbitol esters, microcrystalline cellulose, methanolic aluminum, agar, and the mixtures of these substances.

The composition for parenteral injection may comprise physiologically acceptable sterile aqueous or anhydrous solutions, dispersions, suspensions or emulsions, and sterile powders for redissolution into sterile injectable solutions or dispersions. Suitable aqueous and nonaqueous carriers, diluents, solvents, or excipients include water, ethanol, polyols, and suitable mixtures thereof.

Dosage forms of the compound of the invention for topical administration include ointments, powders, patches, propellants and inhalants. The active ingredient is mixed under sterile conditions with a physiologically acceptable carrier and any preservatives, buffers, or propellants as may be required if necessary.

The compound of the present invention can be administered alone or in combination with other pharmaceutically acceptable compounds (such as antineoplastic drugs).

The treatment method of the present invention can be used alone or in combination with other treatment methods or drugs.

When using the pharmaceutical composition, a safe and effective amount of the compound of the present invention is administered to mammals (such as humans) in need of treatment, where the dosage at the time of administration is the pharmaceutically considered effective dose, which is typically 1 to 2,000 mg per day, more preferably 50 to 1000mg per day for a 60 kg body weight human. Of course, the specific dosage should also consider the route of administration, the patient's health and other factors, which are within the skill range of skilled doctors.

Compared with the prior art, the present invention has the following main advantages:

(1) The compound has excellent CDK kinases inhibitory activity;
(2) The compound has excellent cytostatic activity;
(3) The compound has excellent efficacy *in vivo.*

The invention is further illustrated below in conjunction with specific examples. It is to be understood that these examples are intended to illustrate the invention only and not to limit the scope of the invention. The experimental methods that do not indicate specific conditions in the following examples usually follow the conventional conditions, such as those described in Sambrook et al., Molecular Cloning: A Laboratory Manual (New York: Cold Spring Harbor Laboratory Press, 1989), or the conditions suggested by the manufacturer. Unless otherwise specified, percentages and portions are calculated by weight.

Unless otherwise defined, all professional and scientific terms used herein have the same meanings as commonly familiar to those skilled in the art. In addition, any methods and materials similar or equivalent to those described can be applied to the method of the present invention. The preferred implementation methods and materials described herein are for demonstration purposes only.

**<u>Example synthesis route:</u>**

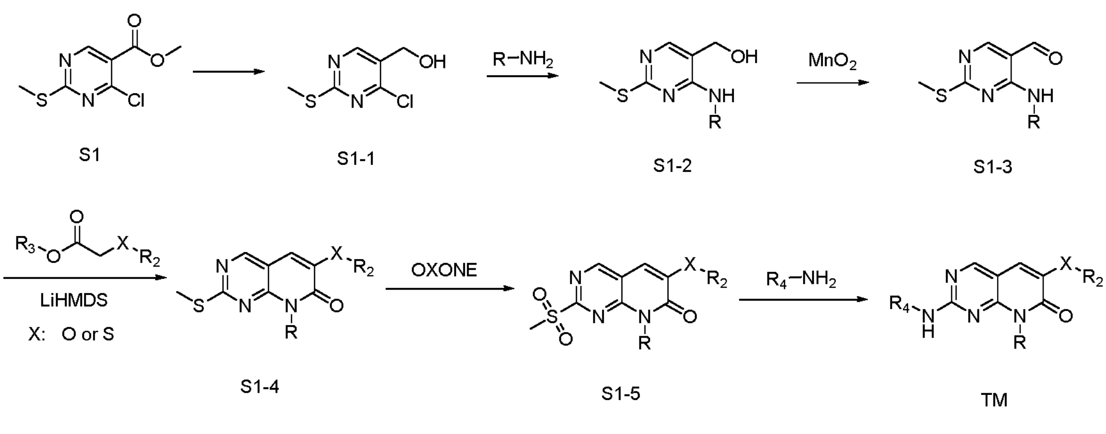


S1 S1-1 S1-2 S1-3

S1-4 S1-5 TM

The starting material S1 is reduced by lithium aluminum hydride to obtain the intermediate S1-1, and then nucleophilic substituted with various substituted primary amines to obtain the product S1-2, and then oxidized with manganese dioxide to obtain the aldehyde pyrimidine compound S1-3, and then subjected a cyclization reaction with acetate containing different substituents, followed by being oxidized to obtain sulfone-pyrimidinone intermediate S1-5, and the sulfone-pyrimidinone intermediate S1-5 reacted with substituted primary amine to obtain the final product.

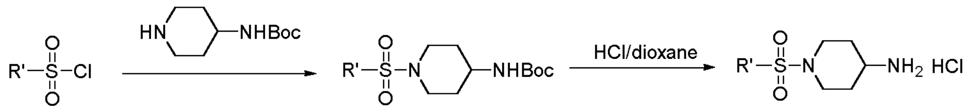


S2 S2-1 S2-3

Substituted sulfonyl chloride S2 is reacted with 4-Boc-aminopiperidine to obtain sulfonamide intermediate S2-1, and then deprotected to obtain intermediate S2-3.

**Preparation of intermediates:**

**Synthesis of intermediate int 1:**

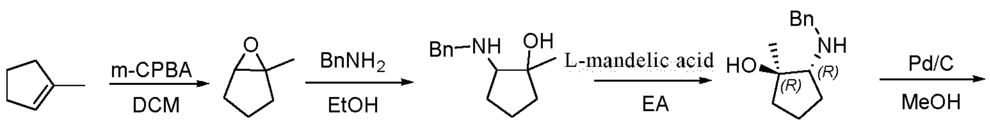


int1-1 int1-2 (+/-) int1-3 int1-4

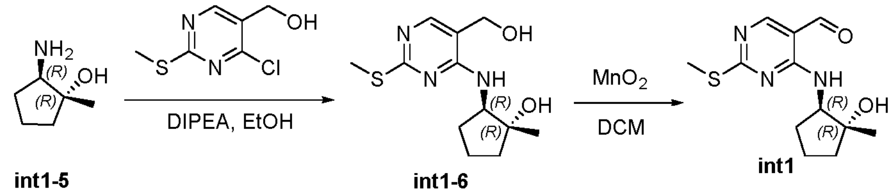


int1-5 int1-6 int1

1. Synthesis of int 1-2

1-Methylcyclopentene (57.0 g, 0.695 mol) and dichloromethane (1200 mL) were added to a 2000 mL single-necked flask, and m-chloroperoxybenzoic acid (211.0 g, 1.043 mol) was added at 0 °C, then the mixture was warmed to room temperture and reacted for 16 h. After the reaction was completed, the mixture was filtered, and the filter cake was washed twice with dichloromethane (100 ml x2), and the filtrate was washed three times with saturated sodium carbonate aqueous solution (300 ml x3), two to three times with saturated sodium bicarbonate aqueous solution (300 ml), and two to three times with saturated sodium chloride aqueous solution (300 ml x2). The organic phase was dried with anhydrous sodium sulfate, filtered, and then distilled under reduced pressure at controlled temperature below 5°C to obtain 79g of crude product, which was directly used in the next step.

2. Synthesis of int 1-3

Int1-2 (40.0 g, 0.347 mol), benzylamine (74.0 g, 0.694 mol) and ethanol (170 mL) were added to a 350 mL sealed tube, and the mixture was reacted at 90 °C for 40 h. The reaction mixture was then cooled to room temperature, concentrated, water (100 mL) was added, and the resulting mixture was extracted twice with ethyl acetate (150 mL x2), the organic phase was dried with anhydrous sodium sulfate, filtered, concentrated, and 35 g of product was obtained by column chromatography. 1H NMR (400 MHz,CDCl3) δ = 7.36-7.31 (m, 4H), 7.28-7.23 (m,1H), 3.91-3.85 (m, 1H), 3.79-3.74 (m, 1H), 2.86 (dd, J=7.8, 8.5Hz, 1H),2.12-2.03 (m, 1H), 1.75-1.53 (m, 5H), 1.37-1.27 (m, 1H), 1.22 (s, 3H).

3. Synthesis of int 1-4

Int1-3 (35.0 g, 0.17 mol) and ethyl acetate (350 mL) were added to a 2000 mL single-necked flask, under the condition of heated to slightly refluxing, a solution of L-mandelic acid (13.0 g, 0.085 mol) in ethyl acetate (200 mL) was added dropwise, after dropping, the temperature was then cooled to room temperature naturally, and the reaction system was stirred for 16 h. The reaction mixture was filtered, the filter cake was washed with cold ethyl acetate, added to water (150ml) and ethyl acetate (200mL), pH was adjusted to 1 with 4N hydrochloric acid aqueous solution, the liquid was separated, then the aqueous phase was adjusted to pH 11 with 6N sodium hydroxide aqueous solution, extracted with ethyl acetate (200mL x2), the organic phase was dried with anhydrous sodium sulfate, filtered, concentrated. The above operation repeated until the ee value was greater than 99%, and 8g of product with 99.3% ee was obtained. 1H NMR (400 MHz,CDCl3) $\delta$ = 7.38-7.30 (m, 4H), 7.27-7.23 (m, 1H), 3.94-3.75 (m, 2H), 2.88 (dd,J=7.8, 8.4Hz, 1H), 2.16-2.03 (m, 1H), 1.79-1.57 (m, 4H), 1.53-1.39 (m, 2H), 1.38-1.28 (m, 1H), 1.25 (s, 3H).The chiral HPLC analysis was performed on an Agilent LC 1260 using a Lux® 5$\mu$m i-Amylose-1, 4.6*250mm, S/N:H19-351585 column, which was heated to 30 °C and eluted with a mobile phase of A (0.1% diethylamine/hexane): B (0.1% diethylamine/isopropanol) = 95%:5% (flow rate 1 ml/min, detection wavelength of 254 nm) for 20 min, the retention time of the product was 7.659 min.

4. Synthesis of int 1-5

Int1-4 (18.7 g, 0.091 mol), Pd/C (2.0 g, 10%), and methanol (350 mL) were added to a 1000 mL single-necked flask, and gas was displaced 3 times with nitrogen, followed by 3 times with hydrogen. The reaction was carried out at room temperature for 16h. Then the resulting mixture was filtered, the filter cake was washed with methanol (30 mL x3) and concentrated to obtain 10 g of product, which was used directly in the next step. 1H NMR (400 MHz,CDCl3) $\delta$ = 3.03 (t, J=7.4 Hz, 1H), 2.19-2.01 (m, 1H), 1.83-1.58 (m, 4H), 1.42(s, 3H), 1.35-1.25 (m, 1H), 1.22 (s, 3H).

5. Synthesis of int 1-6

[4-Chloro-2-(methylthio)pyrimidin-5-yl]methanol (8.33 g, 43.83 mmol), int1-5 (4.8 g, 41.74 m mol), ethanol (100 mL) and N,N-diisopropylethylamine (16.15 g, 125.22 mmol) were added to a 250 mL single-necked flask. The temperature was warmed to 80°C and the reaction was carried out for 16 h. Then the resulting mixture was cooled to room temperature, concentrated, and 10.5 g of product was obtained by column chromatography with petroleum ether: ethyl acetate=5:1 to 1:1.

6. Synthesis of int 1

Int1-6 (10.5 g, 39.03 mmol), dichloromethane (300 mL) and manganese dioxide (39.95 g, 390.3 mmol) were added to a 500 mL single-necked flask, and the mixture was stirred at room temperature for 16h.Then the resulting mixture was filtered, the filter cake was washed with dichloromethane (30 mL x2) and concentrated to obtain 9.3g of product. $^1$H NMR (400 MHz,$CDCl_3$) $\delta$ = 9.73 (s, 1H), 8.66 (br s, 1H), 8.35 (s, 1H), 4.39 (ddd, J=6.5,8.2, 9.6 Hz, 1H), 4.16 (s, 1H), 2.57 (s, 3H), 2.33-2.22 (m, 1H), 2.03-1.92(m, 1H), 1.89-1.68 (m, 3H), 1.68-1.56 (m, 1H), 1.17 (s, 3H). MS:268 [M+H]+.

**Synthesis of intermediate int 2**

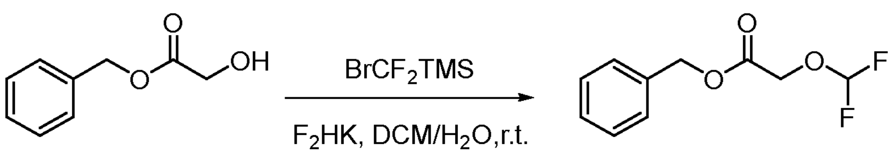


int2-1 int2

Int2-1 (63 g, 0.379 mol), dichloromethane/water (1:1) (38 mL) and potassium difluorohydride (29.6 g, 0.379 mol) were added to a 100 mL three-necked flask, and bromodifluoro(trimethylsilyl)methane (154 g, 0.758 mmol) was added dropwise with stirring at room temperature and the reaction was carried out for 3 h at room temperature. Then water (50 ml) was added under an ice bath and the resulting mixture was extracted with ethyl acetate (30 mL x2), the organic phases were combined, dried with anhydrous sodium sulfate, filtered, the organic phases were concentrated, and 31.5 g of product was obtained by column chromatography.

Synthesis of intermediate int 3

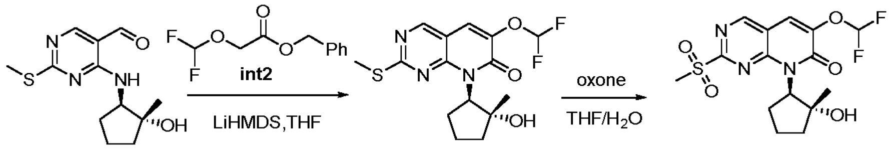

int1 int3-1 int3

1. Synthesis of int 3-1

Compound int2 (290 mg, 2.6eq) was dissolved in dry THF (5 ml), the atmosphere was displaced with nitrogen, then the temperature was cooled to -70°C, and lithium dimethylsilylammonium (1.0M, 2.06 ml, 4.0eq) was added dropwise, after dropping, and then the reaction was held at -70°C for 1 h. A solution of compound 1 (137.8 mg, 1.0eq) in tetrahydrofuran (3 ml) was then added dropwise, after dropping, and then the reaction was carried out at 40°C overnight. The reaction was monitored to completion by TLC, the temperature was then cooled down, and saturated aqueous ammonium chloride solution was added to the reaction system dropwise to quench, the resulting mixture was extracted with ethyl acetate (30 ml × 3), separated, the ethyl acetate phase was collected and dried with anhydrous sodium sulfate, filtered, spun-dried and passed through a column to obtain the product (90 mg).

2. Synthesis of int 3

Compound int3-1 (90mg, 1.0eq) was dissolved in a mixture of tetrahydrofuran and water, the temperature was then cooled to 0°C, and oxone (310mg, 2.0eq) was added, and the mixture was naturally warmed up to room temperature and reacted overnight. The reaction was monitored to completion, and the resulting mixture was filtered, water was added to the filtrate, and the resulting mixture was extracted with ethyl acetate, and then dried and spun-dried to obtain compound 3 (80mg).

**Synthesis of intermediate int 4**

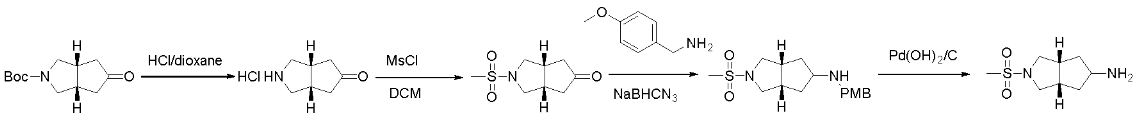

int4-1 int4-2 int4-3 int4-4 int4

1. Synthesis of int 4-2

Int3-1 (3.0 g, 13.33 mmol), 4M HCl/dioxane (30 mL) and dichloromethane (30 mL) were added to a 100 mL single-necked flask. The reaction was carried out for 2h at room temperature. The reaction solution was then concentrated to obtain 1.60 g of product.

2. Synthesis of int 4-3

Int3-2 (1.25 g, 10.0 mmol), 15% NaOH aqueous solution (10 mL) and tetrahydrofuran (20 mL) were added to a 100 mL single-necked flask, and methylsulfonyl chloride (1.72 g, 15.0 mmol) was added dropwise at 0 °C, then the mixture was reacted at room temperature for 2 h after dropping. Water (30 mL) was then added, the resulting mixture was extracted three times with ethyl acetate (20 mL), and the organic phases were combined, dried with anhydrous sodium sulfate, filtered, concentrated, and 800 mg of product was obtained by column chromatography with petroleum ether:ethyl acetate=5:1 to 1:1.

3. Synthesis of int 4-4

Int3-3 (800 mg, 3.94 mmol), sodium cyanoborohydride (347 mg, 5.52 mmol), glacial acetic acid (236 mg, 3.94

mmol), p-methoxybenzylamine (540 mg, 3.94 mmol) and 1,2-dichloroethane (10 mL) were added to a 50 mL single-necked flask. The reaction was carried out at room temperature for 3h. After the reaction was completed, saturated aqueous sodium bicarbonate was added to the reaction solution, the resulting mixture was extracted with dichloromethane, the organic phase was dried with anhydrous sodium sulfate, filtered, concentrated, and 790mg of product was obtained by column chromatography with petroleum ether:ethyl acetate=5:1 to 0:1.

4. Synthesis of int 4

Int 3-4 (790 mg, 2.44 mmol), $Pd(OH)_2/C$ (80 mg, 10%), glacial acetic acid (146 mg, 2.44 mmol) and methanol (20 mL) were added to a 50 mL single-necked flask, and gas was displaced 3 times with nitrogen, followed by 3 times with hydrogen. The reaction was carried out at 70 °C for 16h. Then the resulting mixture was filtered, the filter cake was washed with methanol (50 mL x3) and concentrated to obtain 350 mg of product.

## Synthesis of intermediate int 5

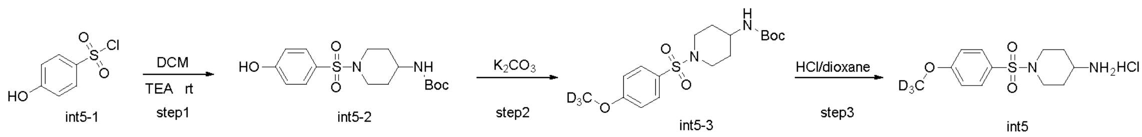


1. Synthesis of int 5-2

4-Boc-aminopiperidine (1.04g, 1.0eq), dichloromethane (10mL), and N,N-diisopropylethylamine (1.05g, 2.0eq) were added to a 100 mL single-necked flask, the temperature was cooled to 0 °C and a solution of int 5-1 (1.0g, 1.0eq) in dichloromethane (2mL) was added dropwise, and the mixture was naturally warmed up to room temperature and reacted for 3h. After the reaction was completed, water was added to the reaction solution, and the resulting mixture was extracted three times with dichloromethane, the organic phase was dried with anhydrous sodium sulfate, filtered, concentrated, and 1.59g of product was obtained by column chromatography with dichloromethane: methanol = 30: 1.

2. Synthesis of int 5-3

Int 5-2 (800mg, 1.0eq), $CD_3I$ (1.95g, 6.0eq), $K_2CO_3$ (1.24g, 4.0eq), and acetone (10mL) were added to a 100 mL single-necked flask, and the resulting mixture was stirred at 65°C overnight. After the reaction was completed, the solvent was evaporated, water (15 mL) was added to the reaction system, the resulting mixture was extracted with ethyl acetate (15 mL×2), the organic phase was dried with anhydrous sodium sulfate, filtered, and concentrated to obtain 792 mg of product.

3. Synthesis of int 5

Int5-3 (720mg, 1.0eq), and dichloromethane (10mL) were added to a 100 mL single-necked flask, then hydrochloric acid/dioxane (15mL) was added at room temperature. The reaction was carried out for 2h at room temperature. The reaction was monitored to completion by TLC, the resulting mixture was filtered and the filter cake was collected to obtain 500mg of product.

## Synthesis of intermediate int 6

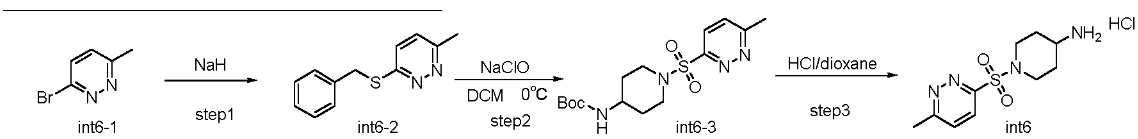

1. Synthesis of int 6-2

NaH (396.3mg, 1.5eq) and tetrahydrofuran (200mL) were added to a 500mL three-necked flask, the atmosphere was protected by $N_2$, and the temperature was cooled to 0 °C, benzyl mercaptan (1.36g, 1.0eq) was added, and the mixture was stirred for 10min, then a solution of C2-1 (2.0g, 1.05eq) in tetrahydrofuran (10mL) was added dropwise, and then the mixture was naturally warmed to room temperature and reacted for 2h. After the reaction was completed, the temperature was cooled to 0 °C, water was added to the reaction solution, the resulting mixture was extracted three times with ethyl acetate, the organic phase was dried with anhydrous sodium sulfate, filtrated, concentrated, and 2.4g of product was obtained by column chromatography with petroleum ether: ethyl acetate = 2: 1.

2. Synthesis of int 6-3

Int 6-2 (1.93g, 1.0eq), and dichloromethane (20mL) were added to a 100mL three-necked flask, the temperature was cooled to 0 °C, then water (8mL), and concentrated hydrochloric acid (2.0mL), and then aqueous sodium hypochlorite (10.4%, 9mL) were added to the reaction flask, the reaction was carried out at room temperature for 10min and then completed. Water was added (20mL) to the reaction system, the mixture was extracted with dichloromethane (20mL×3), the organic phase was dried with anhydrous sodium sulfate, filtrated, the filtrate was cooled to 0 °C, then a mixed solution of triethylamine and 4-Boc-aminopiperidine (1.78g, 1.0eq) in dichloromethane (1.8g, 2.0eq) was added, and the mixture was reacted at room temperature for 2h. After the reaction was completed, water was added to the reaction system, the resulting mixture was separated, the organic phase was dried with anhydrous sodium sulfate, filtrated, concentrated, and 1.2 g of product was obtained by column chromatography with dichloromethane: methanol = 100: 1.

3. Synthesis of int 6

C1-3 (1.2g, 1.0eq), and dichloromethane (10mL) were added to a 100mL three-necked flask, then hydrochloric acid/dioxane (15mL) was added at room temperature. The reaction was carried out for 2h at room temperature. The reaction was monitored to completion by TLC, the resulting mixture was filtered and the filter cake was collected to obtain 1.0g.

## Synthesis of intermediate int 7

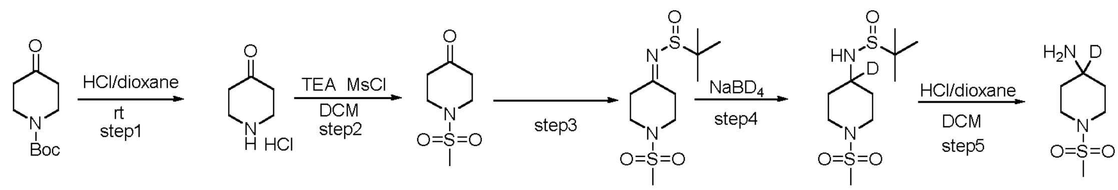


int7-1 int7-2 int7-3 int7-4 int7-5 int7

1. Synthesis of int 7-2

Int7-1 (11.9g, 1.0eq), and ethyl acetate (12mL) were added to a 250mL single-necked flask, then HCl/dioxane (15mL) was added at room temperature. The reaction was carried out for 2h at room temperature. The reaction was monitored to completion by TLC, the resulting mixture was filtered and the filter cake was collected to obtain 8.06g.

2. Synthesis of int 7-3

Int7-2 (8.06g, 1.0eq), dichloromethane (80mL), and triethylamine (12.08g, 2.0eq) were added to a 250mL single-necked flask, and the temperature was cooled to 0 °C then methylsulfonyl chloride (7.53g, 1.1eq) was added dropwise, then the mixture was naturally warmed up to room temperature and reacted for 2h. After the reaction was completed, ammonium chloride aqueous solution was added to the reaction solution, the resulting mixture was extracted three times with dichloromethane, the organic phase was dried with anhydrous sodium sulfate, filtrated, concentrated, slurried with EA: PE of about 1:5, the resulting slurry was filtrated, and the filter cake was collected to obtain 6.67g.

3. Synthesis of int 7-5

Int7-3 (6.67g, 1.0eq), dried tetrahydrofuran (110mL), and tert-butylsulfinamide (5g, 1.1eq) were added to a 250mL single-necked flask, then tetraethyl titanate (14.5g, 1.7eq) was added at room temperature, the atmosphere was protected by $N_2$, and then the mixture was stirred overnight at 70°C. The completion of the reaction was monitored by chromatoplate, the reaction solution was cooled to 0 °C, then $NaBD_4$ was added at once, and the mixture was reacted at room temperature for 3h. After the reaction was completed, water (80mL) was added to the reaction system, and a large amount of solid was precipitated, filtered, the residue was washed 5 times with EA, then the combined filtrates were concentrated and extracted 3 times with ethyl acetate, the ethyl acetate phase was dried, filtered, and 7.14g of product was obtained by column chromatography with dichloromethane: methanol = 80:1.

4. Synthesis of int 7

Int7-5 (7.14g, 1.0eq) and dichloromethane (10mL) were added to a 100mL single-necked flask, then 15mL hydrochloric acid/dioxane was added at room temperature. The reaction was carried out for 2h at room temperature. The reaction was monitored to completion by TLC, the resulting mixture was filtered and the filter cake was collected to obtain 5.88g.

**Synthesis of intermediate int 8**

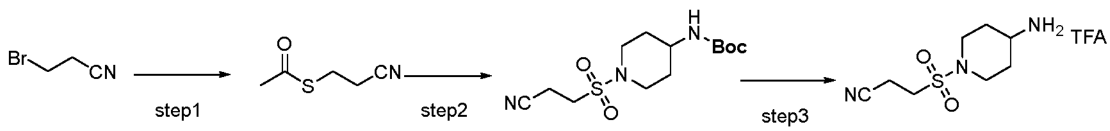


int8-1 int8-2 int8-3 int8

1. Synthesis of int 8-2

C3-1 (4.8g, 1.0eq), N,N-dimethylformamide (50mL), potassium thioacetate (8.2g, 2.0eq) were added to a 100mL single-necked flask, and the mixture was reacted at 60°C overnight. After the reaction was completed, water was added to the reaction solution, and the resulting mixture was extracted three times with ethyl acetate, the organic phase was dried with anhydrous sodium sulfate, filtered, concentrated, and 4.1g of product was obtained by column chromatography with petroleum ether:ethyl acetate=10:1 to 5:1.

2. Synthesis of int 8-3

2M hydrochloric acid-acetonitrile solution (25mL) was added to a 100mL single-necked flask, the temperature was then cooled to below 10 °C, and NCS (8.45g, 2.0eq) was added, the mixture was stirred for 10min with heat preservation, then a solution of C3-2 (4.1g, 1.0eq) in acetonitrile was added, and then the mixture was reacted for 2h. After evaporating away the majority of the acetonitrile, water was added to the reaction system, and the resulting mixture was extracted twice with ethyl acetate, the ethyl acetate phase was dried with anhydrous sodium sulfate, filtered, concentrated, added to a solution of 4-Boc-aminopiperidine (4.5g, 0.7eq) and triethylamine (6.4g, 2.0eq) in dichloromethane (20mL), and the resulting mixture was reacted at room temperature with stirring for 2h. After the reaction was completed, water was added to the reaction solution, and the resulting mixture was extracted three times with dichloromethane, the organic phase was dried with anhydrous sodium sulfate, filtered, concentrated, and 280mg of product was obtained by column chromatography with dichloromethane: methanol = 30: 1.

3. Synthesis of int 8

C3-3 (80mg, 1.0eq) and dichloromethane (3mL) were added to a 100mL single-necked flask, then trifluoroacetic acid (3mL) was added at room temperature. The reaction was carried out for 2h at room temperature. The reaction was monitored to completion by TLC, after the reaction solution was concentrated, ethyl acetate was added and the mixture was sonicated, a solid was precipitated and concentrated again to obtain 62mg.

## Synthesis of intermediate int 9

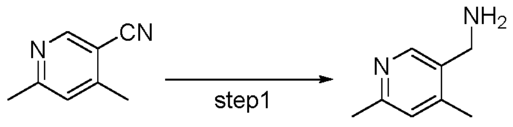


int9-1 int9

1. Synthesis of int 9

C4-1 (2.0 g, 1.0 eq), ethanol (70 mL), hydrazine hydrate (8 mL, 10 eq), and Raney nickel (10 mL) were added to a 100mL single-necked flask, then the mixture was reacted at room temperature overnight. After the reaction was completed, the resulting mixture was suction filtrated, the filtrate was dried with anhydrous sodium sulfate, and concentrated after suction filtrated again to obtain 1.8 g of crude product.

## Synthesis of intermediate int 10

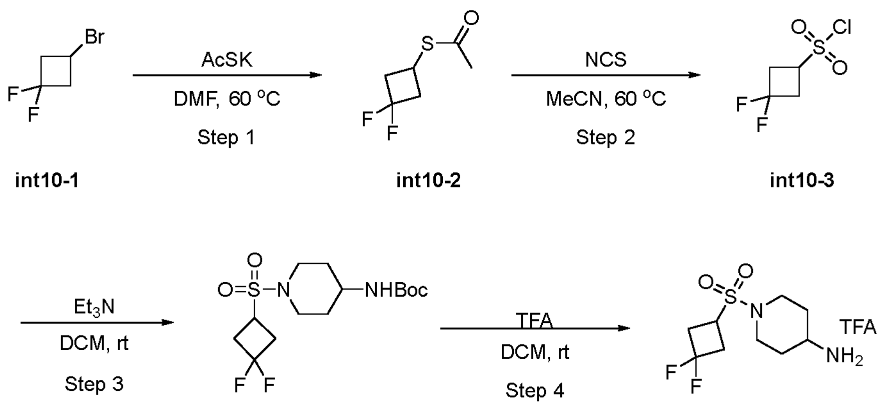


int10-1 int10-2 int10-3 int10-4 int10

1. Synthesis of int 10-2

Int10-1 (0.5 g, 2.94 mmol) was added to a 50mL round-bottomed flask, and 15 mL of dichloromethane was added to dissolve it with stirring, then potassium thioacetate (0.67 g, 5.88 mmol) was added, and the reaction system was moved to the condition at 60 °C and reacted for 4 h with stirring. After the reaction was completed, ethyl acetateand saturated brine were added to extract the organic phase, dried with anhydrous sodium sulfate, concentrated under reduced pressure, and 2.36 g of crude product was obtained by column chromatography.

2. Synthesis of int 10-3

2 mL of HCl:MeCN (v, 5:1) was added to a 50mL three-necked flask, then the temperature was cooled to below 10 °C, and N-bromosuccinimide (619.2 mg, 4.64 mmol) was added, the mixture was stirred for 10-30 min, a solution of int10-2 (382 mg, 2.32 mmol) in acetonitrile was added dropwise, after dropping, the reaction temperature was maintained at about 10 °C, the mixture was stirred for 1-2 h. After the reaction was completed, dichloromethane and saturated aqueous ammonium chloride were added to extract the organic phase, dried with anhydrous sodium sulfate, concentrated under reduced pressure, and 400 mg of crude product was obtained by column chromatography.

3. Synthesis of int 10-4

Int10-3 (210 mg, 1.1 mmol) was added to a 50mL single-necked flask and 6 mL of dichloromethane was added

to dissolve it with stirring, then 4-Boc-aminopiperidine (109.6 mg, 0.55 mmol) was added and homogeneous stirred, then triethylamine (221 mg, 2.2 mmol) was added, and the reaction was carried out at room temperature with stirring overnight. After the reaction mixture was fully reacted, the reaction was concentrated under reduced pressure and slurried with appropriate amount of petroleum ether and ethyl acetate to obtain 319 mg of crude product as a white solid.

4. Synthesis of int 10

Int10 (319 mg, 0.9 mmol) was added to a 50mL round-bottomed flask, and dichloromethane (5 mL) was added to dissolve it with stirring, the reaction system was then cooled to 0 °C, trifluoroacetic acid (1 mL) was added dropwise, and then the mixture was reacted for 2 h. After fully reacted, the reaction solution was concentrated under reduced pressure, then slurried with appropriate amount of petroleum ether and ethyl acetate to obtain 160 mg of crude product as a white solid.

**Synthesis of intermediate int 11**

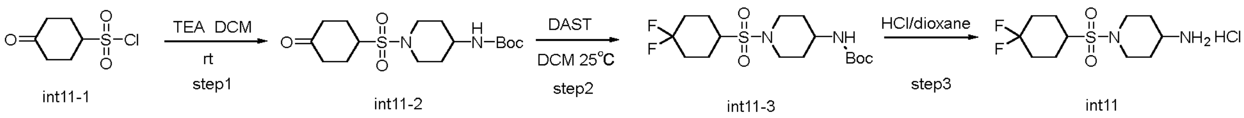


1. Synthesis of int 11-2

4-Boc-aminopiperidine (1.0g,1.0eq), dichloromethane (10mL), and triethylamine (1.01g,2.0eq) were added to a 100mL single-necked flask, the temperature was cooled to 0 °C and then a solution of int11-1 (1.01g,1.0eq) in dichloromethane (2mL) was added dropwise, and themixture was naturally warmed up to room temperature and reacted for 2h. After the reaction was completed, water was added to the reaction solution, and the resulting mixture was extracted three times with dichloromethane, the organic phase was dried with anhydrous sodium sulfate, filtered, concentrated, and 1.73g of product was obtained by column chromatography with petroleum ether:ethyl acetate=5:1 to 1:1.

2. Synthesis of int 11-3

Int11-2 (600mg, 1.0eq) and dichloromethane (30mL) were added to a 100mL single-necked flask, the atmosphere was protected by nitrogen, and DAST (1.34g, 5.0eq) was added dropwise at 0°C, after dropping, the mixture was reacted at room temperature overnight. After the reaction was completed, ice water was added to the reaction system, the resulting mixture was extracted twice with dichloromethane, the organic phase was dried with anhydrous sodium sulfate, filtered, concentrated, and 360mg of product was obtained by column chromatography with petroleum ether:ethyl acetate=8:1 to 6:1.

3. Synthesis of int 11

Int 11-3 (480mg, 1.0eq) and dichloromethane (17mL) were added to a 100mL single-necked flask, then hydrochloric acid/1,4-dioxane (10mL) was added at room temperature. The reaction was carried out for 2h at room temperature. The reaction was monitored to completion by TLC, the resulting mixture was filtered and the filter cake was collected to obtain 370mg.

**Synthesis of intermediate int 12**

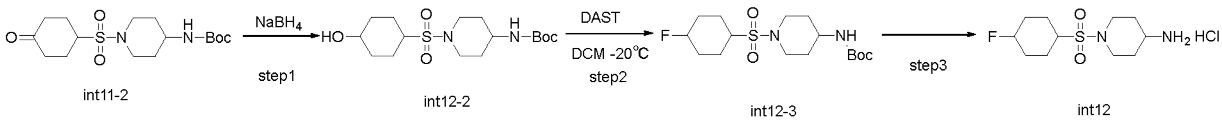

1. Synthesis of int 12-2

Int11-2, followed by dichloromethane/ methanol (50 mL/5 mL) were added to a 100mL single-necked flask, and sodium borohydride (115 mg, 1.1 eq) was added at 0 °C, and then the mixture was reacted at room temperature overnight. After the reaction was completed, the reactionwas quenched by adding water to the reaction system, the resulting mixture was extracted twice with dichloromethane, the organic phase was dried with anhydrous sodium sulfate, and concentrated to obtain 980 mg.

2. Synthesis of int 12-3

C3-2 (780mg, 1.0eq) and dichloromethane (30mL) were added to a 100mL single-necked flask, the atmosphere was protected by nitrogen, and DAST (381mg, 1.1eq) was added dropwise at 0°C, after dropping, the mixture was reacted at room temperature for 3h. After the reaction was completed, ice water was added to the reaction system, the resulting mixture was extracted twice with dichloromethane, the organic phase was dried with anhydrous sodium sulfate, filtered, concentrated, and 120mg of product was obtained by column chromatography with petroleum ether: ethyl acetate=8:1 to 6:1.

3. Synthesis of int 12

C3-3 (160mg, 1.0eq), and dichloromethane (5mL) were added to a 100mL single-necked flask, then hydrochloric acid/1,4-dioxane (5mL) was added at room temperature. The reaction was carried out for 2h at room temperature. The reaction was monitored to completion by TLC, the resulting mixture was filtered and the filter cake was collected to obtain 103mg.

**Synthesis of intermediate int 13**

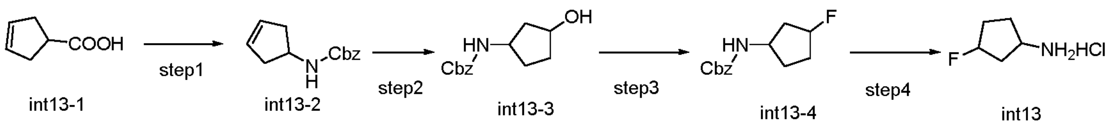


int13-1 int13-2 int13-3 int13-4 int13

1. Synthesis of int 13-2

Int13-1 (6.8 g, 1.0 eq), DPPA (25.0 g, 1.5 eq), triethylamine (30.6 g, 5.0 eq), and 1,4-dioxane (68 mL) were added to a nitrogen-protected 250 mL three-necked flask, then the mixture was reacted at 100 °C for 2 h, the temperature was then cooled to 40 °C to 50 °C, benzyl alcohol (13.1 g, 2.0 eq) was added dropwise, after dropping, and then the mixture was warmed up to 100°C and reacted for 16 hours. The reaction was monitored to completion, the temperature was then cooled to room temperature, and saturated brine was added to the reaction system, the resulting mixture was extracted three times with ethyl acetate, the organic phase was dried with anhydrous sodium sulfate, filtered, concentrated, and 9.8g of product was obtained by column chromatography with petroleum ether: ethyl acetate=10:1.

2. Synthesis of int 13-3

Int13-2 (9.8 g, 1.0 eq) and ultra-dry tetrahydrofuran (98 mL) were added to a nitrogen-protected three-necked flask, and 1.0 M borane tetrahydrofuran (88.2 mL) was added dropwise at 0 °C, after dropping, the temperature was held for 1 hour, and then the mixture was reacted at room temperature overnight. The reaction was monitored to completion, the temperature was then cooled to 0 °C, and water (19.6 mL), and 10% aqueous sodium hydroxide (68.7 mL), then 30% hydrogen peroxide (49 mL) (dropwise) were added to the reaction system, and the mixture was reacted at room temperature for 5 hours. The reaction was monitored to completion, the reaction system was extracted twice with ethyl acetate, the organic phase was dried with anhydrous sodium sulfate , filtered, concentrated, and 4.8g of product was obtained by column chromatography with petroleum ether:ethyl acetate = 10:1.

3. Synthesis of int 13-4

C5-3 (3g, 1.0eq) and dichloromethane (92mL) were added to a 100mL single-necked flask, the atmosphere was protected by nitrogen, DAST (4.1g, 2.0eq) was added dropwise at -20°C, after dropping, then the mixture was

reacted at room temperature for 3h. After the reaction was completed, ice water was added to the reaction system, the resulting mixture was extracted twice with dichloromethane, the organic phase was dried with anhydrous sodium sulfate, filtered, concentrated, and 580mg of product was obtained by column chromatography with petroleum ether:ethyl acetate=10:1.

4. Synthesis of int 13

C5-4 (660mg, 1.0eq) was dissolved in methanol (5mL), Pd/C (13.6mg, 10%) was added, the atmosphere was protected by nitrogen, replaced with hydrogen for three times. The reaction was carried out overnight at room temperature. The reaction was monitored to completion, the resulting mixture was filtered and hydrochloric acid/1,4-dioxane (2 mL) was added, and the resulting mixture was concentrated to obtain 360 mg.

**Synthesis of intermediate int 14**

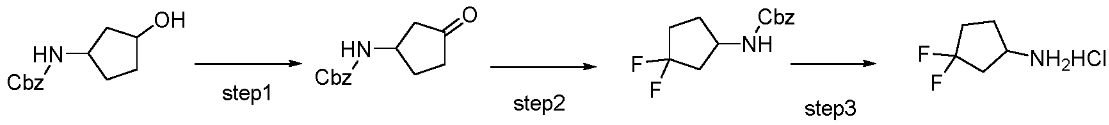


int14-1 int14-2 int14-3 int14

1. Synthesis of int 14-2

Int14-1 (1.86g, 1.0eq) and dichloromethane (74mL) were added to a three-necked flask, after the temperature was cooled to 0 °C, PCC (3.4g, 2.0eq) was added in three batches, the atmosphere was protected by nitrogen, and the mixture was reacted at room temperature for 48h. After the reaction was monitored to completion, saturated brine was added to the reaction system, the resulting mixture was filtrated, the filtrate was extracted twice with dichloromethane, the organic phase was dried with anhydrous sodium sulfate, filtered, concentrated, and then 1.62g of product was obtained by column chromatography with petroleum ether:ethyl acetate=5:1.

2. Synthesis of int 14-3

C6-2 (1.62g, 1.0eq) and dichloromethane (83mL) were added to a 100 mL single-necked flask, the atmosphere was protected by nitrogen, and DAST (5.5g, 5.0eq) was added dropwise at 0°C, after dropping, then the mixture was reacted at room temperature overnight. After the reaction was completed, ice water was added to the reaction system, the resulting mixture was extracted twice with dichloromethane, the organic phase was dried with anhydrous sodium sulfate, filtered, concentrated, and 910mg of product was obtained by column chromatography with petroleum ether: ethyl acetate=10:1.

3. Synthesis of int 14

C6-3 (910mg, 1.0eq) was dissolved in methanol (5mL), Pd/C (91mg, 10%) was added, the atmosphere was protected by nitrogen, replaced with hydrogen for three times, and the mixture was reacted overnight at room temperature. The reaction was monitored to completion, the resulting mixture was filtered, and hydrochloric acid/1,4-dioxane (5 mL) was added and the resulting mixture was concentrated to obtain 650mg.

**Synthesis of intermediate int 15**

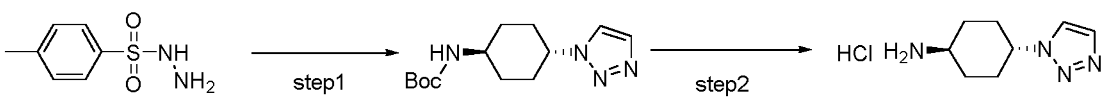


int15-1 int15-2 int15

1. Synthesis of int 15-2

Glyoxal-1,1-dimethyl acetal (500 mg, 1.0 eq), int15-1 (1.48 g, 1.0 eq), and methanol (10 mL) were added to a 100 mL single-necked flask, and the mixture was reacted at room temperature for 2 h. The reaction was monitored to completion, N-Boc-trans-1,4-cyclohexanediamine (1.11 g, 1.1 eq) and acetic acid (288 mg, 1.0 eq) were added to the reaction system, and then the mixture was warmed to 75 °C and reacted overnight. The reaction was monitored to completion, aqueous sodium bicarbonate was added to the reaction system to adjust the PH>7, then water was added, the resulting mixture was extracted three times with ethyl acetate, the organic phase was dried with anhydrous sodium sulfate, filtered, concentrated, and 320mg of product was obtained by column chromatography with petroleum ether:ethyl acetate=2:1.

2. Synthesis of int 15

Int C7-2 (320mg, 1.0eq), and dichloromethane (3mL) were added to a 100 mL single-necked flask, then hydrochloric acid/1,4-dioxane (5mL) was added at room temperature. The reaction was carried out for 2h at room temperature. The reaction was monitored to completion by TLC, the resulting mixture was filtered and the filter cake was collected to obtain 240mg.

**Synthesis of intermediate int 16**

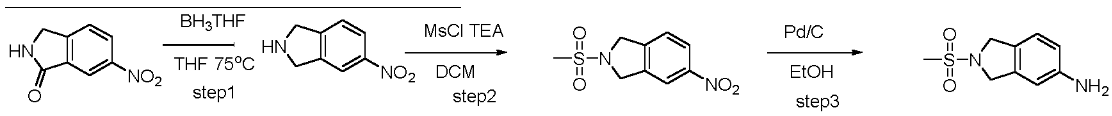


int16-1 int16-2 int16-3 int16

1. Synthesis of int 16-2

Int16-1 (2g, 1.0eq), $BH_3$/THF (1M 34Ml 3eq) were added to a 250 mL single-necked flask, and tetrahydrofuran (80mL) was added at room temperature, then the mixture was reacted at reflux overnight. The reaction was monitored to completion by TLC, the temperature was then cooled to room temperature, and 100ml of 1M HCl was added dropwise, the resulting mixture was extracted with ethyl acetate for 5 times, and then the aqueous phase was cooled to 0 °C, adjusted to neutral with saturated sodium bicarbonate solution and then extracted with ethyl acetate again, and the organic phase was dried and concentrated to obtain 690mg of product.

2. Synthesis of int 16-3

Int 16-2 (690mg, 1.0eq), dichloromethane (30mL), and triethylamine (1.27g, 3eq) were added to a 100 mL single-necked flask, the temperature was cooled to 0 °C, and methylsulfonyl chloride (723mg, 1.5eq) was added dropwise, then the mixture was naturally warmed up to room temperature and reacted for 2h. After the reaction was completed, ammonium chloride aqueous solution was added to the reaction solution, the resulting mixture was extracted three times with dichloromethane, the organic phase was dried with anhydrous sodium sulfate, filtered, concentrated, and 640 mg of product was obtained by column chromatography with dichloromethane: methanol = 200:1 - 100:1.

3. Synthesis of int 16

Int16-3 (640mg, 1.0eq), ethanol (64mL), and Pd/C (64mg) were added to a 100 mL three-necked flask, then the reaction was carried out under hydrogen atmosphere at 50 °C for 2h, after the reaction was completed, the resulting mixture was filtered, and concentrated to obtain 165mg.

**Synthesis of intermediate int 17**

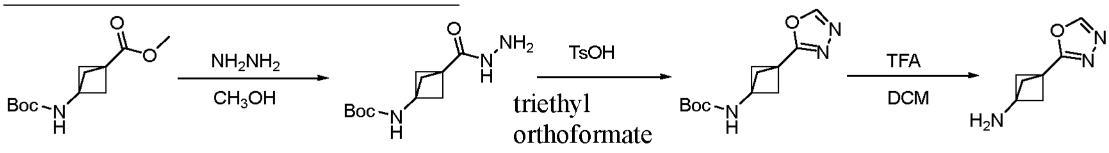


int17-1 int17-2 int17-3 int17

1. Synthesis of int 17-2

Methyl 3 - {[(tert-butoxy)carbonyl]amino}bicyclo[1.1.1]pentane-1-formate (560 mg, 2.32 mmol) and methanol (5 mL) were added to a 50 mL single-necked flask, and hydrazine hydrate was added dropwise to the reaction mixture at room temperature, after adding, and then the mixture was stirred for 30 min at room temperature and heated to 80°C to react for 2-3 hours. The reaction was monitored to completion by LC-MS, and the resulting mixture was concentrated under reduced pressure to obtain the crude product. After the crude product was watered down twice with toluene, a white solid (562 mg) was obtained, which was directly used in the next step without further purification.

2. Synthesis of int 17-3

C8-1 (562 mg, 2.32 mmol) and TsOH (40mg, 0.232 mmol) were added to triethyl orthoformate (8 mL) in a 50 mL single-necked flask, then the mixture was heated to 80 °C and reacted for 4 h. The reaction was monitored to completion by LC-MS, the temperature was then cooled to 50 °C, then the resulting mixture was concentrated under reduced pressure to obtain a crude product, and then 465 mg of white solid was obtained by column chromatography (PE-PE:EA=2:1).

3. Synthesis of int 17

C8-2 (465 mg, 1.852 mmol) and dichloromethane (5 mL) were added to a 50 mL single-necked flask, then the temperature was cooled to 0 °C under ice bath, trifluoroacetic acid (1 mL) was added dropwise, after dropping, and then the mixture was naturally warmed to room temperature and reacted for 4 h. The reaction was monitored to completion by TLC, then the resulting mixture was concentrated under reduced pressure to obtain the crude product, which was slurried with ethyl acetate (20 mL), and the slurry was filtered to obtain a white solid (347 mg).

**Example 1**

The compound synthesized in the present invention:

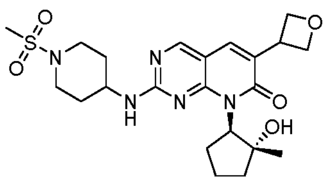

**C1**

The synthetic route and experimental procedure are as follows:

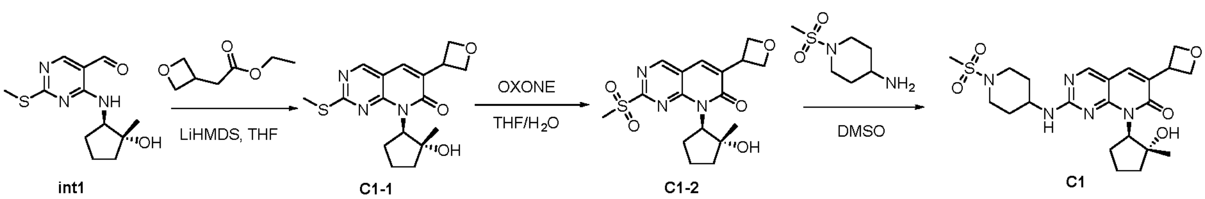


**int1** **C1-1** **C1-2** **C1**

1. Synthesis of **C1-1**

Ethyl 2-(oxetan-3-yl)acetate (200 mg, 1.54 mmol) and dry tetrahydrofuran were added to a 50 mL single-necked flask, and LiHMDS (2.15 mmol) was added dropwise at -78 °C, after dropping, the mixture was reacted for 30 min at

-78 °C, after which a solution of **int1** (165 mg, 1.54 mmol) in tetrahydrofuran was added dropwise, after dropping, and then the mixture was slowly warmed up to room and reacted for 16 h. After the reaction was completed, saturated ammonium chloride was added, the resulting mixture was extracted with ethyl acetate, the organic phase was dried with anhydrous sodium sulfate, filtered, concentrated, and 60 mg was obtained by passing through a column with petroleum ether:ethyl acetate=10:1 to 5:1.

2. Synthesis of **C1-2**

**C2-1** (55 mg, 0.16 mol), tetrahydrofuran (5 mL) and water (1 mL) were added to a 50 mL single-necked flask, the temperature was then cooled to 0 °C, and OXONE (240 mg, 0.40 mol) was added in batches. The reaction was carried out at room temperature for 2 h. After the reaction was completed, the resulting mixture was was filtered, the filter cake was washed twice with EA, the mother liquor was collected and concentrated to obtain 50 mg.

3. Synthesis of **C1**

**C2-2** (50 mg, 0.12 mmol), 1-methylsulfonyl-4-aminopiperidine (32 mg, 0.15 mmol), N,N-diisopropylethylamine (81 mg, 0.3 mmol) and dimethyl sulfoxide (5 mL) were added to a 50 mL single-necked flask. The temperature was heated to 60°C and the reaction was carried out for 16h. After the reaction was completed, water was added to the reaction solution, and the resulting mixture was extracted three times with ethyl acetate, the organic phase was dried with anhydrous sodium sulfate, filtered, concentrated, and 15 mg of the product was obtained by preparative plate separation with petroleum ether:ethyl acetate=1:5. $^1$H NMR (400 MHz, $CDCl_3$) δ 8.47 (s, 1H), 7.46 (d, J = 1.4 Hz, 1H), 5.74 (t, J = 8.5 Hz, 1H), 5.35 (s, 2H), 5.06 (ddd, J = 8.5, 6.0, 2.6 Hz, 2H), 4.70 (ddd, J = 11.1, 7.0, 6.0 Hz, 2H), 4.43 - 4.28 (m, 1H), 3.98 (s, 1H), 3.90 - 3.73 (m, 2H), 3.00 - 2.86 (m, 2H), 2.82 (s, 3H), 2.30 - 2.13 (m, 3H), 2.07 - 1.95 (m, 2H), 1.95 - 1.76 (m, 2H), 1.76 - 1.58 (m, 2H), 1.13 (s, 3H).

## Example 2

The compound synthesized in the present invention:

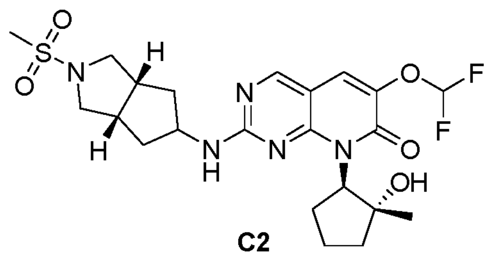

**C2**

The synthetic route and experimental procedure are as follows:

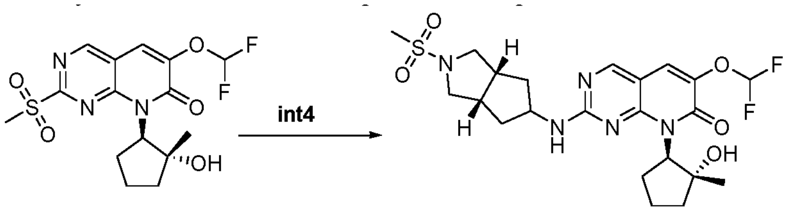


int3

C2

1. Synthesis of C2

Int3 (396 mg, 1.23 mmol), **int4** (300 mg, 1.47 mmol), N,N-diisopropylethylamine (475 mg, 3.68 mmol) and dimethylsulfoxide (5 mL) were added to a 50 mL single-necked flask. The temperature was heated to 60°C and the reaction was carried out for 16 h. After the reaction was completed, water was added to the reaction solution, and the resulting mixture was extracted three times with EA, the organic phase was dried with anhydrous sodium sulfate, filtered, concentrated, and 370 mg of product was obtained by column chromatography with petroleum ether:ethyl acetate=5:1 to 0:1.

## Example 3

The compound synthesized in the present invention:

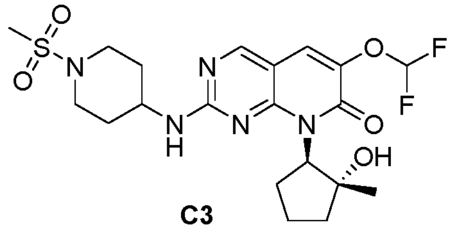

C3

The synthetic route and experimental procedure are as follows:

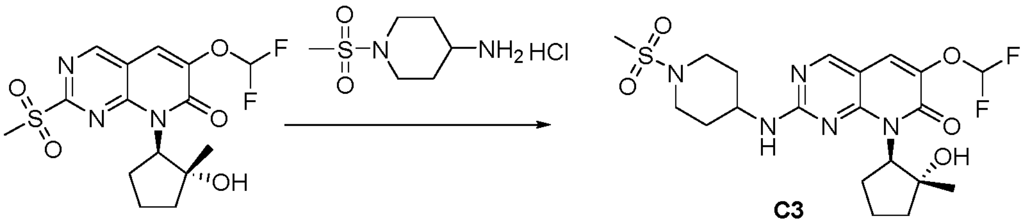



int3

C3

Synthesis of C3

Compound **int3** (80 mg) was dissolved in DMSO, 1-methylsulfonyl-4-aminopiperidine (88.4 mg, 2.0 eq), triethylamine (0.11 ml, 3.0 eq) were added thereto, and the reaction was carried out at 60°C overnight. The reaction was monitored to completion, water was added to the system and the resulting mixture was extracted with ethyl acetate, the organic phases were combined, dried with anhydrous sodium sulfate, concentrated under reduced pressure and the product (50 mg) was obtained by preparative plate separation. $^1H$ NMR (400 MHz, $CDCl_3$) δ 8.45 (s, 1H), 7.37 (s, 1H), 6.77 (t, J = 75.0 Hz, 1H), 5.81 (s, 1H), 5.44 (m, 1H), 3.98 (br, 1H), 3.82 (t, J = 12.1 Hz, 2H), 2.99 - 2.87 (m, 2H), 2.83 (s, 3H), 2.74 (br, 1H), 2.35 - 2.16 (m, 3H), 2.04 (d, J = 7.1 Hz, 3H), 1.93 (t, J = 9.5 Hz, 1H), 1.82 (dd, J = 12.0, 6.4 Hz, 1H), 1.67 (td, J = 12.9, 12.0, 4.0 Hz, 2H), 1.17 (s, 3H).

## Example 4

The compound synthesized in the present invention:

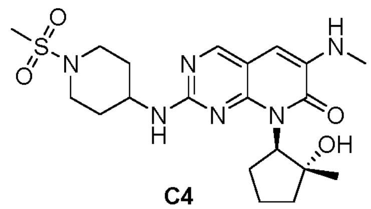

C4

The synthetic route and experimental procedure are as follows:

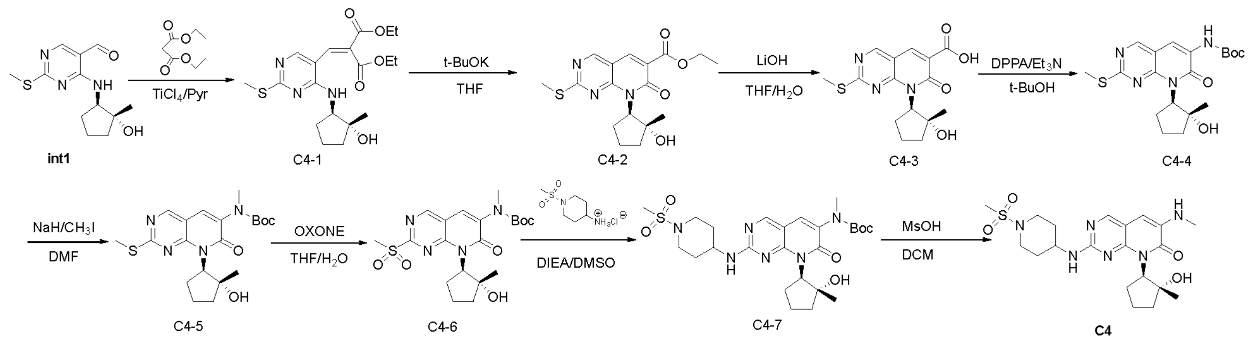


int1, C4-1, C4-2, C4-3, C4-4, C4-5, C4-6, C4-7, C4

Synthesis of C4-1

Tetrahydrofuran (10 mL) was added to a 100 mL three-necked flask, the temperature was cooled to -5 °C, and a solution of titanium tetrachloride (3.04 g, 16 mmol) in dichloromethane (5 mL) was added dropwise. After dropwise, the mixture was stirred for 10 min, a solution of int1 (1.07 g, 4 mmol) and diethyl malonate (1.28 g, 8 mmol) in tetrahydrofuran (10 mL) was added dropwise into the reaction solution, after dropwise, the mixture was stirred for 30 min, pyridine (1.58 g, 20 mmol) was added dropwise, and after dropwise, the reaction solution was warmed up naturally and stirred at room temperature overnight. The reaction was monitored to completion by TLC. The reaction solution was then diluted with water, extracted with ethyl acetate, dried with anhydrous sodium sulfate, filtered, concentrated, and 1.31 g of product was obtained by column chromatography with petroleum ether:ethyl acetate=5:1 to 3:1.

Synthesis of C4-2

C4-1 (1.31 g, 3.20 mmol) and tetrahydrofuran (15 mL) were added to a 50 mL single-necked flask, and a solution of potassium tert-butanolate (36 mg, 0.32 mmol) in tetrahydrofuran (1 mL) was added dropwise at room temperature, and the mixture was stirred for 15 min at room temperature. The reaction was monitored to completion by TLC, and 1.02 g of product was obtained by column chromatography with petroleum ether: ethyl acetate=3:1 to 1:1.

Synthesis of C4-3

C4-2 (1.01 g, 2.78 mmol), lithium hydroxide (200 mg, 8.35 mmol), tetrahydrofuran (10 mL) and water (10mL) were added to a 50 mL single-necked flask, and the mixture was stirred for 15 min at room temperature. The reaction was monitored to completion by TLC. The reaction solution was then diluted with water, extracted with ethyl acetate, dried with anhydrous sodium sulfate, filtered, concentrated to obtain 0.98g of crude product.

Synthesis of C4-4

C4-3 (335mg, 1.00mmol), diphenyl phosphate azide (330 mg, 1.20 mmol), triethylamine (122mg, 1.20mmol), and tert butanol (5mL) were added to a 50 mL single-necked flask, the mixture was heated to 78 °C and reacted for 16 hours. The reaction was monitored to completion by TLC. The reaction solution was then diluted with water, extracted with ethyl acetate, dried with anhydrous sodium sulfate, filtered, concentrated, and 83mg of product was obtained by column chromatography with petroleum ether:ethyl acetate=4:1 to 2:1.

Synthesis of C4-5

Sodium hydride (5.6 mg, 0.139 mmol) was added to a solution of N,N-dimethylformamide (3 ml) of C4-4 (47 mg, 0.116 mmol) at 0°C. After addition, the mixture was warmed naturally to room temperature and reacted for 30 min. Then the reaction system was cooled to 0°C, methyl iodide was added dropwise to the reaction solution, and the mixture was warmed up to room temperature and reacted for 2 hours. The reaction was monitored to completion by TLC. The reaction solution was then diluted with water, extracted with ethyl acetate, dried with anhydrous sodium sulfate, filtered, concentrated, and 48 mg of product was obtained by column chromatography.

Synthesis of C4-6

OXONE (160 mg, 0.268 mmol) was added to a mixed solution of C4-5 (45 mg, 0.107 mmol) in tetrahydrofuran and waterat 0°C, after addition, and the mixture was warmed up naturally to room temperature and reacted for 2 h. The

reaction was monitored to completion by TLC. The reaction solution was then diluted with water, extracted with ethyl acetate, dried with anhydrous sodium sulfate, filtered, concentrated to obtain 51mg of crude product.

Synthesis of C4-7

C4-6 (51mg, 0.113mmol), 1-methylsulfonyl-4-aminopiperidine (49 mg, 0.226 mmol), diisopropylethylamine (59 mg, 0.452 mmol) and dimethyl sulfoxide (2 mL) were added to a 50 mL single-necked flask, the mixture was heated to 60 °C and reacted for 4 hours. The reaction was monitored to completion by TLC. The reaction solution was then diluted with water, extracted with ethyl acetate, dried with anhydrous sodium sulfate, filtered, concentrated, and 45mg of product was obtained by column chromatography.

Synthesis of **C4**

Methane sulfonic acid (78 mg, 0.82 mmol) was added dropwise to a solution of C4-7 (45 mg, 0.082 mmol) in dichloromethane (3 mL) at 0°C, after addition, then the mixture was warmed up naturally to room temperature and reacted for 1 h. The reaction was monitored to completion by TLC. The reaction solution was then diluted with water, extracted with ethyl acetate, dried with anhydrous sodium sulfate, filtered, concentrated, and 25mg of product was obtained by column chromatography. $^1H$ NMR (400 MHz, $CDCl_3$) $\delta$ 8.39 (s, 1H), 6.26 (s, 1H), 5.88 (s, 1H), 5.22 (s, 1H), 4.98 (s, 1H), 3.95 (s, 1H), 3.78 (s, 2H), 2.95 (s, 2H), 2.89 (d, J = 4.3 Hz, 3H), 2.83 (s, 3H), 2.72 (s, 1H), 2.26 - 2.13 (m, 3H), 2.02 (s, 1H), 1.93 - 1.78 (m, 3H), 1.59 (m, 3H), 1.34 (s, 3H).

**Example 5**

The compound synthesized in the present invention:

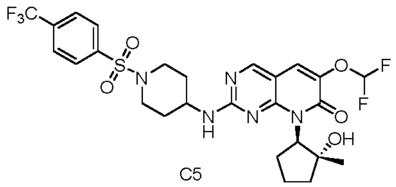

C5

The synthetic route and experimental procedure are as follows:

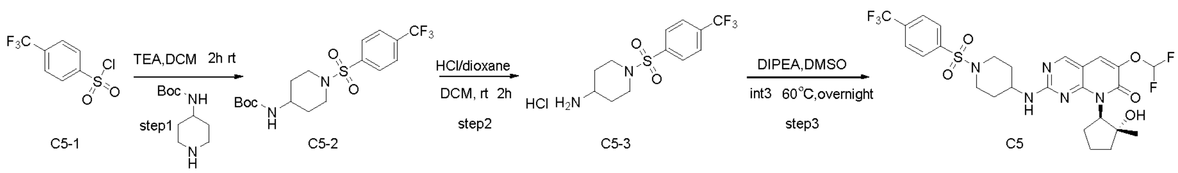


Synthesis of C5-2

4-Boc-aminopiperidine (1.0g, 1.0eq), dichloromethane (10 ml), triethylamine (1.01g, 2.0eq) were added to a 100mL single-necked flask was added, the temperature was cooled to 0°C and a solution of C1-1 (1.28g, 1.0eq) in dichloromethane (2 ml) was added dropwise, and the mixture was naturally warmed up to room temperature and reacted for 2h. After the reaction was completed, water was added to the reaction solution, and the resulting mixture was extracted three times with dichloromethane, the organic phase was dried with anhydrous sodium sulfate, filtered, concentrated, and 1.83g of product was obtained by column chromatography with petroleum ether:ethyl acetate=5:1 to 1:1.

Synthesis of C5-3

C5-2 (1.83g, 1.0eq), dichloromethane (18ml) were added to a 100mL single-necked flask, and then HCl/dioxane (10ml) was added at room temperature. The reaction was carried out for 2h at room temperature. The reaction was monitored to completion by TLC, the resulting mixture was filtered and the filter cake was collected to obtain 1.7g.

Synthesis of C5

C5-3 (74.3 mg, 1.2eq), int3 (70 mg, 1.0eq), diisopropylethylamine (70 mg, 3.0eq), and dimethyl sulfoxide (3ml) were added to a 50 mL single-necked flask. The reaction was carried out at 60 °C overnight. The reaction was monitored to completion by LC-MS, water was added to the reaction solution, and the resulting mixture was extracted three times with ethyl acetate, dried with anhydrous sodium sulfate, filtered, concentrated, and 24mg of product was obtain by Prep-TLC. MS[M+1]:618.6. $^1$H NMR (400 MHz, $CDCl_3$) $\delta$ 8.38 (d, *J* = 14.1 Hz, 1H), 7.92 (d, *J* = 8.2 Hz, 2H), 7.83 (d, *J* = 8.3 Hz, 2H), 7.33 (s, 1H), 6.75 (t, *J* = 75.1 Hz, 1H), 5.76 (t, *J* = 8.4 Hz, 1H), 5.64 - 5.27 (m, 1H), 3.77 (t, *J* = 43.4 Hz, 3H), 3.06 - 2.43 (m, 3H), 2.36 - 2.09 (m, 4H), 2.07 - 1.66 (m, 6H), 1.14 (s, 3H).

## Example 6

The compound synthesized in the present invention:

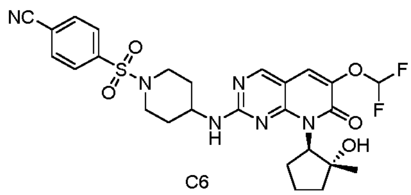

C6

The synthetic route and experimental procedure are as follows:

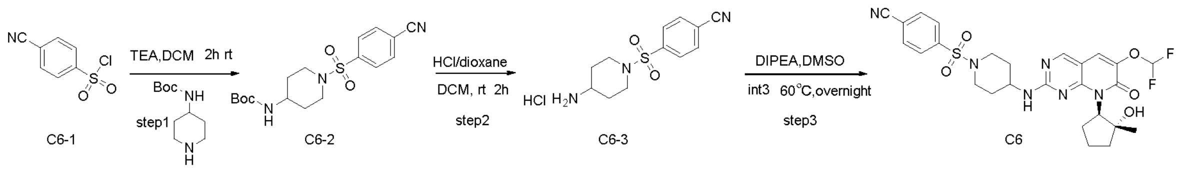


Synthesis of C6-2

4-Boc-aminopiperidine (1.0g, 1.0eq), dichloromethane (10 ml), and triethylamine (1.01g, 2.0eq) were added to a 100mL single-necked flask, the temperature was cooled to 0 °C and a solution of C2-1 (1.058g, 1.0eq) in dichloromethane (2 ml) was added dropwise, and the mixture was naturally warmed up to room temperature and reacted for 2h. After the reaction was completed, water was added to the reaction solution, and the resulting mixture was extracted three times with dichloromethane, the organic phase was dried with anhydrous sodium sulfate, filtered, concentrated, and 1.74g of product was obtained by column chromatography with petroleum ether: ethyl acetate=5:1 to 1:1.

Synthesis of C6-3

C6-2 (1.74g, 1.0eq) and dichloromethane (17ml) were added to a 100mL single-necked flask, and then HCl/dioxane (10ml) was added at room temperature. The reaction was carried out for 2h at room temperature. The reaction was monitored to completion by TLC, the resulting mixture was filtered and the filter cake was collected to obtain 1.5g.

Synthesis of C6

C6-3 (67 mg, 1.2eq), int3 (70 mg, 1.0eq), diisopropylethylamine (70 mg, 3.0eq), and dimethylsulfoxide (3ml) were added to a 50 mL single-necked flask. The reaction was carried out at 60 °C overnight. The reaction was monitored to completion by LC-MS, water was added to the reaction solution, and the resulting mixture was extracted three times with ethyl acetate, dried with anhydrous sodium sulfate, filtered, concentrated, and 34 mg of product was obtain by Prep-TLC. $^1$H NMR (400 MHz, $CDCl_3$) $\delta$ 8.43 (s, 1H), 7.36 (s, 1H), 6.82 (t, *J* = 75.3 Hz, 1H), 5.73 (s, 1H), 5.32 (m, *J* = 62.4, 53.3 Hz, 2H), 4.37 - 4.12 (m, 1H), 3.80 (d, *J* = 10.9 Hz, 3H), 2.93 (m, *J* = 25.6, 14.7 Hz, 4H), 2.51 - 2.02 (m, 7H), 1.98 - 1.65 (m, 4H), 1.26 (s, *J* = 6.6 Hz, 3H).
MS[M+1]:575.6

**Example 7**

The compound synthesized in the present invention:

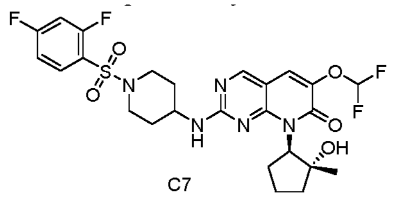

C7

The synthetic route and experimental procedure are as follows:

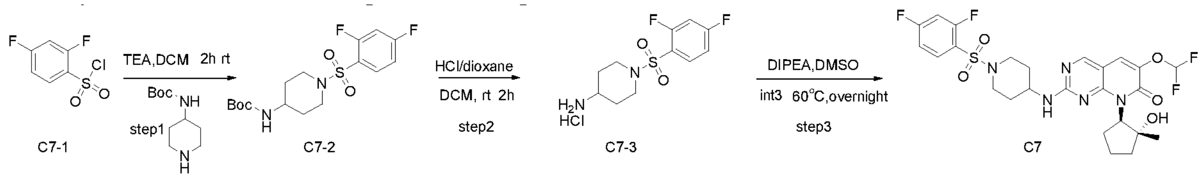


Synthesis of C7-2

4-Boc-aminopiperidine (1.0g, 1.0eq), dichloromethane (10 ml), and triethylamine (1.01g, 2.0eq) were added to a 100mL single-necked flask, the temperature was cooled to 0 °C and C3-1 (1.06g, 1.0eq) was added dropwise, then the mixture was naturally warmed up to room temperature and reacted for 2h. After the reaction was completed, water was added to the reaction solution, and the resulting mixture was extracted three times with dichloromethane, the organic phase was dried with anhydrous sodium sulfate, filtered, concentrated, and 1.89g of product was obtained by column chromatography with petroleum ether:ethyl acetate=5:1 to 1:1.

Synthesis of C7-3

C7-2 (1.74g, 1.0eq) and dichloromethane (17ml) were added to a 100mL single-necked flask, and then HCl/dioxane (10ml) was added at room temperature. The reaction was carried out for 2h at room temperature. The reaction was monitored to completion by TLC, the resulting mixture was filtered and the filter cake was collected to obtain 1.51g.

Synthesis of C7

C7-3 (128.7mg, 2.0eq), int3 (80 mg, 1.0eq), diisopropylethylamine (105.7 mg, 4.0eq), and dimethylsulfoxide (4ml) were added to a 50 mL single-necked flask. The reaction was carried out at 60 °C overnight. The reaction was monitored to completion by LC-MS, water was added to the reaction solution, and the resulting mixture was extracted three times with ethyl acetate, dried with anhydrous sodium sulfate, filtered, concentrated, and 42 mg of product was obtain by Prep-TLC. $^1$H NMR (400 MHz, $CDCl_3$) $\delta$ 8.42 (s, 1H), 7.89 (m, *J* = 14.4, 8.1 Hz, 1H), 7.34 (s, 1H), 7.06 - 6.95 (m, 2H), 6.76 (t, *J* = 75.1 Hz, 1H), 5.78 (t, *J* = 8.7 Hz, 1H), 5.48 (d, *J* = 34.3 Hz, 1H), 4.08 - 3.73 (m, 3H), 3.03 - 2.62 (m, 3H), 2.24 (m, *J*= 41.8, 22.4, 9.7 Hz, 3H), 2.09 - 1.66 (m, 7H), 1.14 (s, 3H). MS[M+1]:586.5.

**Example 8**

The compound synthesized in the present invention:

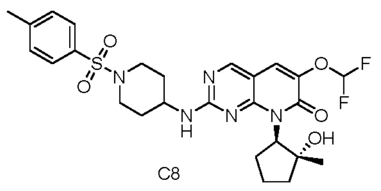

C8

The synthetic route and experimental procedure are as follows:

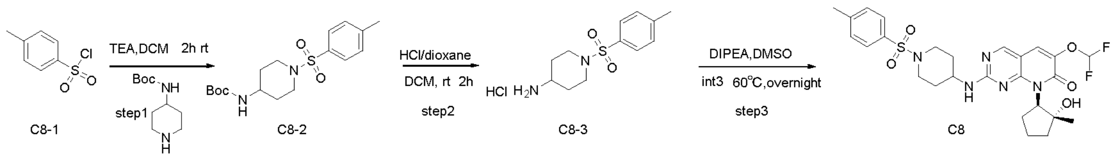

C8-1 C8-2 C8-3 C8

Synthesis of C8-2

4-Boc-aminopiperidine (1.0g, 1.0eq), dichloromethane (10 ml), and triethylamine (1.01g, 2.0eq) were added to a 100mL single-necked flask, the temperature was cooled to 0 °C and a solution of C4-1 (1.0g, 1.05eq) in dichloromethane (2 ml) was added dropwise, then the mixture was naturally warmed up to room temperature and reacted for 2h. After the reaction was completed, water was added to the reaction solution, and the resulting mixture was extracted three times with dichloromethane, the organic phase was dried with anhydrous sodium sulfate, filtered, concentrated, and 1.88g of product was obtained by column chromatography with petroleum ether:ethyl acetate=5:1 to 1:1.

Synthesis of C8-3

C8-2 (1.88g, 1.0eq) and dichloromethane (19ml) were added to a 100mL single-necked flask, and then HCl/dioxane (13ml) was added at room temperature. The reaction was carried out for 2h at room temperature. The reaction was monitored to completion by TLC, the resulting mixture was filtered and the filter cake was collected to obtain 1.44g.

Synthesis of C8

C8-3 (149.5mg, 2.0eq), int3 (100mg, 1.0eq), diisopropylethylamine (105.7 mg, 4.0eq), and dimethylsulfoxide (5ml) were added to a 50 mL single-necked flask. The reaction was carried out at 60 °C overnight. The reaction was monitored to completion by LC-MS, water was added to the reaction solution, and the resulting mixture was extracted three times with ethyl acetate, dried with anhydrous sodium sulfate, filtered, concentrated, and 72 mg of product was obtain by Prep-TLC. $^1$H NMR (400 MHz, $CDCl_3$) $\delta$ 8.40 (s, 1H), 7.66 (d, *J* = 8.2 Hz, 2H), 7.39 -7.30 (m, 3H), 6.75 (t, *J* = 75.1 Hz, 1H), 5.76 (t, *J* = 8.5 Hz, 1H), 5.45 (d, *J* = 54.7 Hz, 1H), 3.94 - 3.57 (m, 3H), 2.79 - 2.49 (m, 3H), 2.46 (s, 3H), 2.31 - 2.07 (m, 3H), 2.06 - 1.67 (m, 7H), 1.13 (s, 3H).

MS[M+1]:564.6.

## Example 9

The compound synthesized in the present invention:

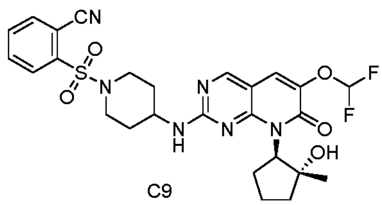
C9

The synthetic route and experimental procedure are as follows:

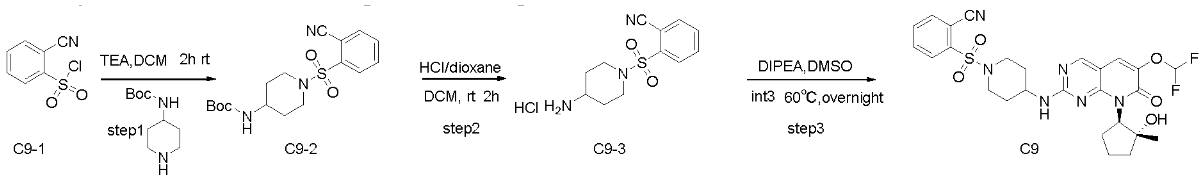

C9-1 C9-2 C9-3 C9

Synthesis of C9-2

4-Boc-aminopiperidine (300 mg, 1.0eq), dichloromethane (6ml), triethylamine (303mg, 2.0eq) were added to a 50mL single-necked flask, the temperature was cooled to 0 °C and a solution of C5-1 (303 mg, 1.01eq) in dichloromethane (2 ml) was added dropwise, then the mixture was naturally warmed up to room temperature and reacted for

2h. After the reaction was completed, water was added to the reaction solution, and the resulting mixture was extracted three times with dichloromethane, the organic phase was dried with anhydrous sodium sulfate, filtered, concentrated, and 550mg of product was obtained by column chromatography with petroleum ether:ethyl acetate=5:1 to 1:1.

Synthesis of C9-3

C9-2 (550mg, 1.0eq) and dichloromethane (8ml) were added to a 50mL single-necked flask, and then HCl/dioxane (6ml) was added at room temperature. The reaction was carried out for 2h at room temperature. The reaction was monitored to completion by TLC, the resulting mixture was filtered and the filter cake was collected to obtain 443mg.

Synthesis of C9

C9-3 (93mg, 1.5eq), int3 (80mg, 1.0eq), diisopropylethylamine (79.3mg, 3.0eq), and dimethylsulfoxide (5ml) were added to a 50 mL single-necked flask. The reaction was carried out at 60 °C overnight. The reaction was monitored to completion by LC-MS, water was added to the reaction solution, and the resulting mixture was extracted three times with ethyl acetate, dried with anhydrous sodium sulfate, filtered, concentrated, and 39 mg of product was obtain by Prep-TLC. $^1H$ NMR (400 MHz, $CDCl_3$) δ 8.42 (s, 1H), 8.08 (d, *J* = 7.8 Hz, 1H), 7.90 (d, *J* = 7.5 Hz, 1H), 7.75 (dt, *J* = 21.6, 7.5 Hz, 2H), 7.34 (s, 1H), 6.76 (t, *J* = 75.1 Hz, 1H), 5.79 (t, *J* = 8.5 Hz, 1H), 5.51 (s, 1H), 3.92 (d, *J*= 13.1 Hz, 3H), 2.90 (d, *J* = 54.2 Hz, 2H), 2.70 (dd, *J* = 19.2, 8.6 Hz, 1H), 2.24 (m, *J* = 36.2, 20.3, 9.9 Hz, 4H), 2.07 - 1.68 (m, 6H), 1.14 (s, 3H).

MS[M+1]:575.6.

## Example 10

The compound synthesized in the present invention:

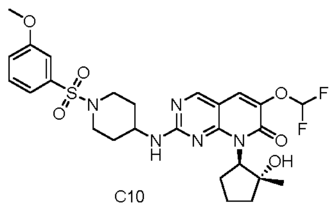

C10

The synthetic route and experimental procedure are as follows:

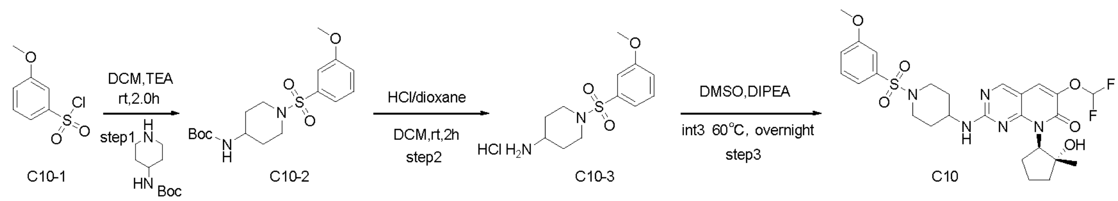


Synthesis of C10-2

4-Boc-aminopiperidine (300mg, 1.0eq), dichloromethane (6ml), and triethylamine (303mg, 2.0eq) were added to a 50mL single-necked flask, the temperature was cooled to 0 °C and C6-1 (313mg, 1.01eq) was added dropwise, then the mixture was naturally warmed up to room temperature and reacted for 2h. After the reaction was completed, water was added to the reaction solution, and extracted three times with dichloromethane, the organic phase was dried with anhydrous sodium sulfate, filtered, concentrated, and 530mg of product was obtained by column chromatography with PE : EA=5:1 to 1:1.

Synthesis of C10-3

C10-2 (530mg, 1.0eq) and dichloromethane (8ml) were added to a 50mL single-necked flask, and then HCl/dioxane (6ml) was added at room temperature. The reaction was carried out for 2h at room temperature. The reaction was monitored to completion by TLC, the resulting mixture was filtered and the filter cake was collected to obtain 380mg.

Synthesis of C10

C10-3 (94.5mg, 1.5eq), int3 (80mg, 1.0eq), diisopropylethylamine (79.3mg, 3.0eq), and dimethylsulfoxide (5ml) were added to a 50 mL single-necked flask. The reaction was carried out at 60 °C overnight. The reaction was monitored to completion by LC-MS, water was added to the reaction solution, and the resulting mixture was extracted three times with ethyl acetate, dried with anhydrous sodium sulfate, filtered, concentrated, and 38 mg of product was obtain by Prep-TLC. $^1$H NMR (400 MHz, $CDCl_3$) δ 8.40 (s, 1H), 7.46 (t, *J* = 8.0 Hz, 1H), 7.40 - 7.30 (m, 2H), 7.28 (s, 1H), 7.15 (dd, *J* = 8.2, 1.9 Hz, 1H), 6.75 (t, *J* = 75.1 Hz, 1H), 5.76 (t, *J* = 8.5 Hz, 1H), 5.36 (d, *J* = 45.1 Hz, 1H), 3.94 - 3.63 (m, 6H), 2.71 (s, 3H), 2.33 - 2.08 (m, 3H), 2.07 - 1.65 (m, 7H), 1.14 (s, 3H). MS[M+1]:580.6.

**Example 11**

The compound synthesized in the present invention:

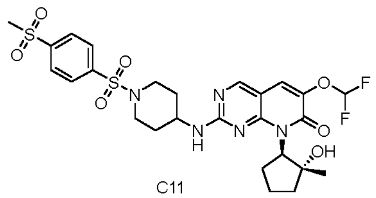

C11

The synthetic route and experimental procedure are as follows:

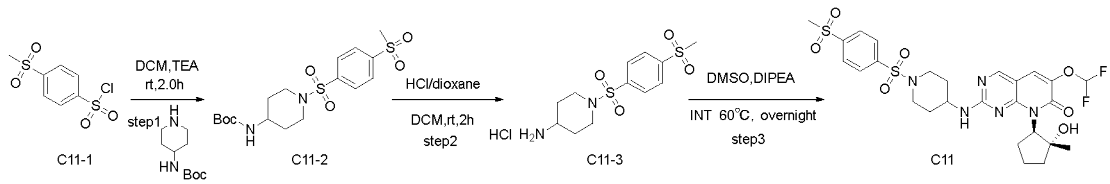


Synthesis of C11-2

4-Boc-aminopiperidine (300 mg, 1.0eq), DCM (6ml), and TEA (303mg, 2.0eq) were added to a 50mL single-necked flask, the temperature was cooled to 0 °C and a solution of C7-1 (385.8 mg, 1.01eq) in DCM (3.0ml) was added dropwise, then the mixture was naturally warmed up to room temperature and reacted for 2h. After the reaction was completed, water was added to the reaction solution, and the resulting mixture was extracted three times with DCM, the organic phase was dried with anhydrous sodium sulfate, filtered, concentrated, and 500mg of product was obtained by column chromatography with PE : EA=5:1 to 1:1.

Synthesis of C11-3

C7-2 (500mg, 1.0eq) and DCM (8ml) was added to a 50mL single-necked flask, and then HCl/dioxane (6ml) was added at room temperature. The reaction was carried out for 2h at room temperature. The reaction was monitored to completion by TLC, the resulting mixture was filtered and the filter cake was collected to obtain 383mg.

Synthesis of C11

C11-3 (109.3mg, 1.5eq), INT (80mg, 1.0eq), DIPEA (79.3mg, 3.0eq), and DMSO (5ml) were added to a 50 mL single-necked flask. The reaction was carried out at 60 °C overnight. The reaction was monitored to completion by LC-MS, water was added to the reaction solution, and the resulting mixture was extracted three times with EA, dried with anhydrous sodium sulfate, filtered, concentrated, and 29mg of product was obtain by Prep-TLC. $^1$H NMR (400 MHz, $CDCl_3$) δ 8.40 (s, 1H), 8.14 (d, *J* = 8.3 Hz, 2H), 7.99 (d, *J* = 8.3 Hz, 2H), 7.33 (s, 1H), 6.75 (t, *J* = 75.1 Hz, 1H), 5.77 (t, *J* = 8.5 Hz, 1H), 5.47 (s, 1H), 3.83 (d, *J* = 38.5 Hz, 3H), 3.14 (s, 3H), 2.66 (dt, *J* = 41.4, 21.1 Hz, 3H), 2.24 (m, *J* = 37.0, 20.3, 9.8 Hz, 3H), 2.07 - 1.67 (m, 7H), 1.13 (s, 3H). MS[M+1]:628.8.

**Example 12**

The compound synthesized in the present invention:

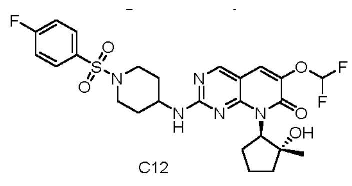

C12

The synthetic route and experimental procedure are as follows:

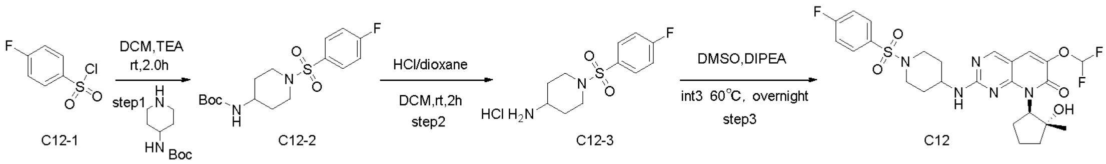


Synthesis of C12-2

4-Boc-aminopiperidine (1.0g, 1.0eq), dichloromethane (10 ml), and triethylamine (1.01g, 2.0eq) were added to a 100mL single-necked flask, the temperature was cooled to 0 °C and a solution of C8-1 (1.18g, 1.0eq) in dichloromethane (2 ml) was added dropwise, and the mixture was naturally warmed up to room temperature and reacted for 2h. After the reaction was completed, water was added to the reaction solution, and the resulting mixture was extracted three times with dichloromethane, the organic phase was dried with anhydrous sodium sulfate, filtered, concentrated, and 1.64g of product was obtained by column chromatography with petroleum ether:ethyl acetate=5:1 to 1:1.

Synthesis of C12-3

C12-2 (1.64g, 1.0eq) and dichloromethane (16ml) was added to a 100mL single-necked flask, and then HCl/dioxane (10ml) was added at room temperature. The reaction was carried out for 2h at room temperature. The reaction was monitored to completion by TLC, the resulting mixture was filtered and the filter cake was collected to obtain 1.32g.

Synthesis of C12

C12-3 (120 mg, 2.0eq), int3 (80mg, 1.0eq), diisopropylethylamine (80mg, 3.0eq), and dimethylsulfoxide (3ml) were added to a 50 mL single-necked flask. The reaction was carried out at 60 °C overnight. The reaction was monitored to completion by LC-MS, water was added to the reaction solution, and the resulting mixture was extracted three times with ethyl acetate, dried with anhydrous sodium sulfate, filtered, concentrated, and 42 mg of product was obtain by Prep-TLC. $^1$H NMR (400 MHz, $CDCl_3$) $\delta$ 8.41 (s, 1H), 7.80 (m, $J$ = 7.9, 5.0, 2.4 Hz, 2H), 7.34 (s, 1H), 7.26 - 7.20 (m, 2H), 6.75 (t, $J$ = 75.0 Hz, 1H), 5.76 (t, $J$ = 8.5 Hz, 1H), 5.40 (s, 1H), 3.81 (d, $J$ = 37.9 Hz, 3H), 2.62 (d, $J$ = 63.5 Hz, 3H), 2.41 - 2.08 (m, 4H), 2.08 - 1.64 (m, 6H), 1.15 (d, $J$ = 12.6 Hz, 3H). MS[M+1]:568.5.

**Example 13**

The compound synthesized in the present invention:

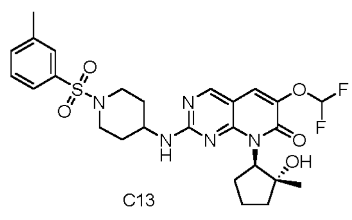

C13

The synthetic route and experimental procedure are as follows:

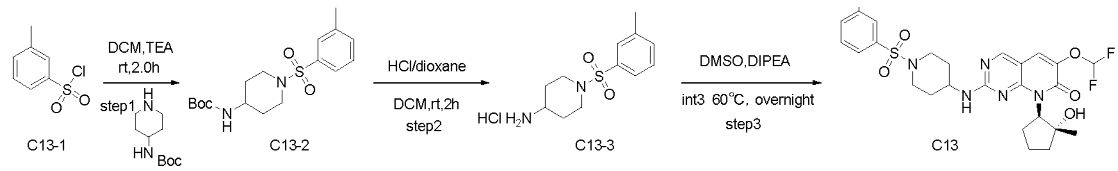


Synthesis of C13-2

4-Boc-aminopiperidine (1.0g, 1.0eq), dichloromethane (10 ml), triethylamine (1.01g, 2.0eq) were added to a 100mL single-necked flask, the temperature was cooled to 0 °C and a solution of C9-1 (1.0g, 1.05eq) in dichloromethane (2 ml) was added dropwise, then the mixture was naturally warmed up to room temperature and reacted for 2h. After the reaction was completed, water was added to the reaction solution, and the resulting mixture was extracted three times with dichloromethane, the organic phase was dried with anhydrous sodium sulfate, filtered, concentrated, and 1.74g of product was obtained by column chromatography with PE : EA=5:1 to 1:1.

Synthesis of C13-3

C9-2 (1.74g, 1.0eq) and dichloromethane (17ml) were added to a 100mL single-necked flask, and then HCl/dioxane (10ml) was added at room temperature. The reaction was carried out for 2h at room temperature. The reaction was monitored to completion by TLC, the resulting mixture was filtered and the filter cake was collected to obtain 1.31g.

Synthesis of C13

C9-3 (149.5mg, 2.0eq), int3 (100mg, 1.0eq), diisopropylethylamine (105.7mg, 4.0eq), and dimethylsulfoxide (5ml) were added to a 50 mL single-necked flask. The reaction was carried out at 60 °C overnight. The reaction was monitored to completion by LC-MS, water was added to the reaction solution, and the resulting mixture was extracted three times with ethyl acetate, dried with anhydrous sodium sulfate, filtered, concentrated, and 63 mg of product was obtain by Prep-TLC. $^1$H NMR (400 MHz, $CDCl_3$) $\delta$ 8.43 (s, 1H), 7.58 (d, $J$ = 4.9 Hz, 2H), 7.45 (t, $J$ = 6.8 Hz, 2H), 7.38 (s, 1H), 6.74 (t, $J$ = 75.2 Hz, 1H), 5.87 - 5.40 (m, 2H), 3.75 (d, $J$ = 72.5 Hz, 3H), 2.59 (d, $J$ = 61.7 Hz, 3H), 2.46 (s, 3H), 2.21 - 1.94 (m, 4H), 1.75 (d, $J$ = 61.1 Hz, 6H), 1.21 (d, $J$ = 32.5 Hz, 3H). MS[M+1]:564.6.

## Example 14

The compound synthesized in the present invention:

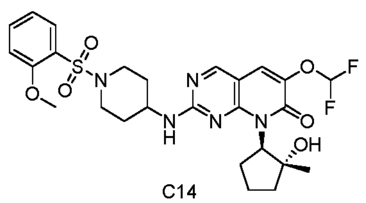

C14

The synthetic route and experimental procedure are as follows:

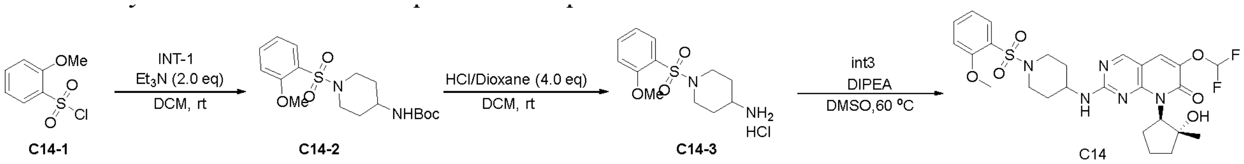


Synthesis of C14-2

C14-1 (500 mg, 2.4mmol) was added to a 50 mL single-necked flask, and 10 mL of dichloromethane was added to dissolve with stirring, then 4-Boc-aminopiperidine (480 mg, 2.4 mmol) was added and homogeneous stirred, then triethylamine (485 mg, 4.8 mmol) was added, and the reaction was carried out at room temperature with stirring overnight.

After the mixture was fully reacted, the reaction was concentrated under reduced pressure and slurried with appropriate amount of petroleum ether/ethyl acetate to obtain 942 mg of product as a white solid.

Synthesis of C14-3

C14-2 (942 mg, 2.54 mmol) was added to a 100 mL round-bottomed flask, dichloromethane (20 mL) was added to dissolve it with stirring, and then HCl/dioxane (6 mL, 24 mmol) was added dropwise, after dropping, the reaction was carried out for 2h, and reaction was monitored to completion by TLC. The reaction solution was then concentrated under reduced pressure and slurried with appropriate amount of petroleum ether/ethyl acetate to obtain 730 mg of product.

Synthesis of C14

Int3 (60 mg, 0.154 mmol) was added to a 10 mL sealed tube, followed by addition of dimethyl sulfoxide (3 mL), the mixture was stirred well, then C14-3 (94.4 mg, 0.308 mmol) was added, the mixture was heated to 60 °C and stirred overnight. The reaction was then cooled to room temperature, appropriate amount of water and ethyl acetate were added to extract the organic phase, the organic phase was dried with anhydrous sodium sulfate, concentrated under reduced pressure, and 61 mg was obtained by column chromatography (petroleum ether: ethyl acetate=1:1), then slurried and purified to obtain 37 mg of product.

## Example 15

The compound synthesized in the present invention:

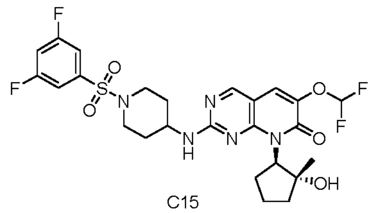

C15

The synthetic route and experimental procedure are as follows:

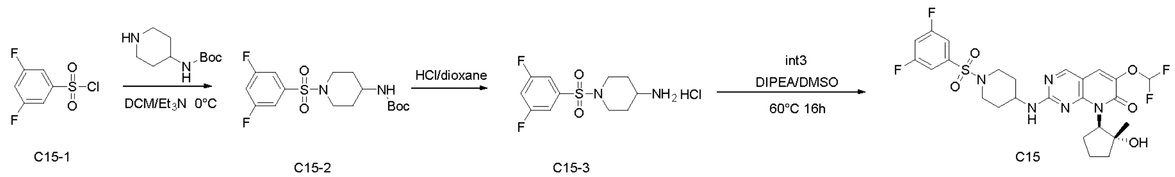


Synthesis of C15-2

C15-1 (1 g, 4.7 mmol), triethylamine (1.25 mL, 9.4 mmol) and dichloromethane (5 mL) were added to a 100 mL single-necked flask, and a solution of 4-Boc-aminopiperidine (0.94 g, 4.7 mmol) in dichloromethane (5 mL) was added dropwise at 0 °C, and then the mixture was warmed up to room temperature and reacted for 2h. The reaction was monitored to completion by LC-MS, water was added to the reaction solution, and the resulting mixture was extracted three times with ethyl acetate, dried with anhydrous sodium sulfate, filtered, and concentrated to obtain 1.73 g of product. MS [M+1]:377

Synthesis of C15-3

C15-2 (1.73 g, 4.6 mmol), and dichloromethane solution(20 mL) were added to a 100 mL single-necked flask, and then HCl/dioxane (10 mL) was added dropwise at room temperature with stirring, then the reaction was carried out for 2h. The reaction was monitored to completion by LC-MS, the reaction solution was concentrated, a small amount of ethyl acetate and a large amount of petroleum ether were added for sonication, and the resulting mixture was washed, then filtered and dried to obtain 1.47 g of crude product. MS[M+1]:277

Synthesis of C15

C15-3 (131 mg, 0.42 mmol), int3 (80 mg, 0.21 mmol), diisopropylethylamine (0.15 mL, 0.84 mmol) and dimethyl sulfoxide (3 mL) were added to a 50 mL single-necked flask, then the temperature was elevated to 60 °C and the reaction was carried out for 16 h. After the reaction was complete, water was added to the reaction solution, and the resulting mixture was extracted three times with ethyl acetate, dried with anhydrous sodium sulfate, filtered, concentrated, purified by Prep-TLC, and 69 mg of product was obtained (developer is dichloromethane:methanol=60:1) .MS[M+1]:567 1H NMR (400 MHz, $CDCl_3$) δ 8.41 (s, 1H), 7.32 (d, J = 7.3 Hz, 3H), 7.08 (t, J = 8.6 Hz, 1H), 6.76 (s, 1H), 5.78 (t, J = 8.5 Hz, 1H), 5.36 (s, 1H), 3.76 (m, 3H), 2.70 (m, 3H), 2.17 (d, J = 11.7 Hz, 4H), 2.01 (d, J = 11.5 Hz, 3H), 1.80 (m,3H),1.14 (s, 3H).

**Example 16**

The compound synthesized in the present invention:

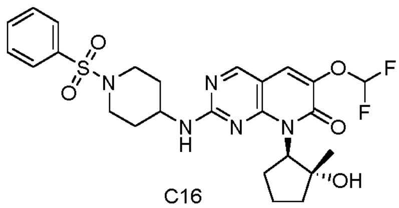

C16

The synthetic route and experimental procedure are as follows:

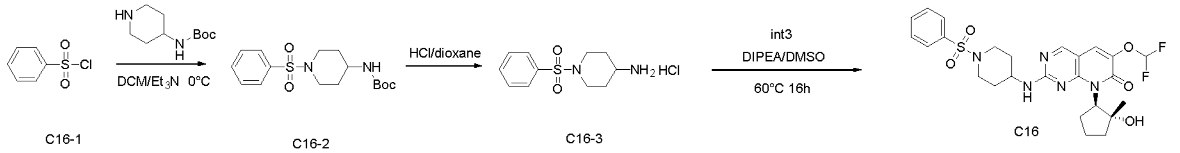


Synthesis of C16-2

C16-1 (883 mg, 5 mmol), triethylamine (1.38 mL, 10 mmol) and dichloromethane (5 mL) were added to a 100 mL single-necked flask, and a solution of 4-Boc-aminopiperidine (1 g, 5 mmol) in dichloromethane (5 mL) was added dropwise at 0 °C, and then the mixture was warmed up to room temperature and reacted for 2h. The reaction was monitored to completion by LC-MS, water was added to the reaction solution, and the resulting mixture was extracted three times with EA, dried with anhydrous sodium sulfate, filtered, and concentrated to obtain 1.64 g of product. MS[M+1] :340

Synthesis of C16-3

C16-2 (1.64 g, 4.8 mmol), and dichloromethane solution (10 mL) were added to a 100 mL single-necked flask, and then HCl/dioxane (10 mL) was added dropwise at room temperature with stirring, after dropping, then the reaction was carried out for 2h. The reaction was monitored to completion by LC-MS, the reaction solution was concentrated, a small amount of ethyl acetate and a large amount of petroleum ether were added for sonication, and the resulting mixture was washed, then filtered and dried to obtain 1.32 g of crude product. MS[M+1]:240

Synthesis of C16

C16-3 (143.5 mg, 0.52 mmol), int3 (100 mg, 0.26 mmol), diisopropylethylamine (0.18 mL, 1.04 mmol) and dimethyl sulfoxide (3 mL) were added to a 50 mL single-necked flask, then the temperature was heated to60 °C the reaction was carried out for 16 h. After the reaction was complete, water was added to the reaction solution, and the resulting mixture was extracted three times with ethyl acetate, dried with anhydrous sodium sulfate, filtered, concentrated, purified by Prep-TLC (developer is dichloromethane:methanol=60:1), and 67 mg of product was obtained. MS[M+1]:548 1H NMR (400 MHz, $CDCl_3$) δ 8.39 (s, 1H), 7.79 (d, J = 8.0 Hz, 2H), 7.64 (t, J = 7.4 Hz, 1H), 7.56 (t, J = 7.7 Hz, 2H), 7.33 (s, 1H), 6.75 (s, 1H), 5.76 (t, J = 8.6 Hz, 1H),5.30(s, 1H), 3.79 (d, J = 34.0 Hz, 3H), 2.70 (m, 3H), 2.06 (dd, J = 62.3, 10.2 Hz, 10H), 1.13 (s, 3H).

## Example 17

The compound synthesized in the present invention:

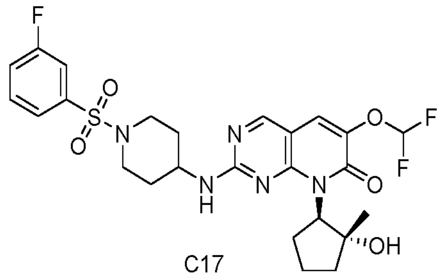

C17

The synthetic route and experimental procedure are as follows:

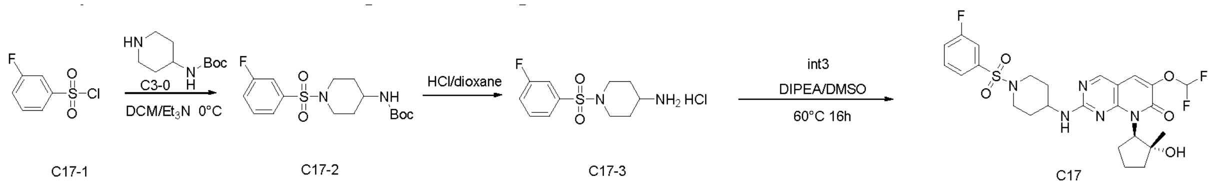


Synthesis of C17-2

C17-1 (1 g, 5 mmol), triethylamine (2 mL, 10 mmol) and dichloromethane (5 mL) were added to a 100 mL single-necked flask, and a solution of 4-Boc-aminopiperidine (1 g, 5 mmol) in dichloromethane (5 mL) was added dropwise at 0 °C, and then the mixture was warmed up to room temperature and reacted for 2h. The reaction was monitored to completion by LC-MS, water was added to the reaction solution, and the resulting mixture was extracted three times with EA, dried with anhydrous sodium sulfate, filtered, and concentrated to obtain 1.8 g of product. MS[M+1]:259

Synthesis of C17-3

C17-2 (1.8 g, 5 mmol), and dichloromethane solution (18 mL) were added to a 100 mL single-necked flask, and then HCl/dioxane (10 mL) was added dropwise at room temperature with stirring, after dropping, the reaction was carried out for 2h. The reaction was monitored to completion by LC-MS, the reaction solution was concentrated, a small amount of ethyl acetate and a large amount of petroleum ether were added for sonication, and the resulting mixture was washed, then filtered and dried to obtain 1.57 g of crude product. MS[M+1]:259

Synthesis of C17

C17-3 (118 mg, 0.40 mmol), int3 (77 mg, 0.20 mmol), diisopropylethylamine (0.14 mL, 0.80 mmol) and dimethyl sulfoxide (3 mL) were added to a 50 mL single-necked flask, then the mixture was heated to 60 °C and the reaction was carried out for 16 h. After the reaction was complete, water was added to the reaction solution, and the resulting mixture was extracted three times with ethyl acetate, dried with anhydrous sodium sulfate, filtered, concentrated, purified by Prep-TLC (developer is dichloromethane:methanol =60:1), and 29 mg of product was obtained. MS[M+1]:604 $^1H$ NMR (400 MHz, $CDCl_3$) $\delta$ 8.40 (s, 1H), 7.62 - 7.46 (m, 3H), 7.33 (s, 2H), 6.75 (s,1H), 5.77 (t, J = 8.5 Hz, 1H), 5.40(s, 1H),3.82 (d, J = 45.3 Hz, 3H), 2.70 (s, 3H), 2.15 (d, J = 12.9 Hz, 3H), 2.09 - 1.84 (m, 4H), 1.78 (s, 3H), 1.14 (s, 3H).

## Example 18

The compound synthesized in the present invention:

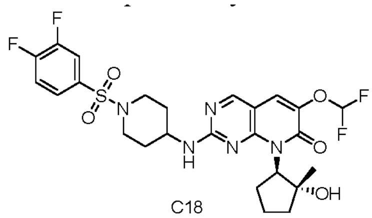

C18

The synthetic route and experimental procedure are as follows:

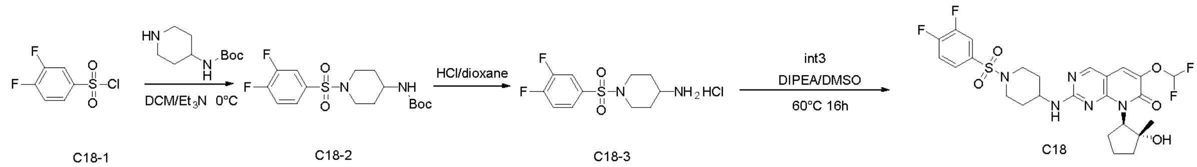


C18-1 C18-2 C18-3 C18

Synthesis of C18-2

C18-1 (1.06 g, 5 mmol), triethylamine (1.38 mL, 10 mmol) and dichloromethane (5 mL) were added to a 100 mL single-necked flask, and a solution of 4-Boc-aminopiperidine (1 g, 5 mmol) in dichloromethane (5 mL) was added dropwise at 0 °C, and then the mixture was warmed up to room temperature and reacted for 2h. The reaction was monitored to completion by LC-MS, water was added to the reaction solution, and the resulting mixture was extracted three times with ethyl acetate, dried with anhydrous sodium sulfate, filtered, and concentrated to obtain 1.69 g of product. MS[M+1]:377

Synthesis of C18-3

C18-2 (1.69 g, 4.5 mmol), and ethyl acetate solution (20 mL) were added to a 100 mL single-necked flask, and then HCl/dioxane (10 mL) was added dropwise at room temperature with stirring, after dropping, the reaction was carried out for 2h. The reaction was monitored to completion by LC-MS, the reaction solution was concentrated, a small amount of ethyl acetate and a large amount of petroleum ether were added for sonication, and the resulting mixture was washed, then filtered and dried to obtain 1.57 g of crude product. MS[M+1]:277

Synthesis of C18

C18-3 (162 mg, 0.52 mmol), int (100 mg, 0.26 mmol), diisopropylethylamine (0.18 mL, 1.04 mmol) and dimethyl sulfoxide (3 mL) were added to a 50 mL single-necked flask, then the mixture was heated to 60 °C and the reaction was carried out for 16 h. After the reaction was complete, water was added to the reaction solution , and the resulting mixture was extracted three times with ethyl acetate, dried with anhydrous sodium sulfate, filtered, concentrated, purified by Prep-TLC (developer is dichloromethane:methanol=60:1), and 55 mg of product was obtained. MS[M+1]:567 1H NMR (400 MHz, $CDCl_3$) $\delta$ 8.34 (s, 1H), 7.56 (t, J = 8.2 Hz, 1H), 7.50 (d, J = 8.8 Hz, 1H), 7.35 - 7.24 (m, 2H), 6.75 (s,1H), 5.70 (t, J = 8.6 Hz, 1H), 5.35(s, 1H), 3.74 (d, J = 53.6 Hz, 3H), 2.63 (s, 3H), 2.28 - 2.04 (m, 4H), 2.03 - 1.89 (m, 3H), 1.89 - 1.71 (m, 3H), 1.07 (s, 3H).

## Example 19

The compound synthesized in the present invention:

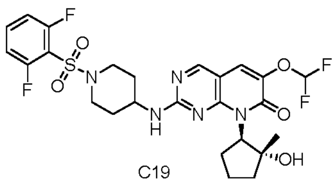

C19

The synthetic route and experimental procedure are as follows:

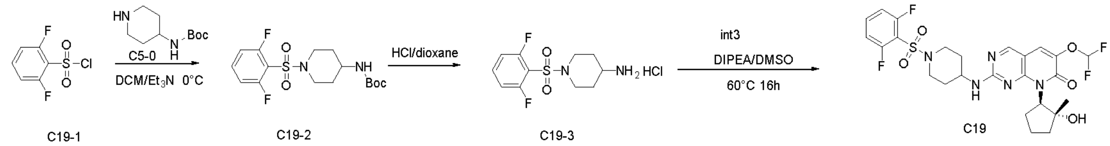


C19-1 C19-2 C19-3 C19

Synthesis of C19-2

C19-1 (1 g, 4.7 mmol), triethylamine (1.25 mL, 9.4 mmol) and dichloromethane (5 mL) were added to a 100 mL single-necked flask, and a solution of 4-Boc-aminopiperidine (0.94 g, 4.7 mmol) in dichloromethane (5 mL) was added dropwise at 0 °C, and then the mixture was warmed up to room temperature and reacted for 2h. The reaction was monitored to completion by LC-MS, water was added to the reaction solution, and the resulting mixture was extracted three times with ethyl acetate, dried with anhydrous sodium sulfate, filtered, and concentrated to obtain 2.35 g of product. MS[M+1]:377

Synthesis of C19-3

C19-2 (2.35 g, 6.2 mmol), and dichloromethane solution (20 mL) were added to a 100 mL single-necked flask, and then HCl/dioxane (10 mL) was added dropwise at room temperature with stirring, after dropping, the reaction was carried out for 2h. The reaction was monitored to completion by LC-MS, the reaction solution was concentrated, a small amount of ethyl acetate and a large amount of petroleum ether were added for sonication, and the resulting mixture was washed, then filtered and dried to obtain 1.47 g of crude product. MS[M+1]:277

Synthesis of C19

C19-3 (162 mg, 0.52 mmol), int3 (100 mg, 0.26 mmol), diisopropylethylamine (0.18 mL, 1.04 mmol) and dimethyl sulfoxide (3 mL) were added to a 50 mL single-necked flask, then the mixture was heated to 60 °C and the reaction was carried out for 16 h. After the reaction was complete, water was added to the reaction solution, and the resulting mixture was extracted three times with ethyl acetate, dried with anhydrous sodium sulfate, filtered, concentrated, purified by Prep-TLC (developer is dichloromethane:methanol=60:1), and 93 mg of product was obtained. MS[M+1]:567 1H NMR (400 MHz, $CDCl_3$) δ 8.35 (s, 1H), 7.48(m,1H),7.27 (s, 1H), 6.98 (t, J = 8.9 Hz, 2H), 6.75 (s,1H), 5.71 (d, J = 8.7 Hz, 1H), 5.01 (s, 1H), 3.88 (d, J = 11.9 Hz, 3H), 2.87 (s, 2H), 2.64 (s, 1H), 2.11 (d, J = 10.3 Hz, 3H), 1.96 (d, J = 14.7 Hz, 4H), 1.72 (s, 3H), 1.08 (s, 3H).

## Example 20

The compound synthesized in the present invention:

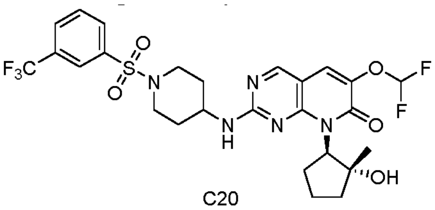

C20

The synthetic route and experimental procedure are as follows:

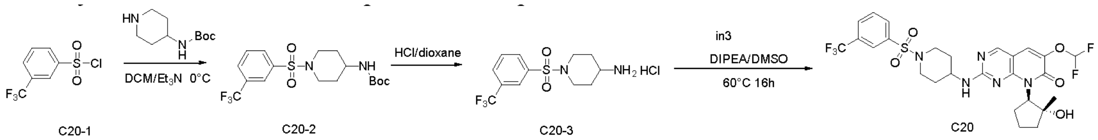


C20-1 C20-2 C20-3 C20

Synthesis of C20-2

C20-1 (0.5 g, 2.04 mmol), triethylamine (0.54 mL, 4.08 mmol) and dichloromethane (5 mL) were added to a 100 mL single-necked flask, and a solution of 4-Boc-aminopiperidine (0.4 g, 2.04 mmol) in dichloromethane (5 mL) was

added dropwise at 0 °C, and then the mixture was warmed up to room temperature and reacted for 2h. The reaction was monitored to completion by LC-MS, water was added to the reaction solution, and the resulting mixture was extracted three times with ethyl acetate, dried with anhydrous sodium sulfate, filtered, and concentrated to obtain 850 mg of product. MS[M+1]:409

Synthesis of C20-3

C20-2 (850 mg, 2.08 mmol), and dichloromethane solution (10 mL) were added to a 100 mL single-necked flask, and then HCl/dioxane (3.8 mL, ) was added dropwise at room temperature with stirring, after dropping, the reaction was carried out for 2h. The reaction was monitored to completion by LC-MS, the reaction solution was concentrated, a small amount of ethyl acetate and a large amount of petroleum ether were added for sonication, and the resulting mixture was washed, then filtered and dried to obtain 692 mg of crude product. MS[M+1]:345

Synthesis of C20

C20-3 (144.5 mg, 0.42 mmol), int (80 mg, 0.21 mmol), diisopropylethylamine (0.15 mL, 0.84 mmol) and dimethyl sulfoxide (2 mL) were added to a 50 mL single-necked flask, then the mixture was heated to 60 °C and the reaction was carried out for 16 h After the reaction was complete, water was added to the reaction solution, and the resulting mixture was extracted three times with ethyl acetate, dried with anhydrous sodium sulfate, filtered, concentrated, purified by Prep-TLC (developer is dichloromethane:methanol=60:1), and 83 mg of product was obtained. MS[M+1]:618 1H NMR (400 MHz, $CDCl_3$) δ 8.40 (s, 1H), 8.04 (s, 1H), 7.98 (d, J = 7.7 Hz, 1H), 7.90 (d, J = 7.8 Hz, 1H), 7.72 (t, J = 7.9 Hz, 1H), 7.33 (s, 1H), 6.76 (t, J = 75.1 Hz, 1H), 5.76 (t, J = 8.6 Hz, 1H), 5.30 (s, 1H), 3.83 (d, J = 33.6 Hz, 3H), 2.70 (m, 3H), 2.33 - 2.10 (m,3H), 1.99 (m, 4H), 1.89 (m, 3H), 1.13 (s, 3H).

## Example 21

The compound synthesized in the present invention:

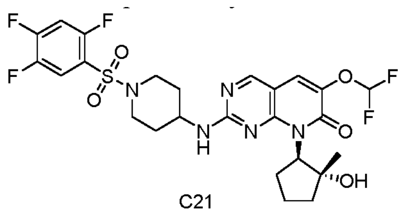

C21

The synthetic route and experimental procedure are as follows:

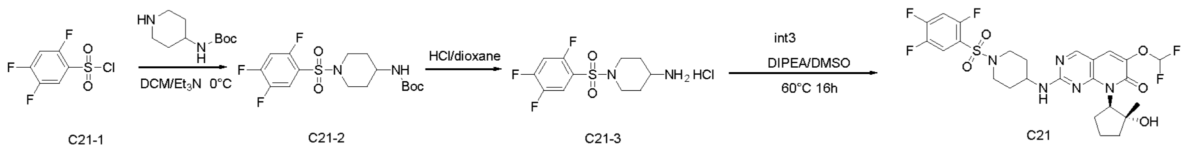


Synthesis of C21-2

C21-1 (0.5 g, 2.17 mmol), triethylamine (0.58 mL, 4.34 mmol) and dichloromethane (5 mL) were added to a 100 mL single-necked flask, and a solution of 4-Boc-aminopiperidine (0.43 g, 2.17 mmol) in dichloromethane (5 mL) was added dropwise at 0 °C, and then the mixture was warmed up to room temperature and reacted for 2h. The reaction was monitored to completion by LC-MS, water was added to the reaction solution was added water, and the resulting mixture was extracted three times with ethyl acetate, dried with anhydrous sodium sulfate, filtered, and concentrated to obtain 860 mg of product. MS[M+1]:395

Synthesis of C21-3

C21-2 (860 mg, 2.18 mmol), and dichloromethane solution (10 mL) were added to a 100 mL single-necked flask, and then HCl/dioxane (4 mL) was added dropwise at room temperature with stirring, after dropping, the reaction was carried out for 2h. The reaction was monitored to completion by LC-MS, the reaction solution was concentrated, a

small amount of ethyl acetate and a large amount of petroleum ether were added for sonication, and the resulting mixture was washed, then filtered and dried to obtain 692 mg of crude product. MS[M+1]:331

Synthesis of C21

C21-3 (139 mg, 0.42 mmol), int (80 mg, 0.21 mmol), diisopropylethylamine (0.15 mL, 0.84 mmol) and dimethyl sulfoxide (2 mL) were added to a 50 mL single-necked flask, then the mixture was heated to 60 °C and the reaction was carried out for 16 h. After the reaction was complete, water was added to the reaction solution, and the resulting mixture was extracted three times with ethyl acetate, dried with anhydrous sodium sulfate, filtered, concentrated, purified by Prep-TLC (developer is dichloromethane:methanol=60:1), and 46 mg of product was obtained. MS[M+1]:604 1H NMR (400 MHz, $CDCl_3$) δ 8.42 (s, 1H), 7.73 (q, J = 8.3 Hz, 1H), 7.34 (s, 1H), 7.12 (m, J = 9.3, 6.0 Hz, 1H), 6.76 (s, J = 75.1 Hz, 1H), 5.79 (t, J = 8.4 Hz, 1H), 5.50 (s, 1H),3.97 (m, 1H), 3.86 (d, J = 14.0 Hz, 2H), 2.90 (m, 2H), 2.71 (m, 1H), 2.38 - 2.11 (m, 4H), 2.10 - 1.97 (m, 3H), 1.80 (m, 3H),1.15 (s, 3H).

## Example 22

The compound synthesized in the present invention:

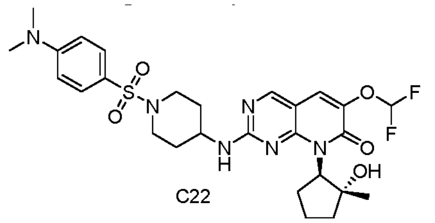

C22

The synthetic route and experimental procedure are as follows:

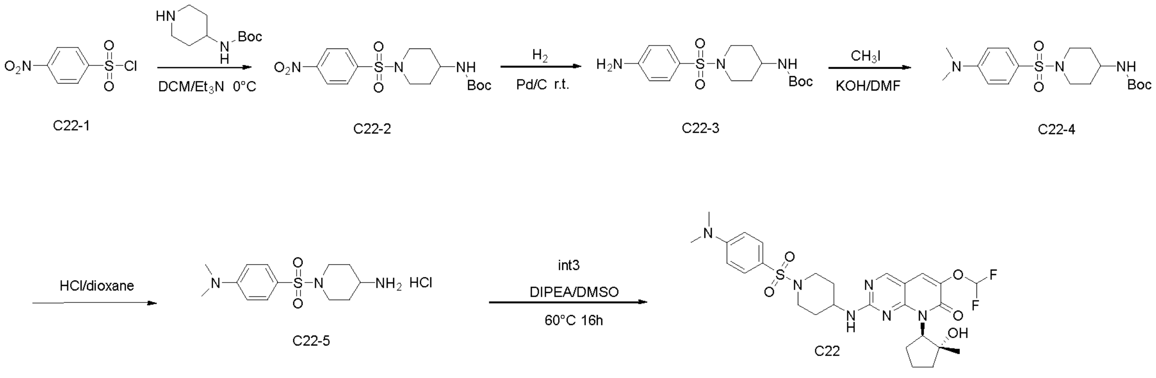


Synthesis of C22-2

C22-1 (2 g, 9 mmol), triethylamine (2.4 mL, 18 mmol) and dichloromethane (10 mL) were added to a 100 mL single-necked flask, and a solution of 4-Boc-aminopiperidine (1.81 g, 9 mmol) in dichloromethane (10 mL) was added dropwise at 0 °C, and then the mixture was warmed up to room temperature and reacted for 2h. The reaction was monitored to completion by LC-MS, water was added to the reaction solution, and the resulting mixture was extracted three times with ethyl acetate, dried with anhydrous sodium sulfate, filtered, and concentrated to obtain 2.2 g of product. MS[M+1]:386

Synthesis of C22-3

C22-2 (1 g, 2.6 mmol), methanol (10 mL) and Pd/C (0.14 g 10%) were added to a 100 mL single-necked flask, and the mixture was stirred at room temperature for 12 h under $H_2$ conditions. The reaction was monitored to completion by LC-MS, then the Pd/C was filtered out and the reaction solution was concentrated to obtain 857 mg of the product. MS[M+1]:356

Synthesis of C22-4

C22-3 (750 mg, 2.11 mmol) and N,N-dimethylformamide (20 mL), iodomethane (2 ml) were added to a 100 mL single-necked flask, and potassium hydroxide solution (0.3 g, 10 mL) was added dropwise at 0 °C, then the mixture was heated to 50 °C after warming to room temperature and stirred at reflux overnight. The reaction was monitored to completion by LC-MS, water was added to the reaction solution, and the resulting mixture was extracted three times with ethyl acetate, the organic phase was dried with anhydrous sodium sulfate, filtered, concentrated, and 80 mg of product was obtained by column chromatography with petroleum ether:ethyl acetate=3:1. MS[M+1]:384

Synthesis of C22-5

C22-4 (634 mg, 1.65 mmol), and dichloromethane solution (10 mL) were added to a 100 mL single-necked flask, and then HCl/dioxane (3 mL) was added dropwise at room temperature with stirring, after dropping, the reaction was carried out for 2h. The reaction was monitored to completion by LC-MS, the reaction solution was concentrated, a small amount of ethyl acetate and a large amount of petroleum ether were added for sonication, and the resulting mixture was washed, then filtered and dried to obtain 260 mg of crude product. MS[M+1]:256

Synthesis of C22

C22-5 (166 mg, 0.42 mmol), int3 (100 mg, 0.26 mmol), diisopropylethylamine (0.18 mL, 1.04 mmol) and dimethyl sulfoxide (3 mL) were added to a 50 mL single-necked flask, then the mixture was heated to 60 °C and the reaction was carried out for 16 h. After the reaction was complete, water was added to the reaction solution, and the resulting mixture was extracted three times with ethyl acetate, dried with anhydrous sodium sulfate, filtered, concentrated, purified with Prep-TLC (developer is dichloromethane:methanol=60:1), and 15 mg of product was obtained. MS[M+1]:593 1H NMR (400 MHz, $CDCl_3$) δ 8.39 (s, 1H), 7.61 (d, J = 8.8 Hz, 2H), 7.32 (s, 1H), 7.03 - 6.88 (m, 1H), 6.70 (d, J = 8.9 Hz, 2H), 5.81 - 5.69 (m, 1H), 5.15(m,1H),3.86 (d, J = 17.0 Hz, 3H), 3.07 (s, 6H), 2.72 (s, 3H), 2.40 - 2.17 (m, 2H), 2.04 (m, 1H), 2.00 (m, 4H), 1.89 (m, 3H), 1.13 (s,3H).

## Example 23

The compound synthesized in the present invention:

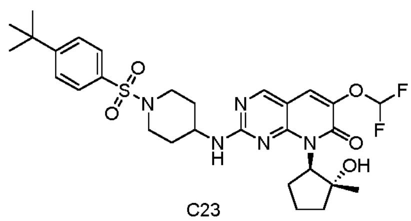

C23

The synthetic route and experimental procedure are as follows:

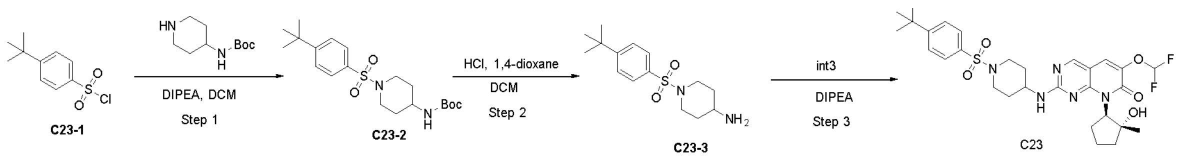


Synthesis of C23-2

Compound 4-Tert-butoxycarbonyl-aminopiperidine (500 mg, 1.00 eq), C19-1 (516 mg, 1.00 eq), and dichloromethane (5 mL) were added to a 50 mL single-necked flask, and triethylamine (504 mg, 1.00 eq) was added dropwise at 0°C, then the mixture was warmed up to room temperature and reacted for 3 h. The reaction was monitored to completion by TLC, then water was added to the reaction solution, and the resulting mixture was extracted three times with dichloromethane, the organic phase was dried with anhydrous sodium sulfate, filtered, concentrated, and the concentrated product was slurried with petroleum ether/ethyl acetate (10/1), and the slurry was filtered to obtain 950 mg.

Synthesis of C23-3

C23-2 (900mg, 1.0eq), and dichloromethane (20 ml) were added to a 100 mL single-necked flask, and then HCl/dioxane (5 ml, 3 eq) was added slowly. The reaction was carried out for 2h at room temperature. The reaction was monitored to completion by TLC, then the resulting mixture was concentrated, the concentrated product was slurried with petroleum ether, and the slurry was filtered to obtain 700 mg.

Synthesis of C23

Int3 (100mg, 1 eq), C23-3 (139mg, 2 eq), diisopropylethylamine (166mg, 2 eq), and dimethyl sulfoxide (5mL) were added to a 50 mL single-necked flask. The reaction was carried out at 60 °C for 16h. The reaction was monitored to completion by LC-MS, water was added to the reaction solution, and the resulting mixture was extracted three times with ethyl acetate, dried with anhydrous sodium sulfate, filtered, concentrated, purified by Prep-TLC, and 21 mg of product was obtained. $^1$H NMR (400 MHz, $CDCl_3$) δ 8.40 (s, 1H), 7.70 (d, *J* = 8.5 Hz, 2H), 7.63 - 7.51 (m, 2H), 7.33 (s, 1H), 6.75 (t, *J* = 75.1 Hz, 1H), 5.76 (t, *J* = 8.6 Hz, 1H), 5.43 (s, 1H), 3.80 (d, *J* = 38.4 Hz, 3H), 2.71 (s, 2H), 2.52 (s, 1H), 2.31 - 2.10 (m, 4H), 2.00 (q, *J* = 10.1, 9.4 Hz, 2H), 1.94 - 1.77 (m, 2H), 1.73 - 1.66 (m, 2H), 1.37 (s, 9H), 1.13 (s, 3H). MS[M+1]:606

**Example 24**

The compound synthesized in the present invention:

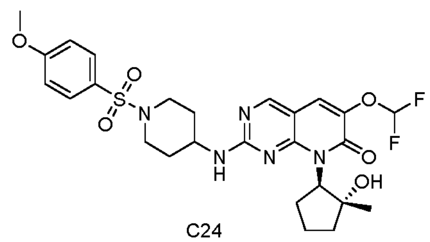

C24

The synthetic route and experimental procedure are as follows:

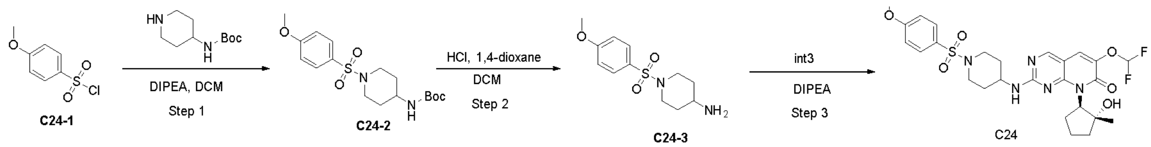


Synthesis of C24-2

The compound 4-tert-butoxycarbonyl-aminopiperidine (500 mg, 1.00 eq), C20-1 (516 mg, 1.00 eq), and dichloromethane (5 mL) were added to a 50 mL single-necked flask, and triethylamine (504 mg, 1.00 eq) was added dropwise at 0°C, then the mixture was warmed up to room temperature and reacted for 3 h. The reaction was monitored to completion by TLC, then water was added to the reaction solution, and the resulting mixture was extracted three times with dichloromethane, the organic phase was dried with anhydrous sodium sulfate, filtered, concentrated, and the concentrated product was slurried with petroleum ether/ethyl acetate (10/1), and the slurry was filtered to obtain 900 mg.

Synthesis of C24-3

C24-2 (900mg, 1.0eq) and dichloromethane (20 ml) were added to a 100 mL single-necked flask, and then HCl/dioxane (5 ml, 3 eq) was added slowly. The reaction was carried out for 2h at room temperature. The reaction was monitored to completion by TLC, then the resulting mixture was concentrated, the concentrated product was slurried with petroleum ether, and the slurry was filtered to obtain 650 mg.

Synthesis of C24

Int3 (100mg, 1 eq), C24-3 (139mg, 2 eq), diisopropylethylamine (166mg, 2 eq), and dimethyl sulfoxide (5mL)

were added to a 50 mL single-necked flask. The reaction was carried out at 60 °C for 16h. The reaction was monitored to completion by LC-MS, water was added to the reaction solution, and the resulting mixture was extracted three times with ethyl acetate, dried with anhydrous sodium sulfate, filtered, concentrated, purified with Prep-TLC, and 11 mg of product was obtained. $^1$H NMR (400 MHz, $CDCl_3$) δ 8.40 (s, 1H), 7.72 (d, *J* = 8.6 Hz, 2H), 7.33 (s, 1H), 7.02 (d, *J* = 8.6 Hz, 2H), 6.75 (t, *J* = 75.1 Hz, 1H), 5.76 (t, *J* = 8.5 Hz, 1H), 5.42 (s, 1H), 3.90 (s, 3H), 3.87 -3.62 (m, 3H), 2.62 (d, *J* = 67.4 Hz, 3H), 2.31-2.20 (m, 1H), 2.13 (d, *J* = 12.8 Hz, 2H), 2.01 (dd, *J* = 16.1, 6.9 Hz, 3H), 1.90 (t, *J* = 9.2 Hz, 1H), 1.79 (s, 1H), 1.26 (t, *J* = 7.2 Hz, 1H), 1.14 (s, 3H), 0.92-0.82 (m, 2H). MS[M+1]:580

## Example 25

The compound synthesized in the present invention:

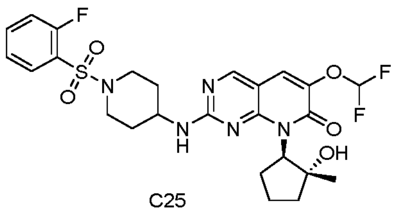

C25

The synthetic route and experimental procedure are as follows:

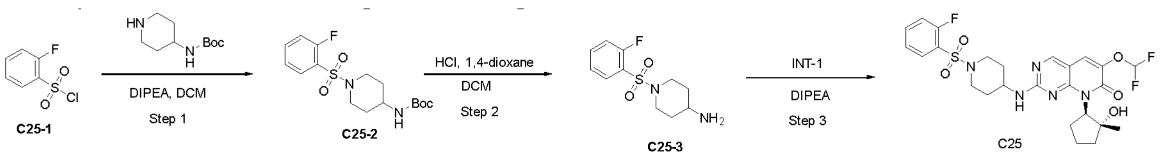


Synthesis of C25-2

The compound 4-tert-butoxycarbonyl-aminopiperidine (1g, 1.00 eq), C21-1 (1.02g, 1.00 eq), and dichloromethane (20 mL) were added to a 50 mL single-necked flask, and triethylamine (1.04g, 1.00 eq) was added dropwise at 0°C, then the mixture was warmed up to room temperature and reacted for 3 h.The reaction was monitored to completion by TLC, then water was added to the reaction solution, and the resulting mixture was extracted three times with dichloromethane, the organic phase was dried with anhydrous sodium sulfate, filtered, concentrated, and the concentrated product was slurried with petroleum ether/ethyl acetate (10/1), and the slurry was filtered to obtain 2.03g.

Synthesis of C25-3

C25-2 (2.03g, 1.0eq) and dichloromethane (20 ml) were added to a 100 mL single-necked flask, and then HCl/dioxane (15 ml, 3 eq) was added slowly. The reaction was carried out for 2h at room temperature. The reaction was monitored to completion by TLC, then the resulting mixture was concentrated, the concentrated product was slurried with petroleum ether, and the slurry was filtered to obtain 1.64g.

Synthesis of C25

Int3 (100mg, 1 eq), C25-3 (80mg, 2 eq), diisopropylethylamine (66mg, 2 eq), and dimethyl sulfoxide (5mL) were added to a 50 mL single-necked flask. The reaction was carried out at 60 °C for 16h. The reaction was monitored to completion by LC-MS, water was added to the reaction solution, and the resulting mixture was extracted three times with ethyl acetate, dried with anhydrous sodium sulfate, filtered, concentrated, purified with Prep-TLC and 30mg of product was obtained. $^1$H NMR (400 MHz, $CDCl_3$) δ 8.42 (s, 1H), 7.87 (td, *J* = 7.4, 1.8 Hz, 1H), 7.66 - 7.56 (m, 1H), 7.39-7.29 (m, 2H), 7.26-7.21 (m, 1H), 6.76 (t, *J* = 75.1 Hz, 1H), 5.78 (t, *J* = 8.5 Hz, 1H), 5.43 (s, 1H), 3.88 (d, *J* = 11.7 Hz, 3H), 2.79 (d, *J* = 57.9 Hz, 3H), 2.26 (td, *J* = 12.0, 11.5, 7.0 Hz, 1H), 2.15 (d, *J* = 12.9 Hz, 2H), 2.01 (q, *J* = 10.1, 8.9 Hz, 3H), 1.89 (q, *J* = 8.3, 7.6 Hz, 1H), 1.80 (d, *J* = 11.1 Hz, 1H), 1.26 (s, 1H), 1.14 (s, 3H), 0.93- 0.81 (m, 1H). MS[M+1]:568

## Example 26

The compound synthesized in the present invention:

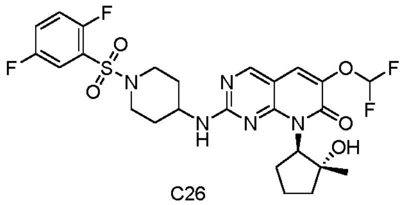

C26

The synthetic route and experimental procedure are as follows:

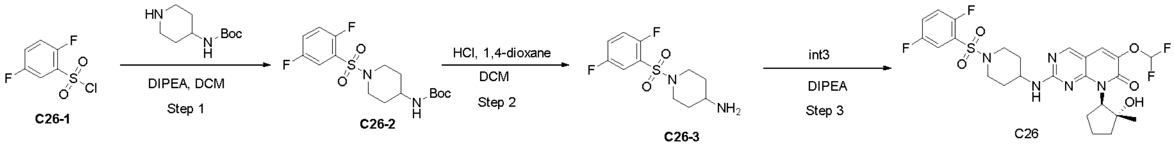


Synthesis of C26-2

The compound 4-tert-butoxycarbonyl-aminopiperidine (0.94g, 1.00 eq), C22-1 (1g, 1.00 eq), and dichloromethane (20 mL) were added to a 50 mL single-necked flask, and triethylamine (0.95g, 1.00 eq) was added dropwise at 0°C, then the mixture was warmed up to room temperature and reacted for 3 h. The reaction was monitored to completion by TLC, then water was added to the reaction solution, and the resulting mixture was extracted three times with dichloromethane, the organic phase was dried with anhydrous sodium sulfate, filtered, concentrated, and the concentrated product was slurried with petroleum ether/ethyl acetate (10/1), and the slurry was filtered to obtain 1.64g.

Synthesis of C26-3

C26-2 (1.64g, 1.0eq) and dichloromethane (20 ml) were added to a 100 mL single-necked flask, and then HCl/dioxane (15 ml, 3 eq) was added slowly. The reaction was carried out for 2h at room temperature. The reaction was monitored to completion by TLC, then the resulting mixture was concentrated, the concentrated product was slurried with petroleum ether, and the slurry was filtered to obtain 1.4g.

Synthesis of C26

Int3 (100mg, 1 eq), C26-3 (85mg, 2 eq), diisopropylethylamine (66mg, 2 eq), and dimethyl sulfoxide (5mL) were added to a 50 mL single-necked flask. The reaction was carried out at 60 °C for 16h. The reaction was monitored to completion by LC-MS, water was added to the reaction solution and the resulting mixture was extracted three times with ethyl acetate, dried with anhydrous sodium sulfate, filtered, concentrated, purified with by Prep-TLC, 35mg of product was obtained. $^1$H NMR (400 MHz, $CDCl_3$) $\delta$ 8.42 (s, 1H), 7.65 - 7.53 (m, 1H), 7.35 (s, 1H), 7.32 - 7.28 (m, 1H), 7.22 (td, $J$ = 9.0, 4.0 Hz, 1H), 6.76 (t, $J$ = 75.1 Hz, 1H), 5.79 (t, $J$ = 8.5 Hz, 1H), 5.46 (s, 1H), 4.06 - 3.78 (m, 2H), 2.90 (s, 2H), 2.72 (s, 1H), 2.27 (td, $J$ = 11.5, 7.0 Hz, 1H), 2.17 (d, $J$ = 12.8 Hz, 2H), 2.02 (td, $J$ = 10.7, 10.1, 5.8 Hz, 3H), 1.97 - 1.75 (m, 3H), 1.27 (q, $J$ = 8.0, 7.5 Hz, 1H), 1.14 (s, 3H), 0.86 (q, $J$ = 7.4, 5.9 Hz, 1H). MS[M+1]:586

## Example 27

The compound synthesized in the present invention:

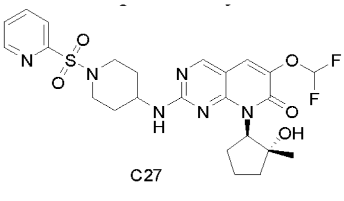

C27

The synthetic route and experimental procedure are as follows:

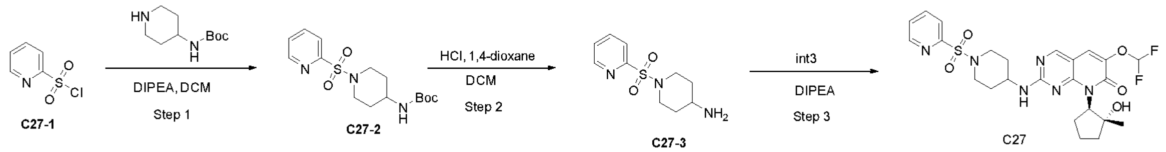


Synthesis of C27-2

The compound 4-tert-butoxycarbonyl-aminopiperidine (0.56g, 1.00 eq), C23-1 (0.52g, 1.00 eq), and dichloromethane (10 mL), and triethylamine (0.57g, 1.00 eq) was added dropwise at 0°C, then the mixture was warmed up to room temperature and reacted for 3 h. The reaction was monitored to completion by TLC, then water was added to the reaction solution, and the resulting mixture was extracted three times with dichloromethane, the organic phase was dried with anhydrous sodium sulfate, filtered, concentrated, and the concentrated product was slurried with petroleum ether/ethyl acetate (10/1), and the slurry was filtered to obtain 0.86 g.

Synthesis of C27-3

C27-2 (0.86g, 1.0eq) and dichloromethane (20 ml) were added to a 100 mL single-necked flask, and then HCl/dioxane (15 ml, 3 eq) was added slowly. The reaction was carried out for 2h at room temperature. The reaction was monitored to completion by TLC, then the resulting mixture was concentrated, the concentrated product was slurried with petroleum ether, and the slurry was filtered to obtain 0.42g.

Synthesis of C27

Int3 (50mg, 1 eq), C27-3 (62mg, 2 eq), diisopropylethylamine (33mg, 2 eq), and dimethyl sulfoxide (5mL) were added to a 50 mL single-necked flask. The reaction was carried out at 60 °C for 16h. The reaction was monitored to completion by LC-MS, water was added to the reaction solution, and the resulting mixture was extracted three times with ethyl acetate, dried with anhydrous sodium sulfate, filtered, concentrated, purified with Prep-TLC, and 18mg of product was obtained. $^1$H NMR (400 MHz, $CDCl_3$) $\delta$ 8.74 (d, $J$ = 4.8 Hz, 1H), 8.42 (s, 1H), 8.02 - 7.89 (m, 2H), 7.57 - 7.49 (m, 1H), 7.35 (d, $J$ = 10.2 Hz, 1H), 6.76 (t, $J$ = 75.1 Hz, 2H), 5.78 (t, $J$ = 8.6 Hz, 1H), 5.44 (s, 1H), 3.96 (d, $J$ = 12.7 Hz, 3H), 3.14 - 2.98 (m, 2H), 2.73 (s, 1H), 2.29 - 2.21 (m, 1H), 2.14 (d, $J$ = 11.6 Hz, 2H), 2.01 (p, $J$ = 8.8 Hz, 3H), 1.95 - 1.87 (m, 1H), 1.82 (s, 1H), 1.17 (d, $J$ = 2.9 Hz, 1H), 1.14 (s, 3H). MS[M+1]:551

**Example 28**

The compound synthesized in the present invention:

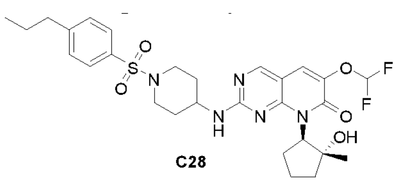

C28

The synthetic route and experimental procedure are as follows:

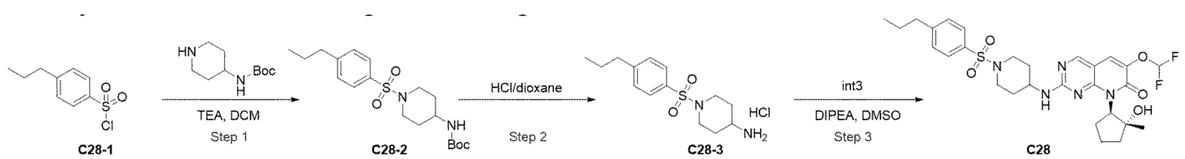


Synthesis of C28-2

The compound 4-tert-butoxycarbonyl-aminopiperidine (1g, 1.00 eq), C24-1 (1.15g, 1.05 eq), and dichloromethane (10 mL) were added to a 50 mL single-necked flask, and triethylamine (1.01g, 2.00 eq) was added dropwise at 0°C, then the mixture was warmed up to room temperature and reacted for 3 h. The reaction was monitored to completion by TLC, then water was added to the reaction solution, and the resulting mixture was extracted three times with dichloromethane, the organic phase was dried with anhydrous sodium sulfate, filtered, concentrated, and the concentrated product was slurried with petroleum ether/ethyl acetate (10/1), and the slurry was filtered to obtain 1.8 g.

Synthesis of C28-3

C28-2 (1.8 g, 1.0eq) and dichloromethane (20 ml) were added to a 100 mL single-necked flask, and then HCl/dioxane (15 ml, 3 eq) was added slowly. The reaction was carried out for 2h at room temperature. The reaction was monitored to completion by TLC, then the resulting mixture was concentrated, the concentrated product was slurried with petroleum ether, and the slurry was filtered to obtain 1.2 g.

Synthesis of C28

Int3 (100 mg, 1 eq), C28-3 (164mg, 2 eq), diisopropylethylamine (132mg, 4 eq), and dimethyl sulfoxide (3mL) were added to a 50 mL single-necked flask. The reaction was carried out at 60 °C for 16h. The reaction was monitored to completion by LC-MS, water was added to the reaction solution, and the resulting mixture was extracted three times with ethyl acetate, dried with anhydrous sodium sulfate, filtered, concentrated, purified with Prep-TLC, and 16 mg of product was obtained. $^1$H NMR (400 MHz, $CDCl_3$) δ 8.40 (s, 1H), 7.68 (d, *J* = 8.1 Hz, 2H), 7.37 -7.33 (m, 2H), 6.75 (s, 1H), 5.76 (t, *J*= 8.6 Hz, 1H), 5.46 (s, 1H), 3.80 (d, *J*= 38.1 Hz, 3H), 2.68 (t, *J*= 7.7 Hz, 3H), 2.52 (s, 1H), 2.29 -2.19 (m, 1H), 2.13 (d, *J*= 12.6 Hz, 2H), 2.08-1.94 (m, 3H), 1.92 -1.76 (m, 2H), 1.70 (dt, *J*= 15.0, 7.4 Hz, 4H), 1.26 (q, *J*= 5.1 Hz, 2H), 1.13 (s, 3H), 0.97 (t, *J* = 7.3 Hz, 3H), 0.92 - 0.82 (m, 2H). MS[M+1]:592

**Example 29**

The compound synthesized in the present invention:

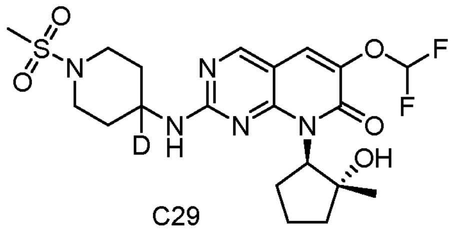

C29

The synthetic route and experimental procedure are as follows:

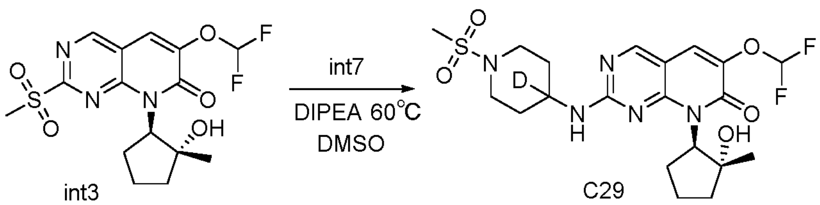


int3 C29

Compound int3 (80 mg) was dissolved in DMSO, int7 (89mg, 2.0 eq) and triethylamine (0.11 ml, 3.0 eq) were added, and the reaction was carried out at 60°C overnight. The reaction was monitored to completion, water was added to the system and the resulting mixture was extracted with ethyl acetate, the organic phases were combined, dried with anhydrous sodium sulfate, concentrated under reduced pressure and the product (50 mg) was obtained by preparative plate separation. $^1$H NMR (400 MHz, $CDCl_3$) δ 8.45 (s, 1H), 7.36 (s, 1H), 6.77 (t, *J* = 75.1 Hz, 1H), 5.82 (s, 1H), 5.57 (d, *J* = 55.6 Hz, 1H), 3.89 - 3.73 (m, 2H), 2.91 (m, *J* = 11.8, 2.8 Hz, 2H), 2.83 (s, 3H), 2.74 (s, 1H), 2.34 - 2.11 (m, 4H), 2.10 - 1.98 (m, 2H), 1.99 - 1.78 (m, 2H), 1.78 - 1.58 (m, 2H), 1.16 (s, 3H). MS[M+1]:488.5.

The compounds in the following table were synthesized using synthetic methods similar to those described above:

| Example | Structure | 1H NMR | MS [M+1]+ |
|---|---|---|---|
| Example 30 | 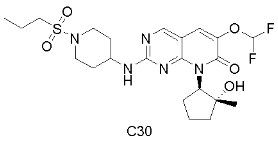 C30 | 1H NMR (400 MHz, $CDCl_3$) δ 8.51 (s, 1H), 7.38 (s, 1H), 6.78 (t, J = 74.0 Hz, 1H), 5.80 (t, J = 8.6 Hz, 1H), 3.71 (s, 2H), 3.15 (d, J = 12.3 Hz, 1H), 2.96 - 2.84 (m, 2H), 2.61 (s, 1H), 2.34 - 1.98 (m, 6H), 1.83 - 1.67 (m, 2H), 1.25 (s, 4H), 1.15 (s, 3H), 1.06 (t, J = 7.4 Hz, 4H), 0.91 - 0.81 (m, 2H). | 516.6 |
| Example 31 | 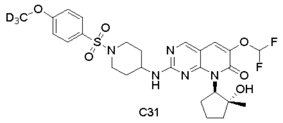 C31 | 1H NMR (400 MHz, $CDCl_3$) δ 8.40 (s, 1H), 7.76 - 7.68 (m, 2H), 7.33 (s, 1H), 7.07 - 6.98 (m, 2H), 6.75 (t, *J* = 75.1 Hz, 1H), 5.76 (t, *J* = 8.5 Hz, 1H), 5.42 (d, *J* = 92.4 Hz, 1H), 3.78 (d, *J* = 54.2 Hz, 3H), 2.61 (d, *J* = 70.4 Hz, 3H), 2.19 (m, *J* = 46.7, 10.8 Hz, 3H), 2.07 - 1.83 (m, 4H), 1.83 - 1.55 (m, 3H), 1.13 (s, 3H) | 582.6 |
| Example 32 | 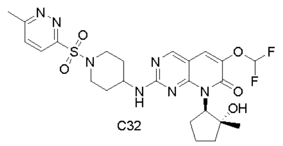 C32 | 1H NMR (400 MHz,$CDCl_3$) δ 8.44 (s, 1H), 7.95 (d, *J* = 8.6 Hz, 1H), 7.56 (d, *J* = 8.6 Hz, 1H), 7.36 (s, 1H), 6.76 (t, *J* = 75.0 Hz, 1H), 5.81 (s, 1H), 5.52 (d, *J* = 85.6 Hz, 1H), 4.07 (d, *J* = 39.2 Hz, 3H), 3.25 (d, *J* = 12.2 Hz, 2H), 2.85 (s, 3H), 2.72 (p, *J* = 8.4, 8.0 Hz, 1H), 2.40 - 2.11 (m, 2H), 2.10 - 1.87 (m,2H), 1.70 (d, *J* = 23.8 Hz, 6H), 1.14 (s, 3H). | 589.6 |
| Example 33 | 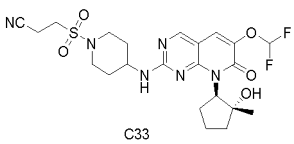 C33 | 1H NMR (400 MHz,$CDCl_3$) δ 8.46 (s, 1H), 7.36 (d, J = 3.9 Hz, 1H), 6.77 (t, J = 75.1 Hz, 1H), 5.83 (s, 1H), 5.46 (s, 1H), 3.97 (s, 5H), 4.72-4.63 (m, 2H), 2.61-2.54(m, 2H), 2.76 (d, J = 26.3 Hz, 1H), 2.34-2.19 (m, 3H), 2.11-1.87 (m, 4H), 1.85-1.64 (m, 3H), 1.16 (s, 3H). | 526.5 |
| Example 34 | 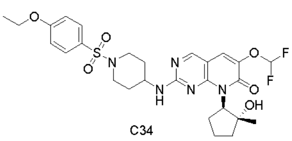 C34 | 1H NMR (400 MHz,$CDCl_3$) δ 8.40 (s, 1H), 7.74 - 7.65 (m, 2H), 7.33 (s, 1H), 7.07 - 6.97 (m, 2H), 6.75 (t, J = 75.1 Hz, 1H), 5.76 (t, J = 8.5 Hz, 1H), 5.37 (d, J = 21.8 Hz, 1H), 4.11 (q, J = 7.0 Hz, 2H), 3.77 (d, J = 53.0 Hz, 3H), 2.61 (d, J = 72.6 Hz, 3H), 2.39 - 1.66 (m, 10H), 1.46 (t, J = 7.0 Hz, 3H), 1.13 (s, 3H) | 593.6 |
| Example 35 | 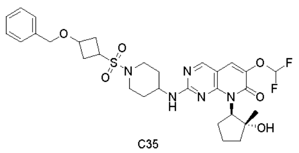 C35 | / | 633.7 |
| Example 36 | 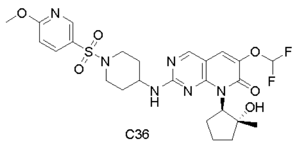 C36 | / | 580.6 |
| Example 37 | 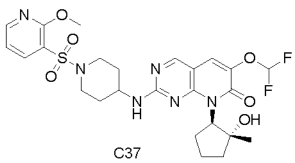 C37 | 1H NMR (400 MHz,$CDCl_3$) δ 8.43 (s, 1H), 8.34 (dd, *J* = 5.0, 1.9 Hz, 1H), 8.18 (dd, *J* = 7.6, 1.9 Hz, 1H), 7.35 (s, 1H), 7.02 (dd, *J* = 7.6, 5.0 Hz, 1H), 6.76 (t, *J* = 75.1 Hz, 1H), 5.79 (t, *J* = 8.6 Hz, 1H), 4.08 (s, 3H), 3.91 (d, *J* = 13.0 Hz, 3H), 2.85 (d, *J* = 88.8 Hz, 3H), 2.39 - 1.68 (m, 11H), 1.15 (s, 3H). | 580.6 |
| Example 38 | 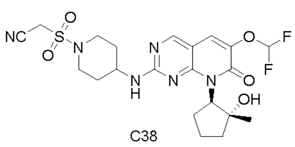 C38 | 1H NMR (400 MHz,$CDCl_3$) δ 8.46 (s, 1H), 7.36 (d, J = 3.9 Hz, 1H), 6.77 (t, J = 75.1 Hz, 1H), 5.83 (s, 1H), 5.46 (s, 1H), 3.97 (s, 5H), 3.36 - 3.16 (m, 2H), 2.76 (d, J = 26.3 Hz, 1H), 2.34 - 2.19 (m, 3H), 2.11 - 1.87 (m, 4H), 1.85 - 1.64 (m, 3H), 1.16 (s, 3H). | 513.5 |

(continued)

| Example | Structure | $^1$H NMR | MS [M+1]$^+$ |
|---|---|---|---|
| Example 39 | 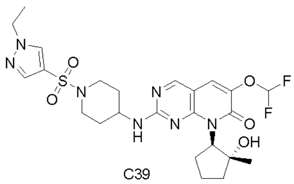 C39 | $^1$H NMR (400 MHz,$CDCl_3$) δ 8.42 (s, 1H), 7.79 (s, 1H), 7.74 (d, J = 0.7 Hz, 1H), 7.34 (s, 1H), 6.76 (t, J = 75.1 Hz, 1H), 5.78 (t, J = 8.5 Hz, 1H), 5.45 (d, J = 84.2 Hz, 1H), 4.25 (q, J = 7.3 Hz, 2H), 3.88 (s, 1H), 3.70 (s, 2H), 2.71 (s, 3H), 2.31 - 2.12 (m, 3H), 2.08 - 1.97 (m, 2H), 1.96 - 1.62 (m, 5H), 1.56 (t, J = 7.3 Hz, 3H), 1.14 (s, 3H). | 568.6 |
| Example 40 | 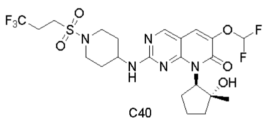 C40 | $^1$H NMR (400 MHz, $CDCl_3$) δ 8.45 (s, 1H), 7.36 (s, 1H), 6.77 (t, *J* = 75.1 Hz, 1H), 5.82 (s, 1H), 5.48 (s, 1H), 4.06 (d, *J* = 28.3 Hz, 1H), 3.85 (td, *J* = 12.5, 5.4 Hz, 2H), 3.17 - 2.98 (m, 4H), 2.72 (d, *J* = 13.1 Hz, 1H), 2.70 - 2.57 (m, 2H), 2.31 - 2.16 (m, 3H), 2.03 (tt, *J* = 11.1, 5.6 Hz, 2H), 1.92 (q, *J* = 8.7, 7.2 Hz, 1H), 1.86 - 1.77 (m, 1H), 1.70 - 1.64 (m, 2H), 1.62 - 1.53 (m, 1H), 1.16 (s, 3H). | 570.5 |
| Example 41 | 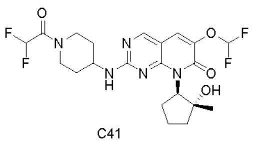 C41 | $^1$H NMR (400 MHz, $CDCl_3$) δ 8.46 (s, 1H), 7.36 (s, 1H), 6.77 (t, *J* = 75.1 Hz, 1H), 6.13 (td, *J* = 53.7, 2.4 Hz, 1H), 5.83 (s, 1H), 5.56 - 5.29 (m, 1H), 4.49 (d, *J* = 14.2 Hz, 1H), 4.14 (d, *J* = 13.9 Hz, 2H), 3.30 (t, *J* = 12.1 Hz, 1H), 3.00 (s, 1H), 2.74 (s, 1H), 2.33 - 2.12 (m, 3H), 2.10 - 1.87 (m, 4H), 1.86 - 1.76 (m, 1H), 1.51 (d, *J* = 11.9 Hz, 2H), 1.17 (s, 3H). | 488.5 |
| Example 42 | 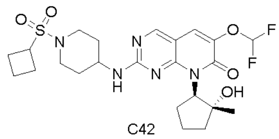 C42 | $^1$H NMR (400 MHz, $CDCl_3$) δ 8.45 (s, 1H), 7.36 (s, 1H), 6.76 (t, *J* = 75.0 Hz, 1H), 5.81 (s, 1H), 5.50 (d, *J* = 32.8 Hz, 1H), 3.96 (s, 1H), 3.89-3.74 (m, 3H), 3.04-2.90 (m, 2H), 2.75 (s, 1H), 2.64-2.50 (m, 2H), 2.35-2.23 (m, 3H), 2.15 (d, *J* = 10.8 Hz, 2H), 2.09-1.99 (m, 4H), 1.99 - 1.87 (m, 1H), 1.87-1.79 (m, 1H), 1.63- 1.54 (m, 1H), 1.32-1.22 (m, 1H), 1.16 (s, 3H), 0.86 (q, *J* = 5.8, 5.2 Hz, 1H). | 528.2 |
| Example 43 | 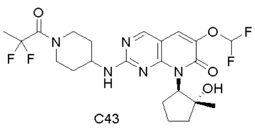 C43 | $^1$H NMR (400 MHz, $CDCl_3$) δ 8.54 (s, 1H), 8.45 (s, 1H), 7.30 (s, 1H), 6.72 (s, 2H), 5.73 (t, *J* = 8.4 Hz, 1H), 4.21 (d, *J* = 46.0 Hz, 3H), 3.45-3.25 (m, 1H), 3.11 (s, 1H), 2.52 (s, 1H), 2.30-2.16 (m, 1H), 2.17-1.93 (m, 5H), 1.89 (s, 1H), 1.83 (s, 1H), 1.78 (s, 2H), 1.73 (s, 3H), 1.20 (d, *J* = 12.0 Hz, 3H), 1.09 (s, 3H). | 502.2 |
| Example 44 | 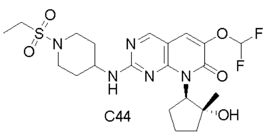 C44 | $^1$H NMR (400 MHz, $CDCl_3$) δ 8.49 (s, 1H), 7.39 (s, 1H), 6.79 (s, 1H), 5.83 (t, *J* = 8.4Hz, 1H), 4.08 (s, 1H), 3.78 (s, 2H), 2.85 (s, 3H), 2.79-2.63 (m, 1H), 2.38- 2.16 (m, 3H), 2.07 (s, 2H), 2.01-1.91 (m, 1H), 1.91 - 1.80 (m, 2H), 1.18 (s, 3H). | 502.2 |
| Example 45 | 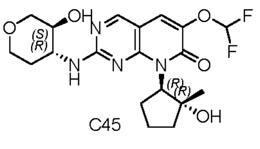 C45 | $^1$H NMR (400 MHz, $CDCl_3$) δ 8.43 (s, 1H), 7.35 (s, 1H), 6.77 (t, *J* = 74.8 Hz, 1H), 5.81 (s, 1H), 5.64 (d, *J* = 6.8 Hz, 1H), 4.08 (dd, *J* = 11.2, 4.8 Hz, 1H), 4.03-3.90 (m, 2H), 3.66 (s, 1H), 3.50 (s, 1H), 3.27 (d, *J* = 21.0 Hz, 1H), 2.66 (d, *J* = 22.8 Hz, 1H), 2.30 (td, *J* = 11.5, 7.1 Hz, 1H), 2.11-2.00 (m, 3H), 1.93 (q, *J* = 9.1 Hz, 1H), 1.87-1.79 (m, 1H), 1.26 (d, *J* = 3.6 Hz, 1H), 1.18 (s, 3H), 0.86 (q, *J* = 7.2, 5.6 Hz, 1H). | 427.2 |

(continued)

| Example | Structure | $^1$H NMR | MS [M+1]$^+$ |
|---|---|---|---|
| Example 46 | 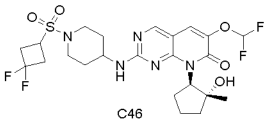 C46 | $^1$H NMR (400 MHz, $CDCl_3$) δ 8.49 (s, 1H), 7.37 (s, 1H), 6.78 (t, J = 74.0 Hz, 1H), 5.80 (t, J = 10.0 Hz, 1H), 4.16 - 4.08 (m, 1H), 3.87 - 3.64 (m, 1H), 3.64 - 3.50 (m, 1H), 3.22 - 3.14 (m, 1H), 3.13 - 2.95 (m, 4H), 2.72 - 2.56 (m, 1H), 2.33 - 2.22 (m, 1H), 2.21 - 2.11 (m, 2H), 2.09 - 2.01 (m, 2H), 1.99 - 1.88 (m, 1H), 1.87 - 1.74 (m, 2H), 1.15 (s, 3H). | 564.2 |
| Example 47 | 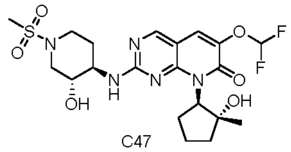 C47 | $^1$H NMR (400 MHz, $CDCl_3$) δ 8.42 (s, 1H), 7.34 (s, 1H), 6.76 (t, J = 76.0 Hz, 1H), 5.87 (s, 1H), 5.82 (t, J = 8.0 Hz, 1H), 4.06 - 3.82 (m, 3H), 3.82 - 3.74 (m, 1H), 2.86 (s, 3H), 2.79 - 2.59 (m, 2H), 2.33 - 2.22 (m, 2H), 2.13 - 1.98 (m, 3H), 1.97 - 1.86 (m, 2H), 1.85 - 1.72 (m, 3H), 1.16 (s, 3H). | 504.2 |
| Example 48 | 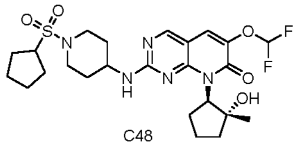 C48 | $^1$H NMR (400 MHz, $CDCl_3$) δ 8.48 (s, 1H), 7.37 (s, 1H), 6.78 (t, J = 74.0 Hz, 1H), 5.81 (t, J = 8.0 Hz, 1H), 4.16 - 4.03 (m, 1H), 3.84 - 3.69 (m, 2H), 3.48 - 3.42 (m, 1H), 3.25 - 3.07 (m, 2H), 2.71 - 2.57 (m, 1H), 2.30 - 2.23 (m, 1H), 2.21 - 2.09 (m, 3H), 2.08 - 1.91 (m, 8H), 1.85 - 1.77 (m, 4H), 1.65 - 1.60 (m, 2H), 1.16 (s, 3H). | 542.2 |
| Example 49 | 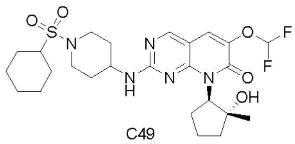 C49 | $^1$H NMR (400 MHz, $CDCl_3$) δ 8.50 (s, 1H), 7.37 (s, 1H), 6.78 (t, J = 74.0 Hz, 1H), 5.81 (t, J = 10.0 Hz, 1H), 4.11 - 3.98 (m, 1H), 3.89 - 3.66 (m, 2H), 3.24 - 3.05 (m, 2H), 2.94 - 2.84 (m, 1H), 2.71 - 2.54 (m, 1H), 2.31 - 2.21 (m, 1H), 2.18 - 2.06 (m, 5H), 1.91 - 1.86 (m, 2H), 1.85 - 1.77 (m, 2H), 1.75 - 1.66 (m, 2H), 1.52 - 1.43 (m, 2H), 1.29 - 1.83 (m, 6H), 1.15 (s, 3H). | 556.2 |
| Example 50 | 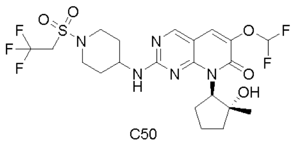 C50 | $^1$H NMR (400 MHz, $CDCl_3$) δ 8.51 (s, 1H), 7.37 (s, 1H), 6.78 (t, J = 74.0 Hz, 1H), 5.81 (t, J = 8.0 Hz, 1H), 4.16 - 4.08 (m, 1H), 3.92 -3.78 (m, 2H), 3.27 - 3.10 (m, 2H), 2.70 - 2.56 (m, 1H), 2.35 - 2.19 (m, 2H), 2.19 - 2.13 (m, 1H), 2.13 - 2.04 (m, 1H), 2.04 - 1.99 (m, 1H), 1.85 - 1.76 (m, 1H), 1.15 (s, 3H). | 556.2 |
| Example 51 | 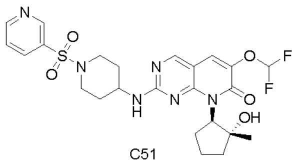 C51 | $^1$H NMR (400 MHz, $CDCl_3$) δ 9.04 - 9.03 (m, 1H), 8.88 (d, *J* = 4.0 Hz, 1H), 8.43 (s, 1H), 8.21 - 8.11 (m, 1H), 7.68 - 7.57 (m, 1H), 7.35 (s, 1H), 6.79 (t, *J* = 74.0 Hz, 1H), 5.75 (t, *J* = 8.0 Hz, 1H), 4.08 - 3.97 (m, 1H), 3.75 - 3.69 (m, 2H), 3.49 - 3.48 (m, 3H), 3.07 - 2.90 (m, 2H), 2.59 - 2.47 (m, 1H), 2.31 - 2.21 (m, 2H), 2.21 - 2.11 (m, 2H), 2.09 -1.97 (m, 2H), 1.96 - 1.86 (m, 2H), 1.85 - 1.71 (m, 2H), 1.13 (s, 3H). | 551.2 |
| Example 52 | 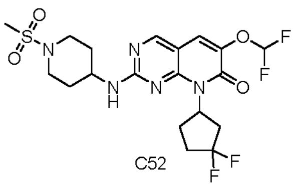 C52 | $^1$H NMR (400 MHz, $CDCl_3$) δ 8.45 (s, 1H), 7.37 (s, 1H), 6.80 (t, *J* = 75.0 Hz, 1H), 6.12 (p, *J* = 8.9 Hz, 1H), 5.47 (d, *J* = 102.5 Hz, 1H), 4.22 - 3.91 (m, 1H), 3.82 (d, *J* = 12.2 Hz, 2H), 3.18 (s, 1H), 2.93 (t, *J* = 11.8 Hz, 2H), 2.83 (d, *J* = 1.2 Hz, 3H), 2.66 (s, 2H), 2.41 - 2.07 (m, 5H), 1.69 (q, *J* = 13.1, 12.4 Hz, 2H). | 494.5 |
| Example 53 | 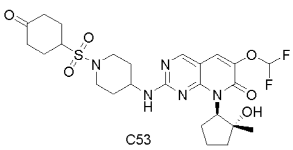 C53 | $^1$H NMR (400 MHz, $CDCl_3$) δ 8.45 (s, 1H), 7.36 (s, 1H), 6.77 (t, *J* = 75.1 Hz, 1H), 5.82 (s, 1H), 5.46 (s, 1H), 4.02 (s, 1H), 3.88 (t, *J* = 12.1 Hz, 2H), 3.41 - 3.27 (m, 1H), 3.23 - 3.04 (m, 2H), 2.75 (s, 1H), 2.66 - 2.51 (m, 2H), 2.49 - 2.24 (m, 5H), 2.23 - 1.77 (m, 9H), 1.64 (m, 2H), 1.16 (s, 3H). | 570.6 |

(continued)

| Example | Structure | $^1$H NMR | MS $[M+1]^+$ |
|---|---|---|---|
| Example 54 | 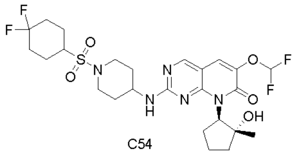 C54 | $^1$H NMR (400 MHz, $CDCl_3$) δ 8.45 (s, 1H), 7.36 (s, 1H), 6.77 (t, *J* = 75.1 Hz, 1H), 5.82 (s, 1H), 5.47 (s, 1H), 4.01 (s, 1H), 3.86 (t, *J* = 11.8 Hz, 2H), 3.20 - 3.04 (m, 2H), 2.98 (t, *J* = 11.0 Hz, 1H), 2.74 (s, 1H), 2.27 (m, 8H), 2.10 - 1.61 (m, 10H), 1.16 (s, 3H). | 592.6 |
| Example 55 | 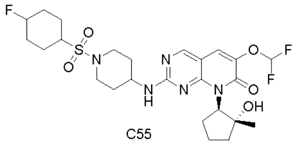 C55 | $^1$H NMR (400 MHz, $CDCl_3$) δ 8.44 (s, 1H), 7.36 (s, 1H), 6.77 (t, *J* = 75.1 Hz, 1H), 5.81 (s, 1H), 5.48 (s, 1H), 4.85 (d, *J* = 47.7 Hz, 1H), 4.01 (s, 1H), 3.87 (t, *J* = 12.0 Hz, 2H), 3.22 - 3.04 (m, 2H), 2.96 (t, *J* = 12.3 Hz, 1H), 2.76 (s, 1H), 2.34 - 2.09 (m, 5H), 2.09 - 1.59 (m, 11H), 1.55 - 1.40 (m, 2H), 1.16 (s, 3H). | 574.6 |
| Example 56 | 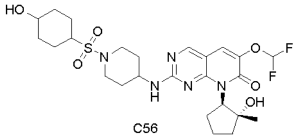 C56 | $^1$H NMR (400 MHz, $CDCl_3$) δ 8.44 (s, 1H), 7.36 (s, 1H), 6.77 (t, *J* = 75.1 Hz, 1H), 5.81 (s, 1H), 5.51 (s, 1H), 3.99 (s, 1H), 3.85 (dd, *J* = 15.5, 7.8 Hz, 2H), 3.64 (dt, *J* = 14.7, 5.3 Hz, 1H), 3.17 - 2.99 (m, 2H), 2.87 (dd, *J* = 16.4, 7.8 Hz, 1H), 2.75 (s, 1H), 2.35 - 2.23 (m, 1H), 2.15 (dd, *J* = 18.1, 9.3 Hz, 6H), 2.09 - 1.77 (m, 5H), 1.67 (d, *J* = 13.5 Hz, 3H), 1.31 (m, 3H), 1.16 (s, 3H). | 572.6 |
| Example 57 | 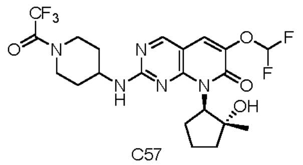 C57 | $^1$H NMR (400 MHz, $CDCl_3$) δ 8.46 (s, 1H), 7.36 (s, 1H), 6.77 (t, *J* = 75.1 Hz, 1H), 5.83 (s, 1H), 5.48 (s, 1H), 4.53 (d, *J* = 14.7 Hz, 1H), 4.34 - 3.88 (m, 2H), 3.33 (t, *J* = 12.2 Hz, 1H), 3.06 (s, 1H), 2.73 (s, 1H), 2.42 - 1.64 (m, 10H), 1.16 (d, *J* = 1.0 Hz, 3H). | 506.4 |
| Example 58 | 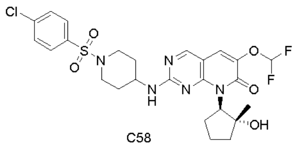 C58 | $^1$H NMR (400 MHz, $CDCl_3$) δ 8.41 (s, 1H), 7.76 - 7.69 (m, 2H), 7.57 - 7.51 (m, 2H), 7.34 (s, 1H), 6.75 (t, *J* = 75.0 Hz, 1H), 5.77 (t, *J* = 8.5 Hz, 1H), 5.41 (d, *J* = 48.9 Hz, 1H), 3.80 (d, *J* = 43.4 Hz, 3H), 2.63 (d, *J* = 56.8 Hz, 3H), 2.34 - 1.84 (m, 8H), 1.78 (s, 2H), 1.14 (s, 3H). | 584.1 |
| Example 59 | 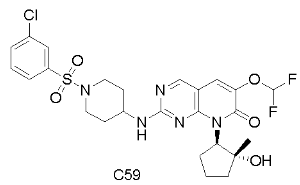 C59 | $^1$H NMR (400 MHz, $CDCl_3$) δ 8.41 (s, 1H), 7.77 (t, *J* = 2.0 Hz, 1H), 7.67 (d, *J* = 7.8 Hz, 1H), 7.61 (dd, *J* = 7.8, 1.7 Hz, 1H), 7.51 (t, *J* = 7.9 Hz, 1H), 7.34 (s, 1H), 6.75 (t, *J* = 75.0 Hz, 1H), 5.77 (t, *J* = 8.5 Hz, 1H), 5.43 (d, *J* = 68.2 Hz, 1H), 3.82 (d, *J* = 43.5 Hz, 3H), 2.70 (s, 3H), 2.34 - 1.72 (m, 10H), 1.14 (s, 3H). | 584.1 |
| Example 60 | 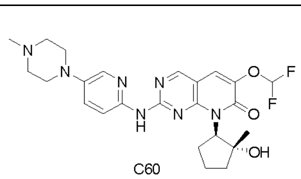 C60 | 1H NMR (400 MHz, CD3OD) δ8.74 (s, 1H), 8.19 (d, J = 9.1 Hz, 1H), 8.01 (d, J = 3.0 Hz, 1H), 7.66 (s, 1H), 7.49 (dd, J = 9.1, 3.0 Hz, 1H), 6.92 (s, 1H), 6.11 -5.95 (m, 1H), 4.59 (s, 1H), 3.27 - 3.15 (m, 4H), 2.71-2.54 (m, 5H), 2.37 (s, 3H), 2.34-2.23 (m, 1H), 2.15 -1.91 (m, 3H), 1.81 (d, J = 12.6 Hz, 1H), 1.37 (d, J = 66.2 Hz, 1H), 1.14 (s, 3H). | 502.2 |
| Example 61 | 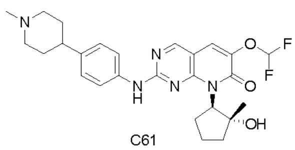 C61 | / | 500.3 |

(continued)

| Example | Structure | $^1$H NMR | MS [M+1]$^+$ |
|---|---|---|---|
| Example 62 | 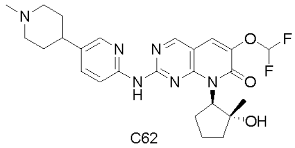 C62 | / | 501.3 |
| Example 63 | 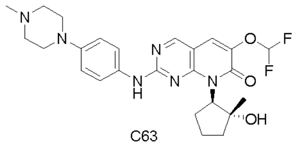 C63 | / | 501.2 |
| Example 64 | 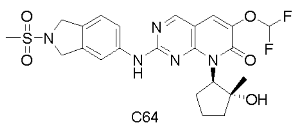 C64 | $^1$H NMR (400 MHz, CDCl3) δ 8.57 (s, 1H), 7.97 (s, 1H), 7.59 (s, 1H), 7.41 (s, 1H), 7.32 (d, *J* = 8.4 Hz, 1H), 7.24 (d, *J* = 7.7 Hz, 1H), 6.80 (t, *J* = 74.9 Hz, 1H), 5.93 (t, *J* = 8.4 Hz, 1H), 4.83 - 4.62 (m, 4H), 2.89 (s, 3H), 2.63 (d, *J* = 11.0 Hz, 1H), 2.36 (q, *J* = 5.0 Hz, 1H), 2.09 - 2.01 (m, 2H), 1.91 (d, *J* = 30.4 Hz, 2H), 1.82 - 1.77 (m, 1H), 1.20 (s, 3H). | 522.2 |
| Example 65 | 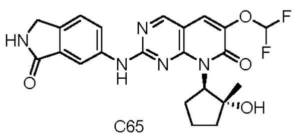 C65 | $^1$H NMR (400 MHz, DMSO-$d_6$) δ 10.30 (s, 1H), 8.86 (s, 1H), 8.63 - 8.52 (m, 1H), 8.15 (d, *J* = 27.5 Hz, 2H), 7.80 (s, 1H), 7.51 (d, *J* = 8.3 Hz, 1H), 7.04 (d, *J* = 74.3 Hz, 1H), 6.00 (t, *J* = 8.5 Hz, 1H), 4.71 (s, 1H), 4.35 (s, 2H), 2.16 (d, *J* = 10.4 Hz, 2H), 1.98 (d, *J* = 8.1 Hz, 2H), 1.88 (s, 2H), 1.06 (s, 3H). | 458.2 |
| Example 66 | 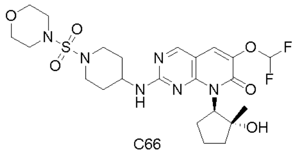 C66 | $^1$H NMR (400 MHz, $CDCl_3$) δ 8.45 (s, 1H), 7.36 (s, 1H), 6.77 (t, *J* = 75.1 Hz, 1H), 5.81 (s, 1H), 5.46 (s, 1H), 4.00 (s, 1H), 3.79 - 3.60 (m, 6H), 3.30 - 3.16 (m, 4H), 3.05 (t, *J* = 11.5 Hz, 2H), 2.75 (s, 1H), 2.28 (ddd, *J* = 12.4, 10.7, 7.0 Hz, 1H), 2.14 (d, *J* = 12.3 Hz, 2H), 2.10 - 1.97 (m, 3H), 1.92 (d, *J* = 10.4 Hz, 1H), 1.82 (dd, *J* = 12.3, 6.8 Hz, 1H), 1.64 (d, *J* = 4.3 Hz, 2H), 1.16 (s, 3H) | 559.2 |
| Example 67 | 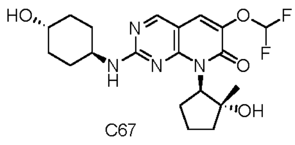 C67 | $^1$H NMR (400 MHz, CDCl3) δ 8.43 (s, 1H), 7.35 (s, 1H), 6.77 (t, J = 75.2 Hz, 1H), 5.78 (s, 1H), 5.35 (d, J = 37.4 Hz, 1H), 3.83 (s, 1H), 3.77 - 3.62 (m, 1H), 2.88 (d, J = 16.3 Hz, 1H), 2.27 - 2.11 (m, 3H), 2.03 (dt, J = 11.4, 8.4 Hz, 4H), 1.91 (d, J = 24.0 Hz, 2H), 1.54 - 1.44 (m, 3H), 1.32 (dd, J = 28.3, 15.2 Hz, 3H), 1.17 (s, 3H). | 425.2 |
| Example 68 | 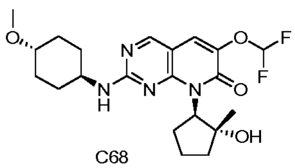 C68 | 1H NMR (400 MHz, $CDCl_3$) δ 8.43 (s, 1H), 7.35 (s, 1H), 6.76 (t, J = 75.3 Hz, 1H), 5.78 (t, J = 8.6 Hz, 1H), 5.43 (s, 1H), 3.85 (s, 1H), 3.37 (s, 3H), 3.22 (d, J = 10.8 Hz, 1H), 2.82 (d, J = 52.0 Hz, 1H), 2.26 - 2.07 (m, 5H), 2.06 - 1.97 (m, 2H), 1.89 (dd, J = 25.6, 11.1 Hz, 2H), 1.51 - 1.20 (m, 5H), 1.17 (s, 3H). | 439.2 |
| Example 69 | 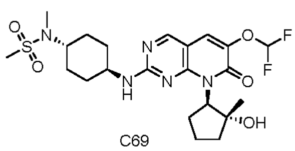 C69 | $^1$H NMR (400 MHz, CDCl3) δ 8.44 (s, 1H), 7.35 (s, 1H), 6.76 (t, *J* = 75.1 Hz, 1H), 5.79 (s, 1H), 5.42 (d, *J* = 46.2 Hz, 1H), 4.02 - 3.60 (m, 2H), 2.86 (s, 3H), 2.82 (s, 3H), 2.80 (s, 1H), 2.26 (m, *J* = 12.3, 10.3, 6.9 Hz, 3H), 2.04 (d, *J* = 6.3 Hz, 2H), 1.98 - 1.80 (m, 4H), 1.80 - 1.62 (m, 3H), 1.55 - 1.35 (m, 2H), 1.17 (s, 3H). | 516.6 |

(continued)

| Example | Structure | $^{1}$H NMR | MS $[M+1]^+$ |
|---|---|---|---|
| Example 70 | 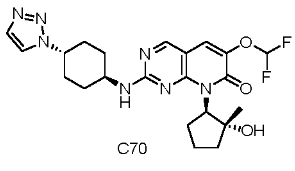 C70 | $^{1}$H NMR (400 MHz, CDCl3) δ 8.47 (d, *J* = 8.7 Hz, 1H), 7.72 (d, *J* = 1.0 Hz, 1H), 7.59 (s, 1H), 7.36 (s, 1H), 6.77 (t, *J* = 75.1 Hz, 1H), 5.83 (s, 1H), 5.46 (s, 1H), 4.57 (m, *J* = 12.1, 8.4, 3.7 Hz, 1H), 4.03 (d, *J* = 50.4 Hz, 1H), 2.78 (s, 1H), 2.47 - 2.19 (m, 6H), 2.13 - 1.79 (m, 6H), 1.60 - 1.45 (m, 2H), 1.17 (s, 3H). | 476.5 |
| Example 71 | 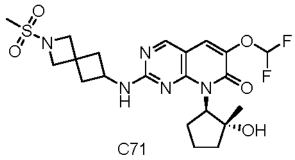 C71 | $^{1}$H NMR (400 MHz, CDCl3) δ 8.44 (s, 1H), 7.35 (s, 1H), 6.77 (t, *J* = 75.1 Hz, 1H), 5.80 (s, 1H), 5.67 (s, 1H), 4.33 (s, 1H), 4.02 (s, 2H), 3.98 - 3.84 (m, 2H), 2.86 (s, 6H), 2.48 - 2.11 (m, 4H), 2.11 - 1.78 (m, 4H), 1.16 (s, 3H). | 500.5 |
| Example 72 | 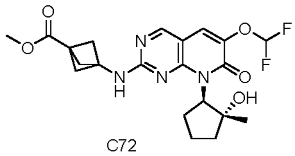 C72 | 1H NMR (400 MHz, CDCl3) δ 8.46 (s, 1H), 7.36 (s, 1H), 6.77 (t, J = 75.1 Hz, 1H), 5.91 (s, 2H), 3.71 (s, 3H), 2.62 (s, 1H), 2.56 - 2.44 (m, 6H), 2.41 - 2.31 (m, 1H), 2.03 (ddd, J = 32.6, 16.5, 6.6 Hz, 4H), 1.78 (dd, J = 12.8, 6.6 Hz, 1H), 1.17 (s, 3H). | 451.4 |
| Example 73 | 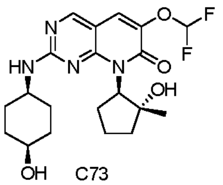 C73 | $^{1}$H NMR (400 MHz, $CDCl_3$) δ 8.43 (s, 1H), 7.35 (s, 1H), 6.76 (t, *J* = 75.2 Hz, 1H), 5.79 (t, *J* = 8.7 Hz, 1H), 5.67 (s, 1H), 3.98 (s, 1H), 3.89 (s, 1H), 2.83 (s, 1H), 2.38 (s, 1H), 2.23 (s, 1H), 2.03 (q, *J* = 8.8 Hz, 2H), 1.93 (t, *J* = 9.2 Hz, 1H), 1.90 - 1.71 (m, 10H), 1.17 (s, 3H). | 425.2 |
| Example 74 | 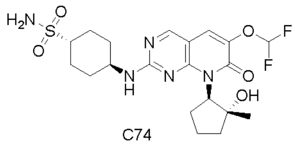 C74 | 1H NMR (400 MHz, DMSO-d6) δ 8.67 (s, 1H), 7.97 (s, 1H), 7.72 (s, 1H), 7.07 (t, J = 74.6 Hz, 1H), 6.68 (s, 2H), 5.77 (s, 1H), 4.44 (s, 1H), 3.97 (s, 1H), 2.88 (s, 1H), 1.93 (d, J = 45.1 Hz, 10H), 1.66 (s, 4H), 1.26 (s, 3H). | 488.2 |
| Example 75 | 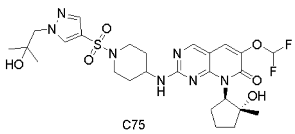 C75 | / | 612.3 |
| Example 76 | 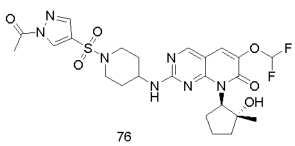 76 | / | 582.2 |
| Example 77 | 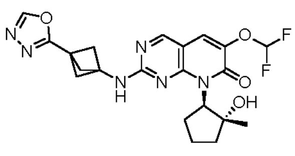 C77 | $^{1}$H NMR (400 MHz, CDCl3) δ 8.48 (s, 1H), 8.36 (s, 1H), 7.37 (s, 1H), 6.78 (t, J = 75.0 Hz, 1H), 6.07 (s, 2H), 2.83 - 2.70 (m, 6H), 2.62 (s, 1H), 2.39 (s, 1H), 2.13 - 2.03 (m, 2H), 1.95 (q, J = 8.5 Hz, 1H), 1.78 (d, J = 12.4 Hz, 2H), 1.18 (s, 3H). | 461.4 |
| Example 78 | 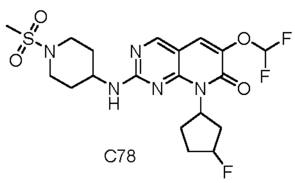 C78 | $^{1}$H NMR (400 MHz, $CDCl_3$) δ 8.42 (s, 1H), 7.35 (s, 1H), 6.82 (t, *J* = 75.4 Hz, 1H), 5.72 (s, 1H), 5.59 - 5.10 (m, 2H), 4.20 (d, *J* = 37.3 Hz, 1H), 3.80 (d, *J*= 10.5 Hz, 2H), 2.92 (t, *J* = 11.6 Hz, 4H), 2.81 (s, 3H), 2.49 - 2.06 (m, 4H), 2.01 - 1.77 (m, 2H), 1.66 (t, *J* = 11.9 Hz, 2H). | 476.5 |

(continued)

| Example | Structure | $^1H$ NMR | MS $[M+1]^+$ |
|---|---|---|---|
| Example 79 | 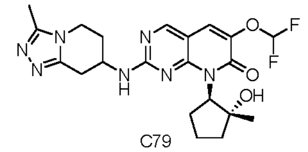 C79 | / | 462.4 |

**Cell proliferation inhibition assay**

1. Experimental Materials

| Cell Lines | Suppliers |
|---|---|
| MCF7 | Shanghai Cell Bank, Chinese Academy of Sciences |
| T47D | Shanghai Cell Bank, Chinese Academy of Sciences |
| OVCAR3 | BeNa Culture Collection |
| HCC1806 | BeNa Culture Collection |

Control compounds PF-06873600 in WO2018033815A1, Palbociclib

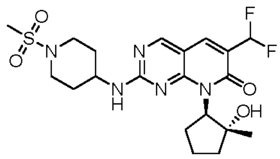

PF-06873600

2. Experimental protocol

MCF7, T47D, OVCAR3 and HCC1806 cells were inoculated in 96-well plates at 3000, 3000, 2500, and 2000 cells/well, respectively, and cultured overnight at 37°C, 5% $CO_2$ incubator.

1. Dilution of compounds

a) The gradient dilution solutions of the tested compounds were prepared: 10mM of Palbociclib and the example compounds were used as stock solution. Then 2.5 μl of the stock solution was dissolved in 497.5 μl of DMSO-free medium, and then a 3-fold serial gradient dilution was performed with 0.1% DMSO medium for a total of 9 concentrations, and the concentrations of the compounds after dilution were as follows:
10000 nM, 3333.33 nM, 1111.11 nM, 370.34 nM, 123.45 nM, 41.15 nM, 13.72 nM, 4.57 nM, and 1.52 nM.
b) After fully mixing, 20 μL of compound solutions were added into cell culture plates containing 80 μL of cells respectively, with 4 replicate wells for each concentration.
c) The cells were transferred to an incubator and incubated for 7 days, and exchanged with fresh culture medium containing the same concentration of compounds when incubated to day 4.

2. MTT assay

a) The cell culture plate was taken out and 10 μL of 5 mg/ml MTT was added to the hood.
b) The cell culture plate was put back into the incubator and continued to incubate for 3 hours.
c) The cell culture plate was taken out to remove the culture medium, and 100 μL of isopropanol (0.4 mMHCl, 0.4% NP40) was added to the plate, and the plate was shaken on a shaker at room temperature for 30 minutes

and read at 570 nm.

3. Data analysis

The survival rate ( % Cell Survival was calculated using the following equation:

$$\% \text{ Cell Survival}=100\%\times(\text{OD_Sample- OD_LCave })/(\text{OD_HC}-\text{OD_LCave })$$

OD_HC: 0.1‰ cell readings of DMSO control
OD_Sample: cell readings of compound addition
OD_LC: readings of blank medium
Analyed by Prizm: Dose-response-Inhibition - Log(inhibitor) vs response (three parameters for the best fit)
IC50 (nM) values were calculated by concentration-response curve fitting.

The results of the cell proliferation inhibition assay are shown in Table 1 below.

Table 1

| Cell lines Examples | MCF7 (IC50 nM) | T47D (IC50 nM) | OVCAR3 (IC50 nM) | HCC1086 (IC50 nM) |
|---|---|---|---|---|
| Palbociclib | A | B | D | D |
| PF-06873600 | B | A | B | B |
| Example 3 | B | B | A | B |
| Example 5 | B | A | B | B |
| Example 6 | A | A | A | A |
| Example 7 | B | A | B | B |
| Example 8 | B | A | A | B |
| Example 9 | B | B | A | B |
| Example 10 | B | A | A | B |
| Example 11 | A | A | A | A |
| Example 12 | B | A | A | A |
| Example 14 | B | A | B | B |
| Example 15 | B | B | B | B |
| Example 16 | A | A | A | A |
| Example 17 | A | A | A | A |
| Example 18 | B | A | A | A |
| Example 20 | C | B | B | C |
| Example 21 | C | B | B | C |
| Example 22 | B | A | A | B |
| Example 23 | B | B | B | C |
| Example 24 | A | A | A | A |
| Example 25 | B | A | A | B |
| Example 26 | B | A | B | B |
| Example 27 | A | A | A | A |
| Example 28 | B | B | B | C |
| Example 29 | A | B | A | A |
| Example 30 | B | B | B | B |

(continued)

| Cell lines Examples | MCF7 (IC50 nM) | T47D (IC50 nM) | OVCAR3 (IC50 nM) | HCC1086 (IC50 nM) |
|---|---|---|---|---|
| Example 31 | A | A | A | A |
| Example 32 | A | / | / | A |
| Example 33 | B | B | B | C |
| Example 34 | A | A | A | A |
| Example 36 | A | A | A | A |
| Example 37 | / | A | A | A |
| Example 38 | A | / | / | B |
| Example 39 | A | / | / | A |
| Example 40 | / | B | A | B |
| Example 42 | A | A | A | B |
| Example 44 | A | A | A | B |
| Example 46 | A | A | A | B |
| Example 48 | B | B | A | B |
| Example 49 | / | B | B | C |
| Example 51 | A | A | A | B |
| Example 53 | C | B | A | C |
| Example 54 | / | B | A | C |
| Example 55 | A | A | B | B |
| Example 56 | B | B | C | C |
| Example 57 | / | C | C | C |
| Example 58 | B | B | B | C |
| Example 59 | B | B | B | B |
| Example 60 | A | A | A | A |
| Example 63 | A | B | A | B |
| Example 64 | B | A | A | B |
| Example 66 | / | B | A | B |
| Example 75 | A | A | A | B |

A represents IC50 value ≤50nM, B represents IC50 value >50nM and ≤200nM, C represents IC50 value >200nM and <1000nM, D represents IC50 value >1000nM.

From the above table, it can be seen that the compounds of this patent have good inhibitory effects on both pabocinib-sensitive and drug-resistant cells, and the compounds of this patent have comparable or even better activities compared with PF-06873600, and thus are expected to be further developed into drugs for regulating CDK kinase activity or treating CDK-related diseases.

**MCF-7-Palbo-R cell antiproliferative assay**

1. Experimental Materials

MCF-7/palbo-R

EMEM medium (ATCC, 30-2003); DMEM/F12 medium (Gibco-11330-032)
Fetal bovine serum (cellmax-SA211.02)
DMSO (SIGMA, D2650)
BrdU ELISA cell proliferation assay kit (Roche, 11647229001)
ELISA (e.g. MD-SpectraMax ID5 or EnVision)

2. Experimental protocol

1. Cell spreading

1) Cells were counted, the concentration of cell suspension was adjusted
2) The MCF7/Palbo-R cell suspension was seeded in 96-well plates at 90 μl volume to make the number of cells per well is 4000-5000;
3) Blank control group was added with 90 μl of culture medium;

2. Dilution of compounds

a) The gradient dilution solutions of the tested compounds were prepared: 10mM of Palbociclib and the example compounds were used as stock solution. A 3-fold serial gradient dilution was performed with 0.1% DMSO medium for a total of 9 concentrations, and the concentrations of the compounds after dilution were as follows: 10000 nM, 3333.33 nM, 1111.11 nM, 370.34 nM, 123.45 nM, 41.15 nM, 13.72 nM, 4.57 nM, and 1.52 nM.
b) After 72 hours of compound treatment:

1) A 10 mM of Brdu stock solution was diluted with RPMI medium and added to a 96-well plate at 20 uL/well and shaken at 350 rpm for 10 minutes.
2) The 96 well plate was placed back into the incubator for 1 or 2 hours.
3) After the medium was removed from the 96-well plate, 200 ul/well of Fixation/Denaturation solution was added and incubated for 30 minutes at room temperature.
4) The fixation solution was removed and 100 ul of peroxidase-coupled anti-Brdu antibody solution (antibody dilution prepared at 1: 100 dilution) was added,
and incubated at 350 rpm for 1 hour at room temperature.
6) The 96-well plate was washed three times with 300ul/well of PBS.
8) 100ul of peroxidase substrate solution was added and shaken at 350 rpm for about 10-20 minutes at room temperature,
until the DMSO-treated cell control wells turn a medium blue color. If a color is too dark, it will cause the absorbance value to out of the reading range.
9) The reaction was terminated by the addition of 25 ul of 1 M sulfuric acid. Shake to ensure uniform color.
10) The absorbance at 450 nm was recorded.

3. Data analysis

The inhibition rate is calculated using the following equation:

$$IR\ (\%) = (1 - (RLU\ compound - RLU\ blank) / (RLU\ control - RLU\ blank)) * 100\%.$$

The results of the MCF-7-Palbo-R cell antiproliferative assay are shown in Table 2

Table 2

| Cell line | Compounds | AbsIC50(pM) | RelC50(μM) | Bottom(%) | Top(%) |
|---|---|---|---|---|---|
| MCF-7-Palbo-R | PF-06873600 | 0.054 | 0.049 | 7.487 | 98.896 |
| | **Example 3** | 0.036 | 0.038 | 6.317 | 98.857 |
| | **Example 12** | 0.026 | 0.026 | 7.117 | 99.196 |
| | Palbociclib | 4.009 | 7.091 | -3.542 | 68.840 |

The compounds of the present patent have a very good inhibition rate against pabocinib resistant cell lines and are superior to PF-06873600.

**Enzyme activity test**

**Preparation of compounds:**

1. The test compounds and pabocinib were configured into a 0.5nM solution of DMSO;
2. 20nL of stock solution was transferred to a 384-well plate using Echo550. DMSO was used as a blank control.

**Experimental steps:**

1. according to the following table to prepare 1.3X enzyme solution containing enzyme, substrate and cofactor;
2. 15uL of 1.3X enzyme solution was added to each well and the mixture was incubated for 30 min at room temperature;
3. 5uL of ATP solution was added to initiate the reaction. The final volume of each well was 20uL;
4. After incubation for 150 min at room temperature, 75uL of termination buffer solution was added to terminate the experiment;
5. The samples were analyzed using EZ reader.

| enzymes | Supplier | Enzyme concentration (nM) | ATP concentration (nM) | Substrate | Cofactor | Reaction time |
|---|---|---|---|---|---|---|
| CDK2/CycE1 | Carna | 0.63 | 100 | FL-Peptide 18 | 10m $MMgCl_2$ | 120 minutes |
| CDK4/CycD3 | Carna | 1.50 | 200 | FL-Peptide34 | 10m $MMgCl_2$ | 120 minutes |
| CDK6/CycD1 | Carna | 1.50 | 300 | FL-Peptide34 | 10m $MMgCl_2$ | 120 minutes |

**Data analysis**

The % inhibition was calculated using the following equation

The one treated with DMSO is the positive control (PC)

The one without enzyme is the negative control (NC)

$$\% \text{ Inhibition} = 100-100*((CR_{PC}-CR_{sample}) / (CR_{PC}-CR_{NC}))$$

The results of enzyme inhibition experiments are shown in Table 3

Table 3

| Examples | CDK2(nM) | CDK4(nM) | CDK6(nM) |
|---|---|---|---|
| Palbociclib | D | A | A |
| PF-06873600 | A | A | A |
| Example 1 | A | B | B |
| Example 3 | A | A | A |
| Example 5 | A | B | A |
| Example 6 | A | A | A |
| Example 7 | A | A | A |
| Example 8 | A | B | A |
| Example 9 | A | A | A |
| Example 10 | A | A | A |
| Example 11 | A | A | A |
| Example 12 | A | A | A |
| Example 14 | A | B | A |
| Example 15 | A | A | A |
| Example 16 | A | A | A |
| Example 17 | A | A | A |
| Example 18 | A | A | A |
| Example 22 | A | B | A |
| Example 23 | A | B | B |
| Example 24 | A | A | A |
| Example 25 | A | B | A |
| Example 26 | A | B | A |
| Example 27 | A | A | A |
| Example 28 | A | B | A |
| Example 29 | A | A | A |
| Example 31 | A | A | / |
| Example 32 | A | A | A |
| Example 33 | A | / | / |
| Example 36 | A | A | / |
| Example 37 | A | A | / |
| Example 40 | A | A | / |
| Example 42 | A | A | / |
| Example 44 | A | A | / |
| Example 45 | B | B | / |
| Example 46 | A | A | / |
| Example 48 | A | A | / |
| Example 51 | A | A | A |
| Example 54 | A | B | / |

(continued)

| Examples | CDK2(nM) | CDK4(nM) | CDK6(nM) |
|---|---|---|---|
| Example 61 | B | A | A |
| Example 64 | A | B | A |
| Example 66 | A | B | A |
| Example 69 | A | B | B |
| Example 75 | A | B | A |

A represents IC50 value ≤ 10 nM, B represents IC50 value > 10 nM and ≤ 100 nM, C represents IC50 value > 100 nM and ≤ 500 nM, and D represents IC50 value > 500 nM. The data suggests that the compounds of the present patent have superior kinases inhibitory activity against CDK2, CDK4, and CDK6.

**Xenograft tumor experiments:**

**xMCF-7/Palbo-R model:**

To establish a xenograft tumor model, 1 × 107 xMCF-7/Palbo-R cells were suspended in 0.2 mL of a mixture of Matrigel and mouse (1:1) and injected subcutaneously into 6-8 week-old Balb/c nube female mice. Tumor growth was observed periodically, and the volume (volume = 1/2 × (length × width 2)) was measured with a vernier caliper. When the tumors grew to an average volume of 100~150 mm3, the drug was administered to mice (12 days after inoculation) in random groups according to the tumor size and their body weights. Mice were randomly assigned to 5 groups, each containing 3 animals, and the doses used in each group were (a) Vehicle; (b) 10 mg/kg PF-06873600; (c) 20 mg/kg C27; (d) 10 mg/kg C3; and (h) 20 mg/kg C3, respectively. The drugs were administered by oral tube feeding twice daily for 20 days. Tumor volume and animal weight were measured twice a week and continued until the end of the experiment. Dosing day was defined as day 0. Measurement time points were day 0, day 3, day 7, day 10, day 14, day 17 and day 20. All mice were euthanized by cervical dislocation. At the end of the experiment, tumors were collected and weighed to calculate TGI values (see Table 4).The experimental results are shown in Figure 1.

Table 4

| Compounds | Dose (mg/kg) | TGI |
|---|---|---|
| PF-06873600 | 10 | 36.2 |
| C27 | 20 | 61.5 |
| C3 | 10 | 64.4 |
| C3 | 20 | 64.6 |

**OVCAR-3 model:**

1. Cell culture

OVCAR-3 cells were cultured in RPMI1640 culture medium containing 20% fetal bovine serum and 10μg/mL Insalin. Cells in the exponential growth phase were collected, and the cells were resuspended in the mixed solution of PBS and Matrigel in a ratio of 1:1 to a suitable concentration for subcutaneous tumor inoculation in nude mice.

2. Animal modeling

To establish a xenograft tumor model, $1\times10^7$ OVCAR-3 cells were suspended in 0.2 mL of a mixture of PBS and Matrigel (1: 1) and injected subcutaneously into 6-8-week-old BALB/c nube female mice on the right back. Tumor growth was observed periodically, and the volume (volume = 1/2 × (length × width $^2$)) was measured with a vernier caliper. When the tumors grew to an average volume of 100~150 $mm^3$, the drug was administered to mice (27 days after inoculation) in random groups according to the tumor size and their body weights. Mice were randomly assigned to six groups, each containing five animals, and the doses used in each group were (a) Vehicle; (b) 60 mg/kg Palbociclib;

(c) 30 mg/kg PF-06873600; (d) 30 mg/kg C3; (e) 30 mg/kg C29; and (f) 50 mg/kg C29. Vehicle is DMSO/Solutol/Saline (5%/10%/85%), Palbociclib, PF-06873600, C3, and C29 were dissolved in vehicle at concentrations of 6 mg/mL, 3 mg/mL, 3 mg/mL, 3 mg/mL, and 5 mg/mL, respectively. Among them, mice in the Vehicle, 30 mg/kg PF-06873600, 30 mg/kg C3 , 30 mg/kg C29 and 50 mg/kg C29 groups were administered twice daily by oral tube feeding for 21 days, and mice in the 60 mg/kg Palbociclib group were administered once daily by oral tube feeding for 21 days. Tumor volume and animal weight were measured twice a week and continued until the end of the experiment. Dosing day was defined as day 0. Measurement time points were day 0, day 3, day 7, day 10, day 14, day 17 and day 21. All mice were euthanized by cervical dislocation. At the end of the experiment, tumors were collected and weighed to calculate TGI values (see Table 5). The experimental results are shown in Figure 2.

Table 5

| Compound | Dose (mg/kg) | TGI |
|---|---|---|
| Palbociclib | 60 | 27.7 |
| PF-06873600 | 30 | 52.6 |
| C3 | 30 | 71.3 |
| C29 | 30 | 69.0 |
| C29 | 50 | 74.8 |

**MV4-11 model:**

1. Cell culture

MV-4-11 cells were cultured in IMEM culture medium containing 10% fetal bovine serum. Cells in the exponential growth phase were collected, and the cells were resuspended in PBS to a suitable concentration for subcutaneous tumor inoculation in nude mice.

2. Animal modeling

To establish a xenograft tumor model, $5\times10^6$ MV4-11 cells were suspended in 0.1 mL of a 1:1 mixture of PBS and Matrigel and injected subcutaneously into the right dorsal side of 6-8-week-old NOD/SCID mice. Tumor growth was observed periodically, and the volume (volume = 1/2 $\times$ (length $\times$ width $^2$)) was measured with a vernier caliper. When the tumors grew to an average volume of 100~150 $mm^3$, the drug was administered to mice (12 days after inoculation) in random groups according to the tumor size and their body weights. Mice were randomly assigned to 5 groups, each containing 5 animals: (a) Vehicle; (b) 20 mg/kg Palbociclib; (c) 10 mg/kg PF-06873600; (d) 10 mg/kg C3; (e) 20 mg/kg C3. Vehicle is DMSO/Solutol/Saline (5%/10%/85%), Palbociclib, PF-06873600, and TYK-00127 groups were dissolved in Vehicle at concentrations of 2 mg/mL, 1 mg/mL, 1 mg/mL, and 2 mg/mL, respectively. Among them, the mice in the Vehicle, 10 mg/kg TY-3301, 10 mg/kg C3 , and 20 mg/kg C3 groups were administered twice a day by oral tube feeding for 15 days, and the mice in the 20 mg/kg TY-3300 group were administered once a day by oral tube feeding for 15 days. Tumor volume and animal weight were measured twice a week and continued until the end of the experiment. Dosing day was defined as day 0. Measurement time points were day 0, day 3, day 7, day 10, day 14, and day 15. All mice were euthanized by cervical dislocation. At the end of the experiment, tumors were collected and weighed to calculate TGI values (see Table 6). The experimental results are shown in Figure 3.

Table 6

| Compound | Dose (mg/kg) | TGI |
|---|---|---|
| Palbociclib | 20 | 39.0 |
| PF-06873600 | 10 | 60.9 |
| C3 | 10 | 69.5 |
| C3 | 20 | 95.3 |

In vivo pharmacodynamic experiments in animal models have shown that the compounds of the present patent exhibit a good tumor inhibitory effect, which is much better than that of the Pabocinib control group, and likewise shows

a large advantage compared to PF-06873600.

The present invention provides a class of compounds with excellent kinase, cellular, and *in vivo* efficacy and the synthesis methods therefor, and this class of compounds is expected to be developed as a solution to the problem of resistance to Pabocinib and the like in the existing clinic, and to bring a new therapeutic option for such patients.

All documents referred to in the present invention are incorporated by reference herein as if each document is individually incorporated by reference. Further, it should be understood that upon reading the above teaching of the present invention, various changes or modifications may be made to the present invention by those skilled in the art, and those equivalents also fall within the scope limited by the appended claims of the present application.

## Claims

**1.** A compound of formula (I), or a pharmaceutically acceptable salt, a stereoisomer, a solvate or a prodrug thereof,

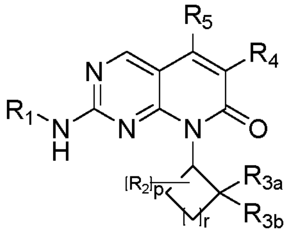
(I)

wherein,

$R_1$ is selected from the group consisting of: unsubstituted or 1-4 $R_6$ substituted C3-C10 cycloalkyl, unsubstituted or 1-4 $R_6$ substituted 5-15 membered fused ring without or containing 1-3 heteroatoms selected from N, O and S, unsubstituted or 1-4 $R_6$ substituted 5-15 membered spiro ring without or containing 1-3 heteroatoms selected from N, O and S, unsubstituted or 1-4 $R_6$ substituted 5-15 membered bridged ring without or containing 1-3 heteroatoms selected from N, O and S, unsubstituted or 1-4 $R_6$ substituted 3-10 membered heterocycloalkyl containing 1-4 heteroatoms selected from N, O and S, unsubstituted or 1-4 $R_6$ substituted C6-C10 aryl, unsubstituted or 1-4 $R_6$ substituted 3-10 membered heteroaryl containing 1-5 heteroatoms selected from N, O and S, and unsubstituted or 1-4 $R_6$ substituted C1-C6 alkyl;

$R_2$ is selected from the group consisting of: H, F, OH, C1-C4 alkyl, C1-C4 haloalkyl, C1-C4 alkoxy, C1-C4 haloalkoxy, and $R_2$ can be connected with atoms on the ring to form a spiro ring, a bridged ring, or a fused ring structure;

$R_{3a}$ and $R_{3b}$ are independently selected from the group consisting of: H, F, OH, C1-C4 alkyl, C1-C4 haloalkyl, C1-C4 alkoxy, and C1-C4 haloalkoxy;

among them, each of the C1-C4 alkyl and C1-C4 haloalkyl in $R_2$, $R_{3a}$ and $R_{3b}$ is optionally substituted by halogen, OH, C1-C4 alkoxy or C1-C4 haloalkoxy;

$R_4$ is selected from the group consisting of: Br, substituted or unsubstituted 3-10 membered heterocycloalkyl containing 1-4 heteroatoms selected from N, O and S, $-OR_4'$, $-SR_4'$, $-NR_4'R_4''$, wherein $R_4'$ and $R_4''$ are independently selected from the group consisting of: H, $COR_7$, substituted or unsubstituted C1-C6 alkyl, substituted or unsubstituted C3-C6 cycloalkyl, substituted or unsubstituted 3-6 membered heterocycloalkyl containing 1-4 heteroatoms selected from N, O and S; and the "substituted" refers to being substituted by 1-3 substituents selected from the group consisting of: deuterium, halogen, OH, CN, =O, C1-C4 alkyl, C1-C4 alkoxy, and C1-C4 haloalkoxy;

$R_5$ is selected from the group consisting of: H, halogen, C1-C2 alkyl, C1-C2 haloalkyl, C1-C4 alkoxy, and C1-C4 haloalkoxy;

each $R_6$ is independently selected from the group consisting of H, deuterium, hydroxyl, halogen, cyano, =O, $COR_7$, $CO_2R_7$, $CONR_8R_9$, $CO_2NR_8R_9$, $SO_2R_7$, $SO_2NR_8R_9$, $NR_8SO_2R_7$ , $NHSO_2NR_8R_9$,

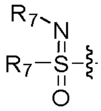
,

$NR_8R_9$, R‴ substituted or unsubstituted 3-10 membered heterocycloalkyl containing 1-3 heteroatoms selected

from N, O and S, C1-C4 alkyl, C1-C4 haloalkyl, C1-C4 alkoxy, C1-C4 haloalkoxy, C3-C8 cycloalkyl, C3-C8 halocycloalkyl, C4-C10 spiro ring, C3-C10 fused ring, C4-C10 bridged ring, C1-C6 thioalkyl, C6-C10 aryl, and 3-10 membered heteroaryl containing 1-5 heteroatoms selected from N, O and S;

R‴ is selected from the group consisting of: =O, NR'R", C1-C4 alkyl, and C1-C4 haloalkyl;

each $R_7$ is independently selected from the group consisting of: C1-C4 alkyl, -L-(C3-C8 cycloalkyl), -L-(3-10 membered heterocycloalkyl containing 1-4 heteroatoms selected from N, O and S), -L-(3-10 membered heteroaryl containing 1-5 heteroatoms selected from N, O and S), -L-(3-10 membered aryl), wherein the C1-C4 alkyl, C3-C8 cycloalkyl, 3-10 membered heterocycloalkyl, 3-10 membered heteroaryl, and 3-10 membered aryl are optionally substituted by 0 to 4 D, OH, halogen, CN, C1-C4 alkyl, C1-C4 haloalkyl, N(C1-C4 alkyl)$_2$, NHCO(C1-C4 alkyl), $SO_2$(C1-C4 alkyl), $CO_2$(C1-C4 alkyl), 3-6 membered heterocycloalkyl containing 1-4 heteroatoms selected from N, O and S, CO(C1-C4 alkyl), C1-C4 alkoxy or C1-C4 haloalkoxy;

$R_8$ and $R_9$ are independently selected from the group consisting of: H, C1-C4 alkyl, C1-C4 haloalkyl, C3-C8 cycloalkyl, -L-(C3-C8 cycloalkyl), -L-(3-10 membered heterocycloalkyl containing 1-4 heteroatoms selected from N, O and S), and -L-(3-10 membered heteroaryl containing 1-5 heteroatoms selected from N, O and S); or $R_8$ and $R_9$ together with the N to which they are attached can form a substituted or unsubstituted 4-6 membered heterocyclyl, and the "substituted" refers to being substituted by 1-3 substituents selected from the group consisting of: =O, NR'R", and C1-C6 alkyl;

each R' and R" is independently selected from the group consisting of: H, and C1-C4 alkyl;

each L is independently a bond or C1-C4 alkylene, and the C1-C4 alkylene is optionally substituted by OH, C1-C4 alkoxy or C1-C4 haloalkoxy;

p is selected from the group consisting of: 0, 1, 2, 3 and 4;

r is selected from the group consisting of: 0, 1, 2, 3 and 4.

**2.** The compound according to claim 1, wherein the compound has the structure shown in formula (II) or formula (III):

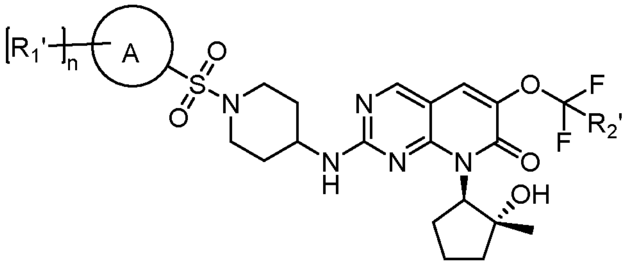

(II)

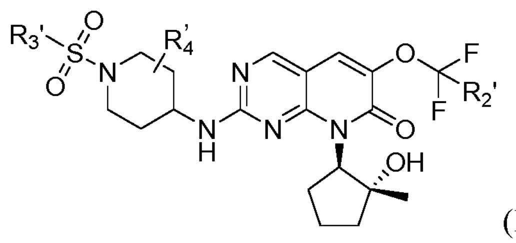

(III)

wherein,

ring A is selected from the group consisting of: 0-4 $R_1$' substituted 6-10 membered aryl, 0-4 $R_1$' substituted C3-C8 cycloalkyl, 0-4 $R_1$' substituted 5-10 membered heteroaryl containing 1-5 heteroatoms selected from N, O and S;

each $R_1$' is independently selected from the group consisting of: deuterium, halogen, OH, CN, $SO_2R_{31}$, $COR_{31}$, $CO_2R_{31}$, $NR_{41}R_{51}$, $NHCOR_{41}$, $CONR_{41}R_{51}$, $OCONR_{41}R_{51}$, $NHCONR_{41}R_{51}$, $NHCOOR_{41}$, C1-C4 alkyl, C1-C4 alkoxy, C1-C4 haloalkoxy, substituted or unsubstituted C3-C6 cycloalkyl, substituted or unsubstituted 3-6 membered heterocycloalkyl containing 1-4 heteroatoms selected from N, O and S, and the "substituted" refers to being substituted by 1-3 substituents selected from the group consisting of: deuterium, halogen, OH, CN, =O, C1-C4 alkyl, C1-C4 alkoxy, and C1-C4 haloalkoxy;

$R_2$' is selected from the group consisting of: H, and deuterium;

$R_3$' is selected from the group consisting of: substituted or unsubstituted C1-C4 alkyl, and the "substituted" refers to being substituted by 1-3 substituents selected from the group consisting of: deuterium, halogen, OH,

CN, =O, C1-C4 alkyl, C1-C4 alkoxy, C1-C4 haloalkoxy;

$R_4$' is selected from the group consisting of: H, deuterium, and OH; $R_{31}$ is selected from the group consisting of: H, C1-C4 alkyl, C1-C4 haloalkyl, C1-C4 alkoxy, C1-C4 haloalkoxy, substituted or unsubstituted C3-C6 cycloalkyl, substituted or unsubstituted 3-6 membered heterocycloalkyl containing 1-4 heteroatoms selected from N, O and S, and the "substituted" refers to being substituted by 1-3 substituents selected from the group consisting of: deuterium, halogen , OH, CN, =O, C1-C4 alkyl, C1-C4 alkoxy, and C1-C4 haloalkoxy;

$R_{41}$ and $R_{51}$ are independently selected from the group consisting of: H, C1-C4 alkyl, C1-C4 haloalkyl, C3-C8 cycloalkyl, -L'-(C3-C8 cycloalkyl), -L'-(3-10 membered heterocycloalkyl containing 1-4 heteroatoms selected from N, O and S), and -L'-(3-10 membered heteroaryl containing 1-5 heteroatoms selected from N, O and S), and L' is a bond or C1-C6 alkylene; or

$R_{41}$ and $R_{51}$ together with the N to which they are attached can form a substituted or unsubstituted 4-6 membered heterocyclyl, and the "substituted" refers to being substituted by 1-3 substituents selected from the group consisting of: halogen, OH, =O, and C1-C6 alkyl;

n is selected from the group consisting of: 0, 1, 2, 3 and 4.

**3.** The compound according to claim 2, wherein ring A is 0-4 $R_1$' substituted 6-10 membered aryl.

**4.** The compound according to claim 2, wherein A is 0-4 $R_1$' substituted C3-C8 cycloalkyl.

**5.** The compound according to claim 2, wherein

ring A is selected from the group consisting of: 0-4 $R_1$' substituted 6-10 membered aryl, 0-4 $R_1$' substituted C3-C8 cycloalkyl, 0-4 $R_1$' substituted 5-10 membered heteroaryl containing 1-5 heteroatoms selected from N, O and S;

each $R_1$' is independently selected from the group consisting of: deuterium, halogen, OH, CN, $SO_2R_{31}$, $COR_{31}$, $CO_2R_{31}$, $NR_{41}R_{51}$, $NHCOR_{41}$, $CONR_{41}R_{51}$, C1-C4 haloalkyl, C1-C4 alkoxy, C1-C4 haloalkoxy, substituted or unsubstituted C3-C6 cycloalkyl, substituted or unsubstituted 3-6 membered heterocycloalkyl containing 1-4 heteroatoms selected from N, O and S;

$R_2$' is selected from the group consisting of: H, and deuterium;

$R_4$' is selected from the group consisting of: H, and deuterium;

$R_{31}$ is C1-C4 alkyl;

$R_{41}$ and $R_{51}$ are independently selected from the group consisting of: H, and C1-C4 alkyl;

n is selected from the group consisting of: 0, 1, 2, 3 and 4

**6.** The compound according to claim 2, wherein

ring A is selected from the group consisting of 0-4 $R_1$' substituted 6-10 membered aryl, and 0-4 $R_1$'substituted C3-C8 cycloalkyl;

$R_1$' is selected from the group consisting of halogen, OH, C1-C4 alkoxy, and C1-C4 haloalkoxy;

$R_2$' is selected from the group consisting of H, and deuterium;

n is selected from the group consisting of: 0, 1, 2, 3 and 4.

**7.** A compound, wherein the compound is selected from the group consisting of:

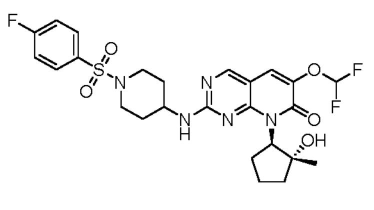
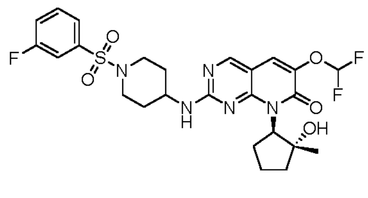
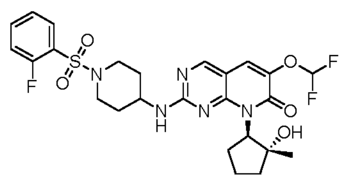

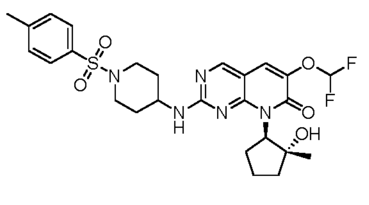
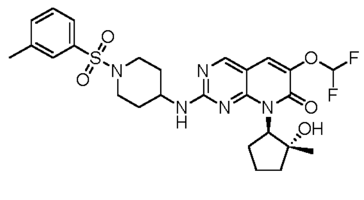
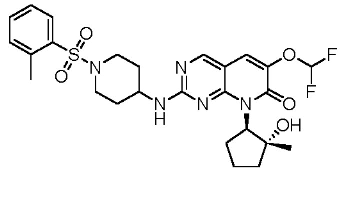

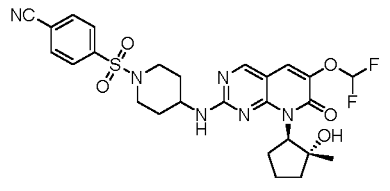
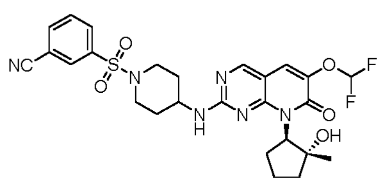
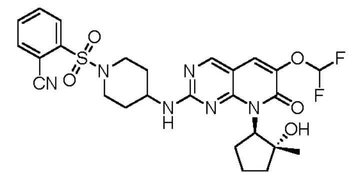
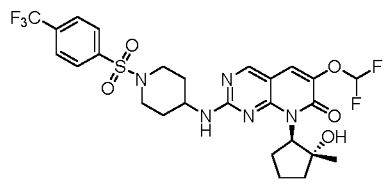
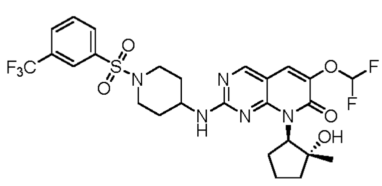
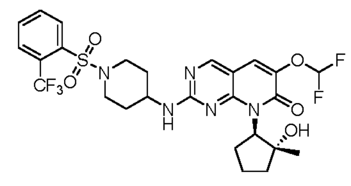
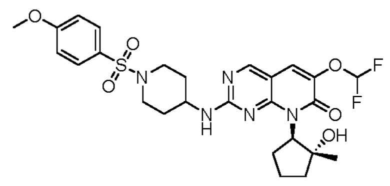
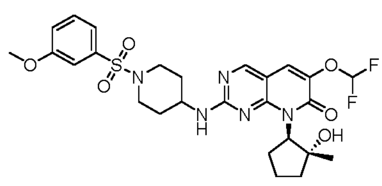
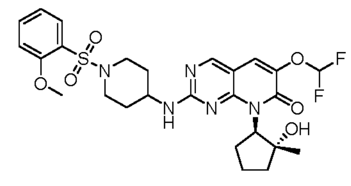
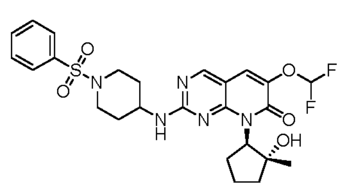
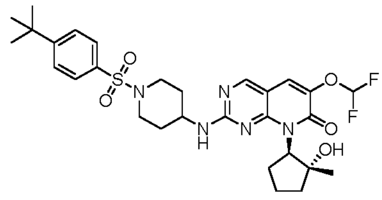
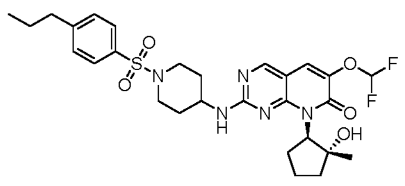
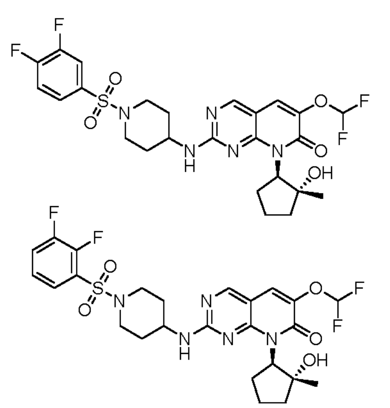
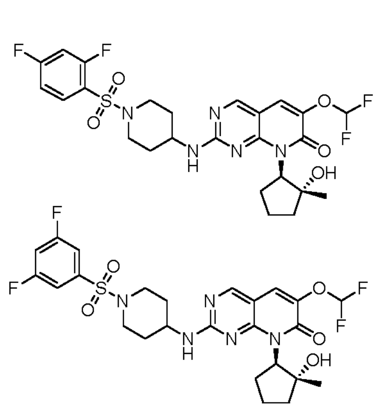
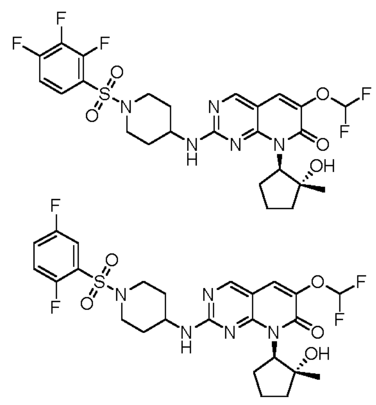
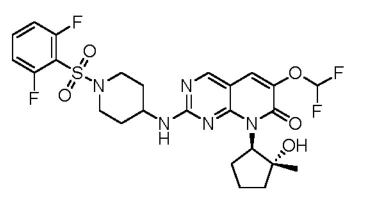
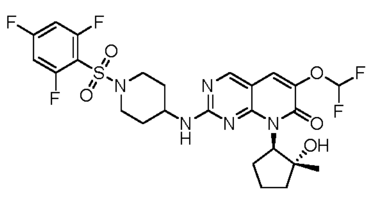
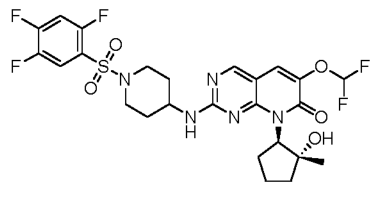

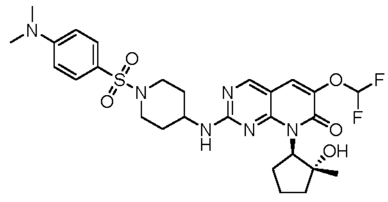
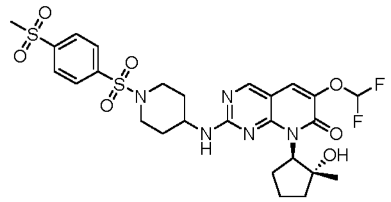
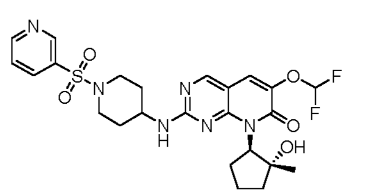
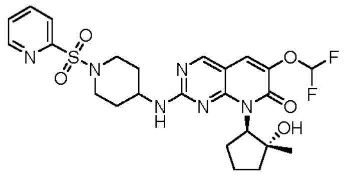
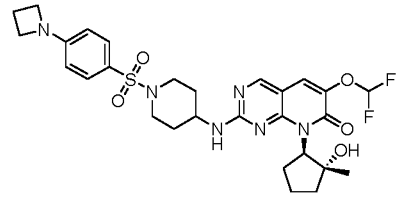
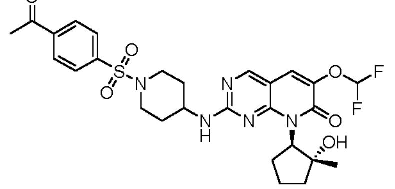
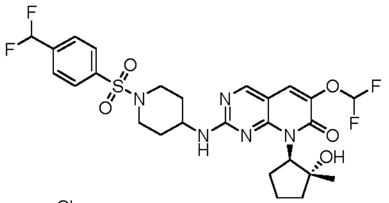
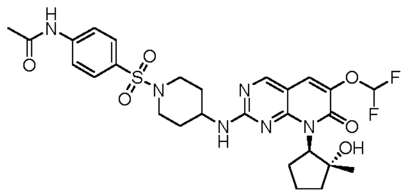
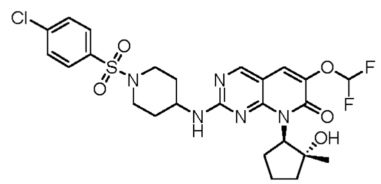
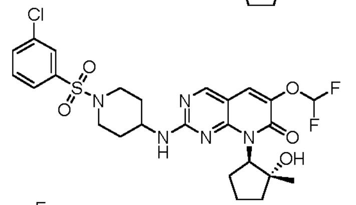
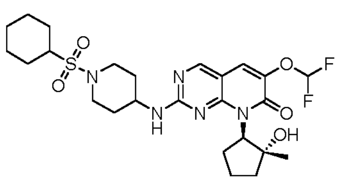
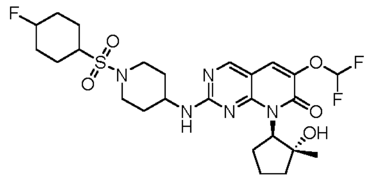
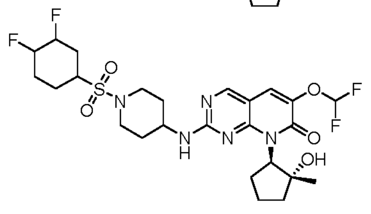
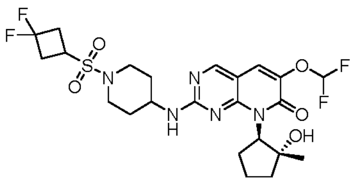
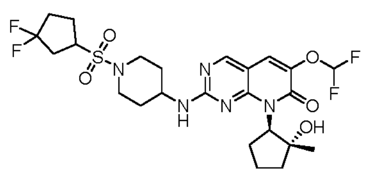
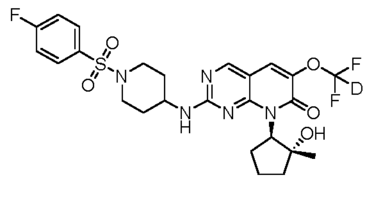
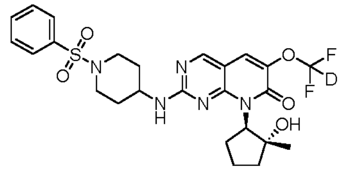
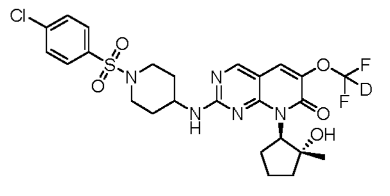
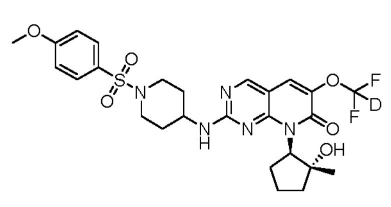
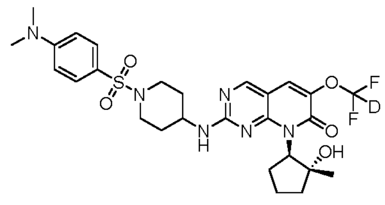
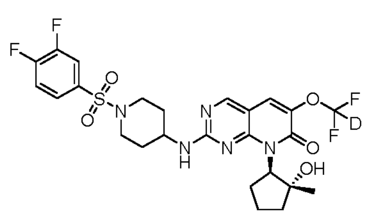
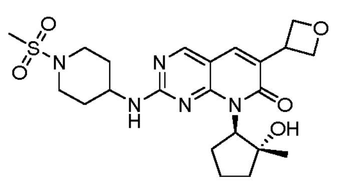
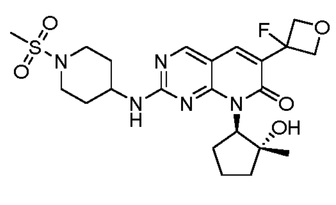
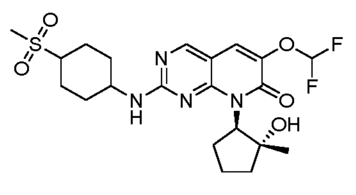

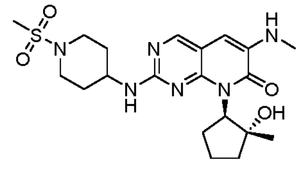
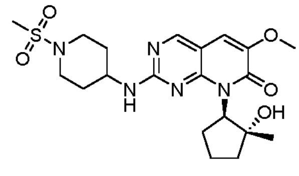
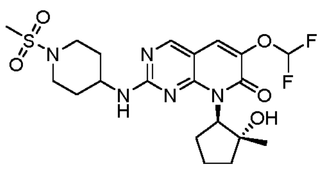

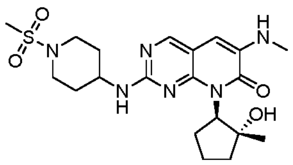
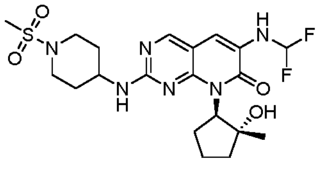
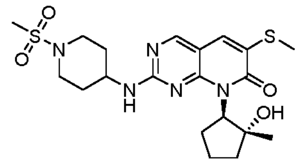

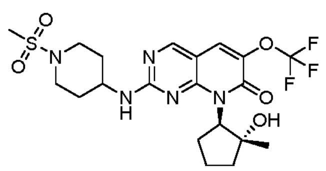
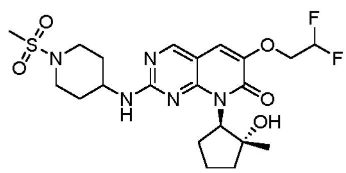
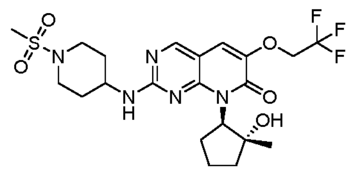

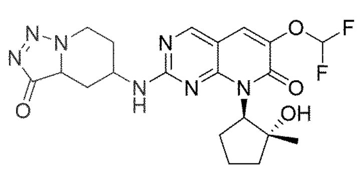
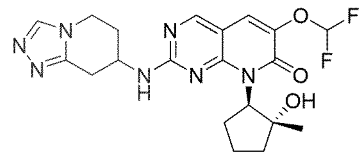
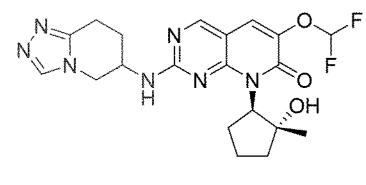

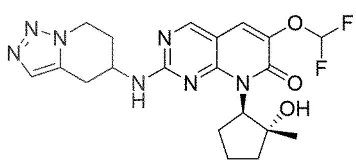
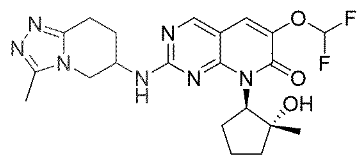
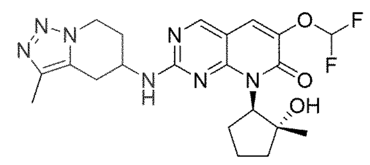

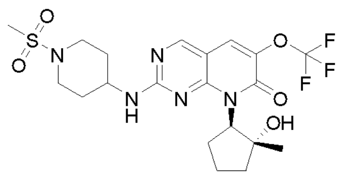
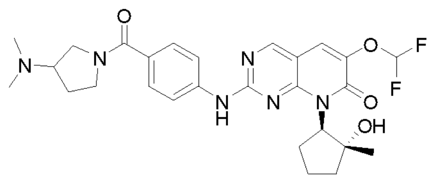
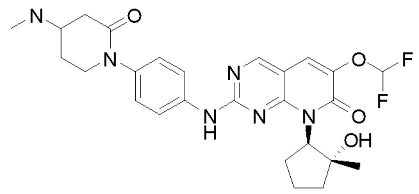

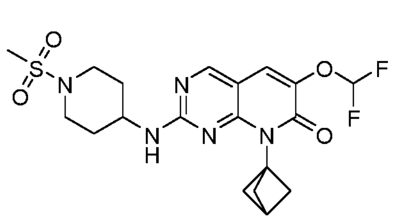
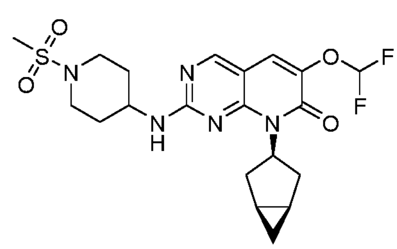
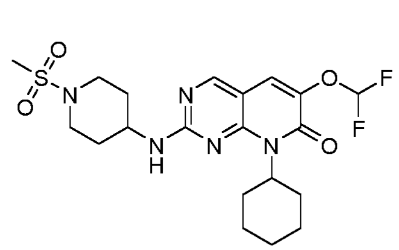

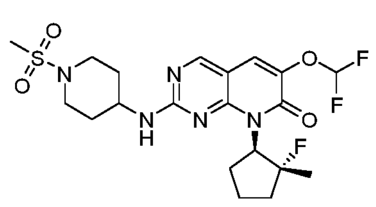
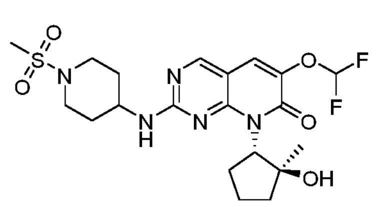
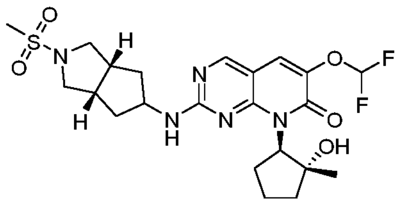

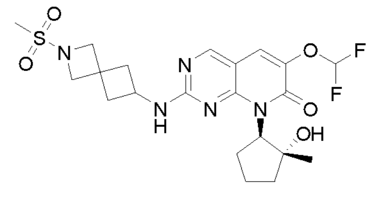
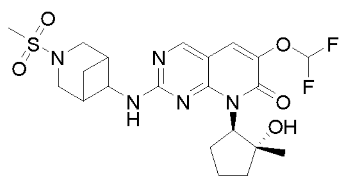
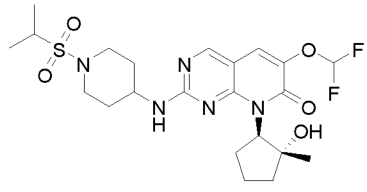

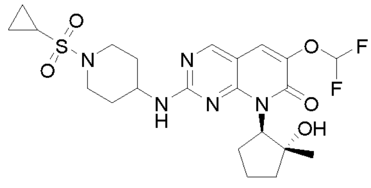
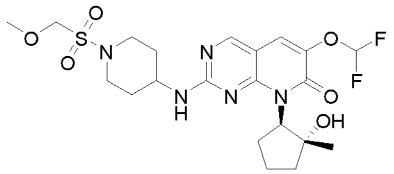
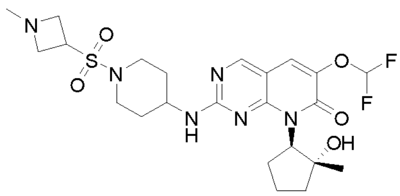

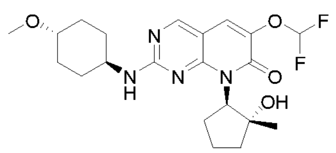
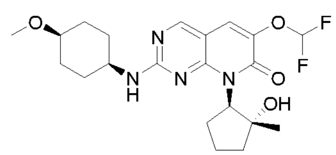
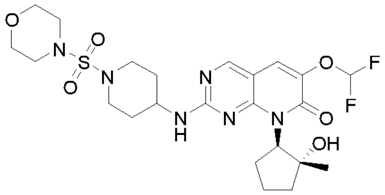

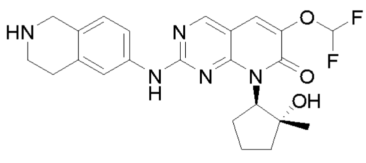
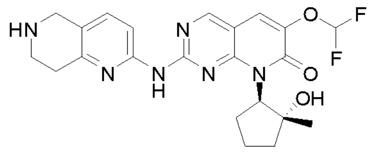
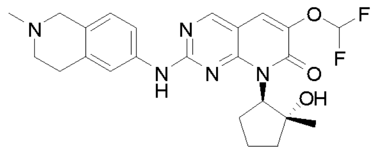

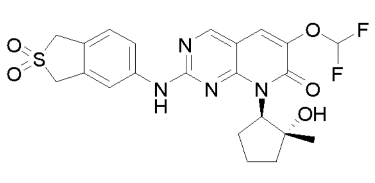
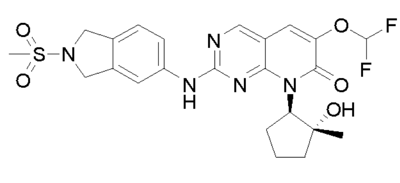
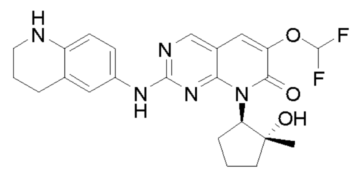

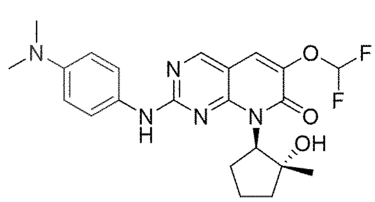
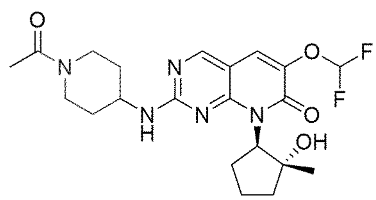
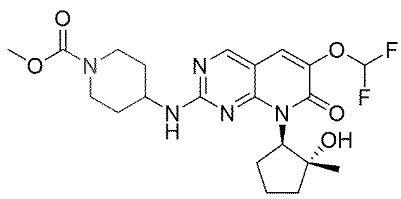

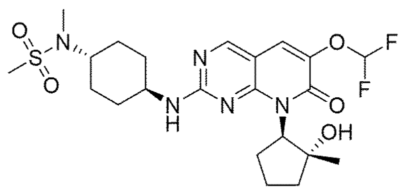
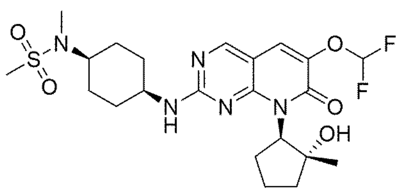
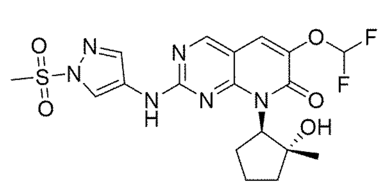
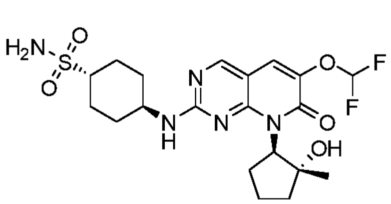
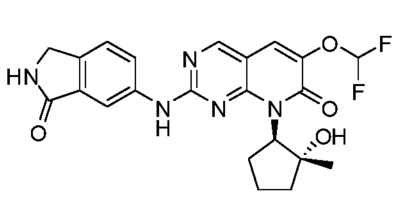
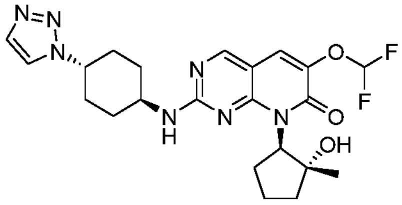
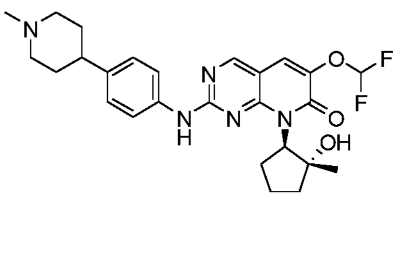
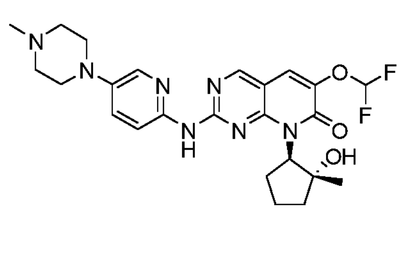
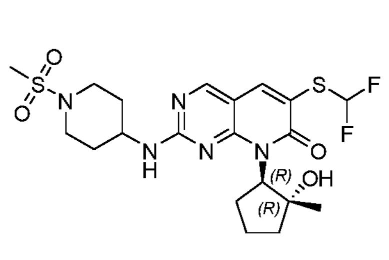
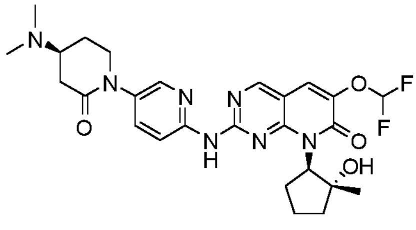
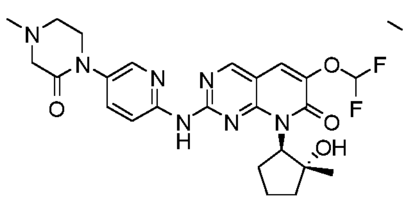
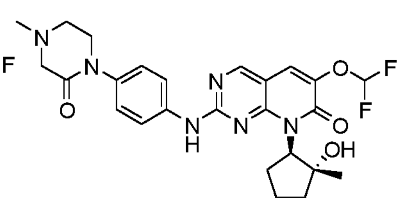
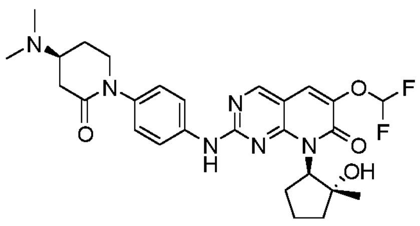
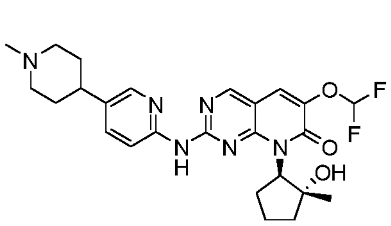
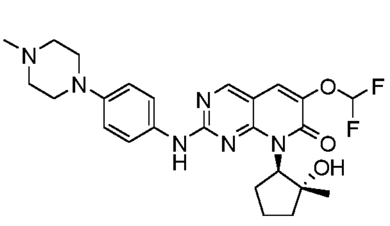
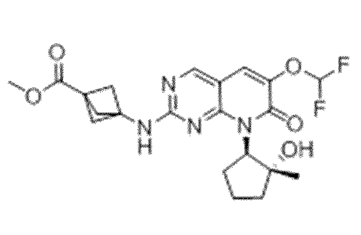
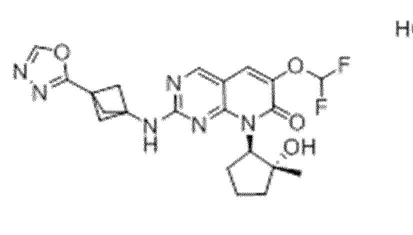
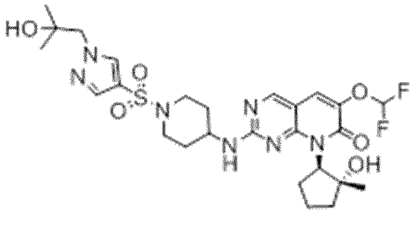
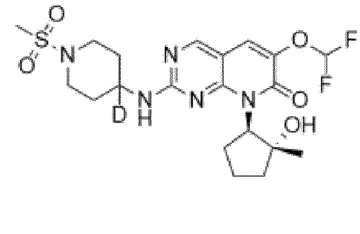
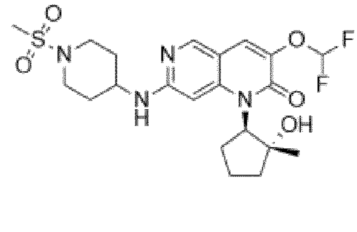
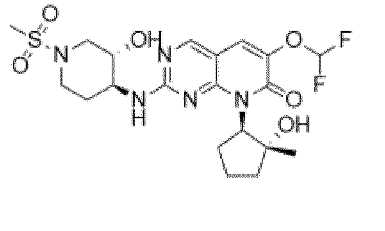

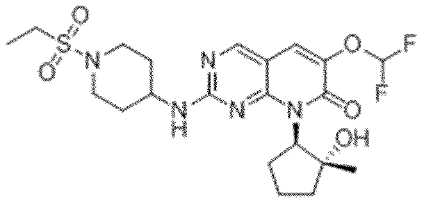
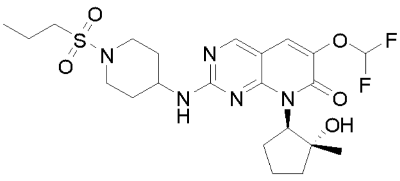
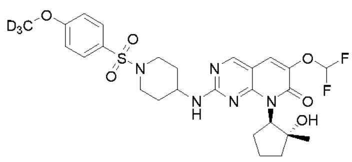

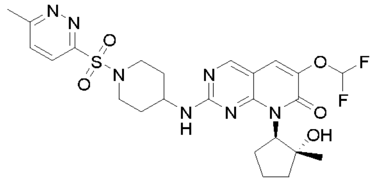
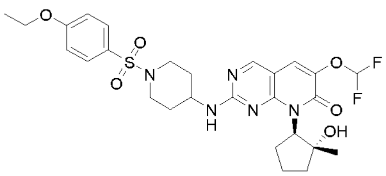
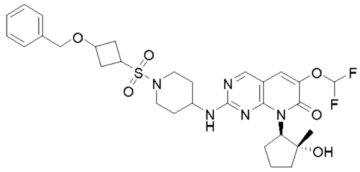

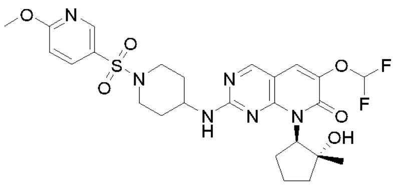
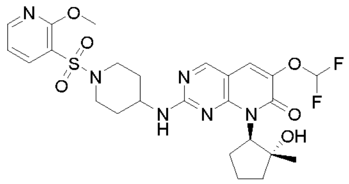
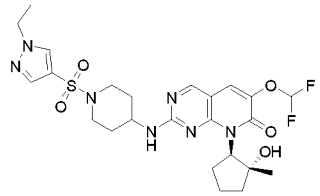

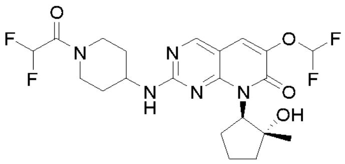
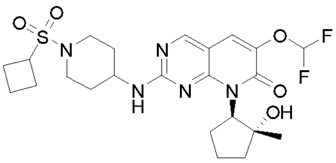
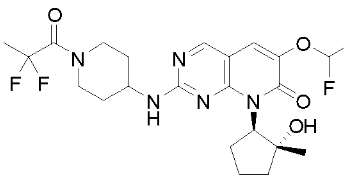

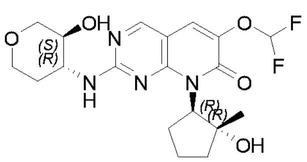
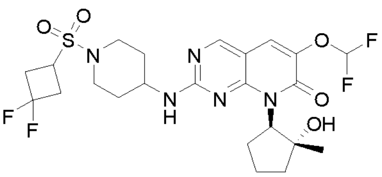
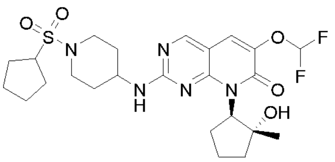

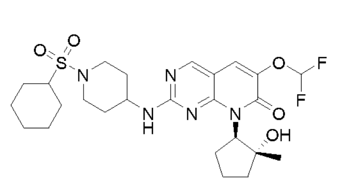
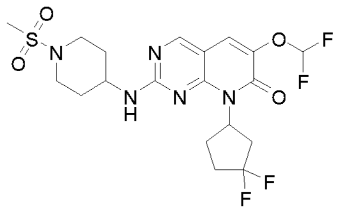
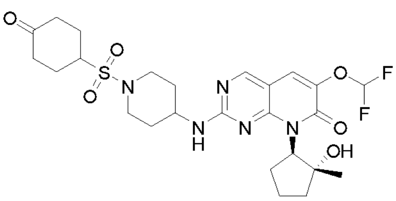

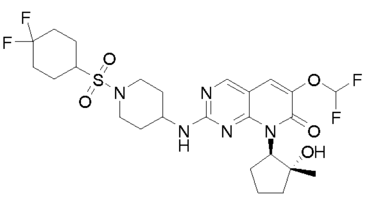
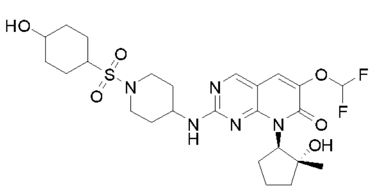
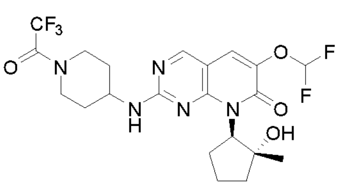

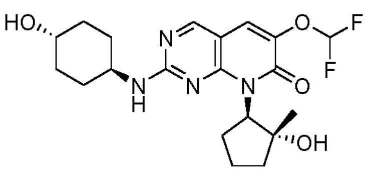 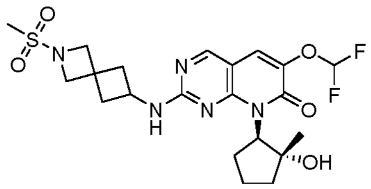 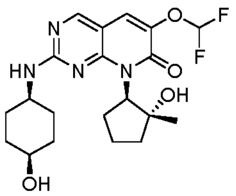

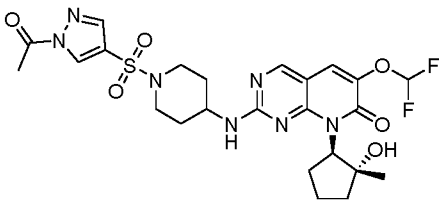 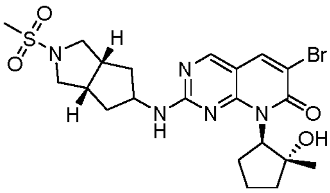 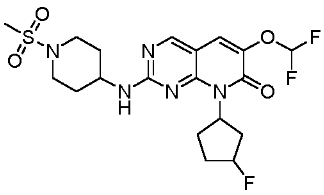

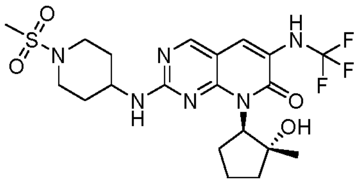 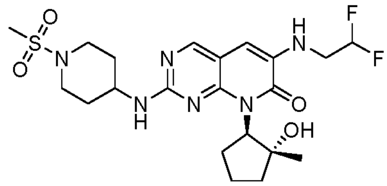 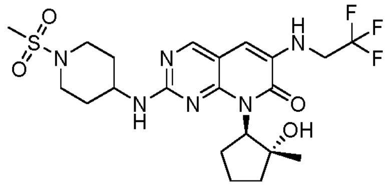

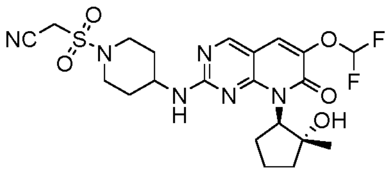 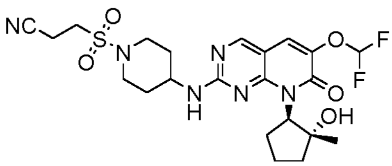 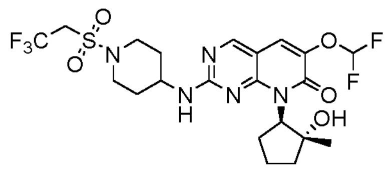

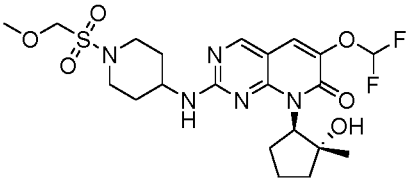 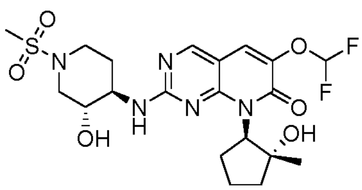 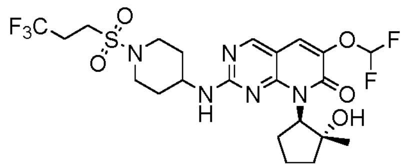 .

**8.** A pharmaceutical composition, comprising a therapeutically effective amount of the compound according to claim 1, or a pharmaceutically acceptable salt, a stereoisomer, a solvate or a prodrug thereof, and a pharmaceutically acceptable carrier.

**9.** A use of the compound according to claim 1, or a pharmaceutically acceptable salt, a stereoisomer, a solvate or a prodrug thereof in the preparation of CDK2/4/6 kinases inhibitor drugs.

**10.** A use of the compound according to claim 1, or a pharmaceutically acceptable salt, a stereoisomer, a solvate or a prodrug thereof in the preparation of drugs for regulating CDK kinase activity or treating CDK-related diseases.

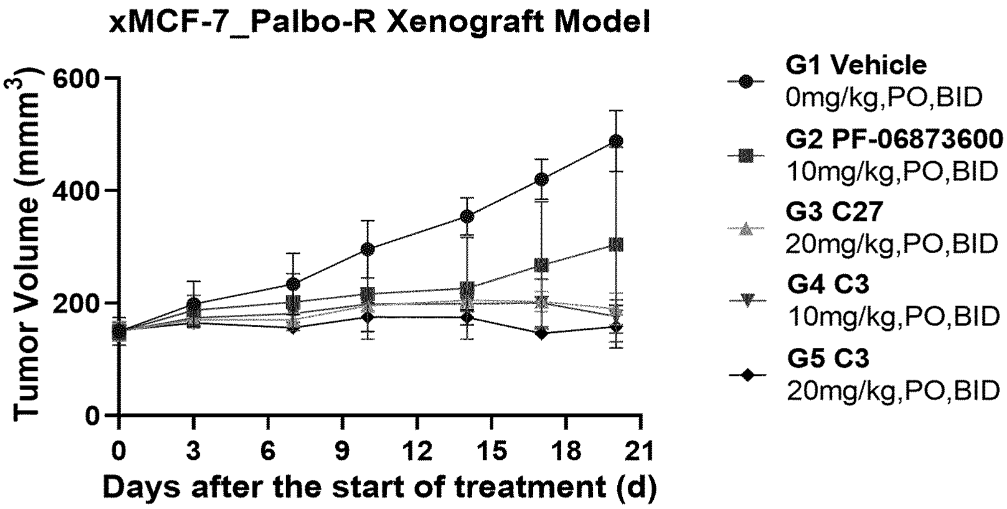


Figure 1

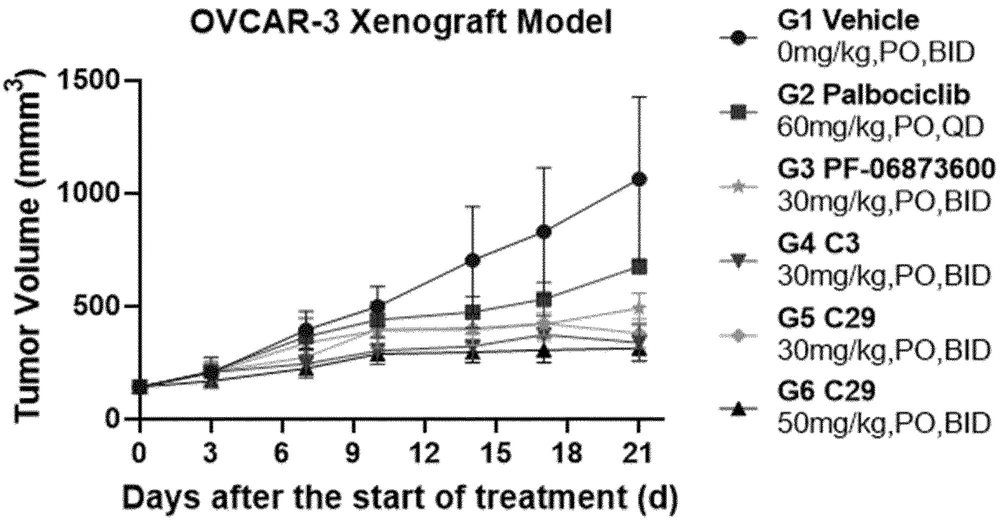


Figure 2

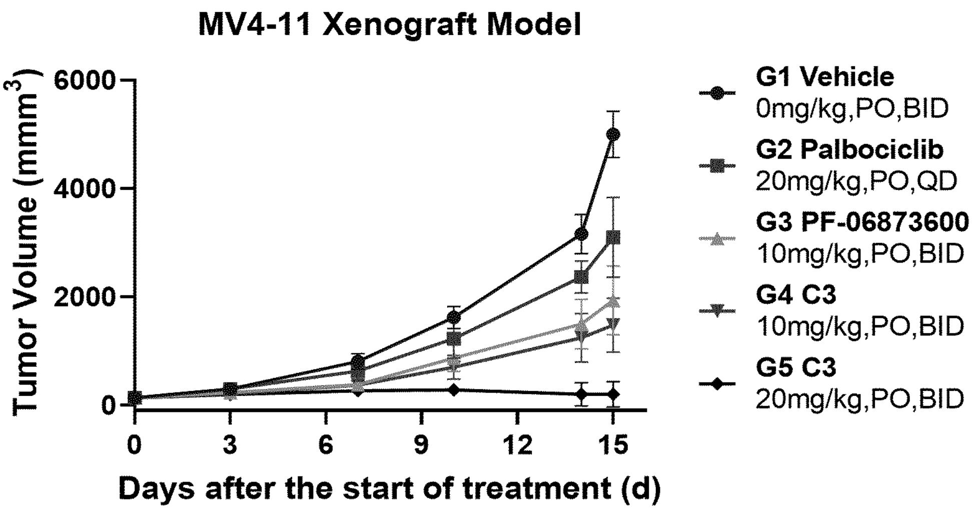


Figure 3

**INTERNATIONAL SEARCH REPORT**

International application No.
**PCT/CN2022/097927**

**A. CLASSIFICATION OF SUBJECT MATTER**

C07D 471/04(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D471/-,A61K31/-

Documentation searched other than minimum documentation to the extent that such documents are included in the fields searched

Electronic data base consulted during the international search (name of data base and, where practicable, search terms used)

CNTXT, CNKI, VEN, STN(REG, CAPLUS): 吡啶并, 嘧啶, 酮, 哌啶, 环戊基, CDK, 激酶, PYRID+, PYRIMIDIN+, PIPERIDIN, CYCLOPENTYL, KINASE, sub-structure search based on formula (II), (III)

**C. DOCUMENTS CONSIDERED TO BE RELEVANT**

| Category* | Citation of document, with indication, where appropriate, of the relevant passages | Relevant to claim No. |
|---|---|---|
| X | US 2018044344 A1 (PFIZER) 15 February 2018 (2018-02-15) description pages 2, 57, 58, 120 | 1-10 |
| X | WO 2020065494 A1 (PFIZER) 02 April 2020 (2020-04-02) description, pages 1-2 | 1-10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 September 2022** | **16 September 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/097927**

| Patent document cited in search report | Publication date (day/month/year) | Patent family member(s) | Publication date (day/month/year) |
|---|---|---|---|
| US 2018044344 A1 | 15 February 2018 | US 2020392142 A1 | 17 December 2020 |
| | | NI 201900013 A | 08 April 2019 |
| | | CU 20190010 A7 | 04 October 2019 |
| | | RS 61934 B1 | 30 July 2021 |
| | | CN 109803968 A | 24 May 2019 |
| | | IL 264687 B | 29 April 2021 |
| | | TN 2019000039 A1 | 15 July 2020 |
| | | ES 2876411 T3 | 12 November 2021 |
| | | PH 12019500329 A1 | 28 October 2019 |
| | | EP 3497103 A1 | 19 June 2019 |
| | | PE 20190475 A1 | 04 April 2019 |
| | | PT 3497103 T | 17 June 2021 |
| | | CL 2019000368 A1 | 10 May 2019 |
| | | JP 6563623 B1 | 21 August 2019 |
| | | GE P20217234 B | 25 March 2021 |
| | | DK 3497103 T3 | 14 June 2021 |
| | | EC SP19011216 A | 29 March 2019 |
| | | CN 114394966 A | 26 April 2022 |
| | | ZA 201900716 B | 30 March 2022 |
| | | UA 124804 C2 | 24 November 2021 |
| | | US 2019135817 A1 | 09 May 2019 |
| | | HR P20210871 T1 | 09 July 2021 |
| | | MX 2019001849 A | 09 May 2019 |
| | | CR 20190062 A | 22 May 2019 |
| | | AU 2017311645 A1 | 28 February 2019 |
| | | SI 3497103 T1 | 30 July 2021 |
| | | MA 45920 A | 19 June 2019 |
| | | BR 112019002610 A2 | 02 July 2019 |
| | | SV 2019005836 A | 19 March 2019 |
| | | CA 2975033 A1 | 15 February 2018 |
| | | MD 3497103 T2 | 31 August 2021 |
| | | KR 20190038915 A | 09 April 2019 |
| | | LT 3497103 T | 26 July 2021 |
| | | SG 11201900799 X A | 27 February 2019 |
| | | PL 3497103 T3 | 25 October 2021 |
| | | RU 2726115 C1 | 09 July 2020 |
| | | CO 2019001240 A2 | 19 February 2019 |
| | | HU E055978 T2 | 28 January 2022 |
| | | WO 2018033815 A1 | 22 February 2018 |
| | | TW 201819383 A | 01 June 2018 |
| | | UY 37352 A | 23 March 2018 |
| | | DO P2019000030 A | 30 June 2019 |
| WO 2020065494 A1 | 02 April 2020 | US 2022056025 A1 | 24 February 2022 |
| | | TW 202024059 A | 01 July 2020 |
| | | EP 3856725 A1 | 04 August 2021 |
| | | CA 3113832 A1 | 02 April 2020 |
| | | CN 113039178 A | 25 June 2021 |
| | | JP 2020097562 A | 25 June 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018033815 A1


### Non-patent literature cited in the description


- **SAMBROOK et al.** Molecular Cloning: A Laboratory Manual. Cold Spring Harbor Laboratory Press, 1989